(19) Europäisches Patentamt
European Patent Office
Office européen des brevets

(11) EP 4 242 208 A1

(12) EUROPEAN PATENT APPLICATION
published in accordance with Art. 153(4) EPC

(43) Date of publication:
**13.09.2023 Bulletin 2023/37**

(21) Application number: **21888622.4**

(22) Date of filing: **04.11.2021**

(51) International Patent Classification (IPC):
***C07D 471/04*** *(2006.01)* ***A61K 31/437*** *(2006.01)*
***A61P 35/00*** *(2006.01)* ***A61P 37/00*** *(2006.01)*
***A61P 29/00*** *(2006.01)* ***A61P 25/28*** *(2006.01)*
***A61P 11/00*** *(2006.01)* ***A61P 9/00*** *(2006.01)*

(52) Cooperative Patent Classification (CPC):
**A61K 31/437; A61P 9/00; A61P 11/00; A61P 25/28; A61P 29/00; A61P 35/00; A61P 37/00; C07D 471/04**

(86) International application number:
**PCT/CN2021/128735**

(87) International publication number:
**WO 2022/095930 (12.05.2022 Gazette 2022/19)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **04.11.2020 CN 202011213775**
**25.12.2020 CN 202011562922**
**22.01.2021 CN 202110085643**

(71) Applicant: **Sichuan Haisco Pharmaceutical Co., Ltd.**
**Sichuan 611130 (CN)**

(72) Inventors:
- **LI, Yao**
  **Chengdu, Sichuan 611130 (CN)**
- **ZHANG, Guobiao**
  **Chengdu, Sichuan 611130 (CN)**
- **ZHANG, Xiaobo**
  **Chengdu, Sichuan 611130 (CN)**
- **TANG, Pingming**
  **Chengdu, Sichuan 611130 (CN)**
- **CHEN, Yashu**
  **Chengdu, Sichuan 611130 (CN)**
- **YU, Yan**
  **Chengdu, Sichuan 611130 (CN)**
- **ZHANG, Chen**
  **Chengdu, Sichuan 611130 (CN)**
- **YAN, Pangke**
  **Chengdu, Sichuan 611130 (CN)**

(74) Representative: **Hoffmann Eitle**
**Patent- und Rechtsanwälte PartmbB**
**Arabellastraße 30**
**81925 München (DE)**

# (54) DEUTERATED DERIVATIVE AS ATX INHIBITOR, AND APPLICATION THEREOF

(57) Disclosed are a compound of formula (I), a stereoisomer, solvate, prodrug, metabolite, pharmaceutically acceptable salt, or eutectic thereof, or a pharmaceutical composition comprising same, and an application thereof as an ATX inhibitor in preparation of a drug for treating a related disease. Groups in formula (I) are as defined in the description.



Processed by Luminess, 75001 PARIS (FR)

(Cont. next page)

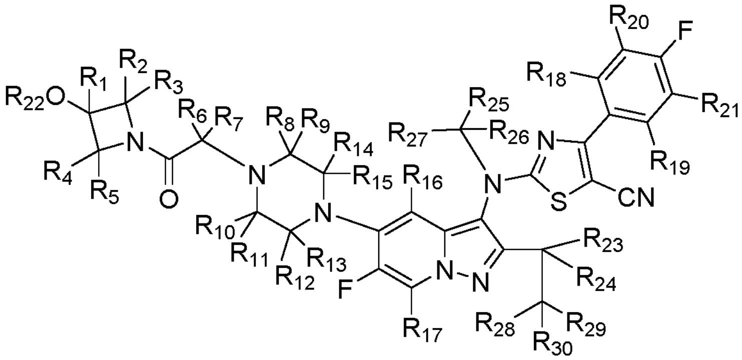

(I)

**Description**

Technical Field

The present invention belongs to the field of medicine, and particularly relates to a deuterated derivative as an ATX inhibitor, or a stereoisomer, solvate, prodrug, metabolite, pharmaceutically acceptable salt or eutectic thereof, and the use thereof in the preparation of a drug for treating a related disease.

Background Art

Autotaxin (ATX) is an extracellular secretase (also known as ENPP2), and its main physiological function is to produce lysophosphatidic acid (LPA) and choline having biological activities by means of hydrolysing lysophosphatidylcholine (LPC). Hydrolysis of LPC by ATX is the major source of LPA in blood. By acting on LPA receptors (at least 6 kinds of $LPA_{1-6}$), LPA produces a series of physiological activities including cell proliferation, survival, movement, etc. ATX-LPA signalling pathways are involved in many pathological processes, including angiogenesis, autoimmune diseases, inflammation, fibrosis, neurodegenerative diseases and pain. The most widely studied pathological processes are fibrosis and tumour, particularly idiopathic pulmonary fibrosis (IPF). In 2014, FDA approved two new drugs Pirfenidone and Nintedanib (a triple angiokinase inhibitor), which are used for treating IPF, primarily for preventing further development and deterioration of IPF, but exhibit less significant curative effect on IPF itself. Therefore, people have been committed to finding more effective drugs for IPF treatment. GLPG-1690 as an ATX inhibitor has a relatively fast clinical progress (phase III clinical trial) for the treatment of IPF, and has shown a good curative effect in a phase II clinical trial.

Summary of the Invention

An object of the present invention is to provide a deuterated ATX inhibitor, which has the advantages of novel structure, good efficacy, high bioavailability, less side effects, rapid onset and long acting.

The present invention relates to a compound of formula (I) or a stereoisomer, solvate, prodrug, metabolite, pharmaceutically acceptable salt or eutectic thereof, wherein

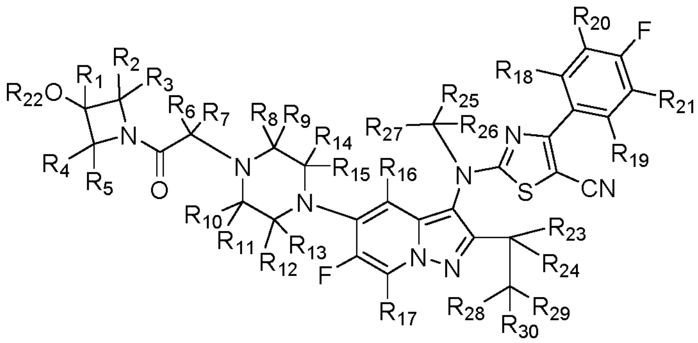

(I)

$R_1$, $R_2$, $R_3$, $R_4$, $R_5$, $R_6$, $R_7$, $R_8$, $R_9$, $R_{10}$, $R_{11}$, $R_{12}$, $R_{13}$, $R_{14}$, $R_{15}$, $R_{16}$, $R_{17}$, $R_{18}$, $R_{19}$, $R_{20}$, $R_{21}$, $R_{22}$, $R_{23}$, $R_{24}$, $R_{25}$, $R_{26}$, $R_{27}$, $R_{28}$, $R_{29}$ and $R_{30}$ are each independently selected from hydrogen or deuterium,

provided that at least one of $R_1$, $R_2$, $R_3$, $R_4$, $R_5$, $R_6$, $R_7$, $R_8$, $R_9$, $R_{10}$, $R_{11}$, $R_{12}$, $R_{13}$, $R_{14}$, $R_{15}$, $R_{16}$, $R_{17}$, $R_{18}$, $R_{19}$, $R_{20}$, $R_{21}$, $R_{22}$, $R_{23}$, $R_{24}$, $R_{25}$, $R_{26}$, $R_{27}$, $R_{28}$, $R_{29}$ and $R_{30}$ is selected from deuterium and the compound of formula (I) does not have the following structure:

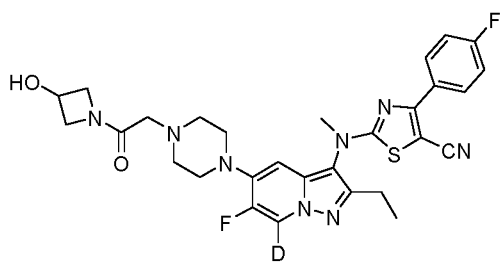

.

In certain embodiments, $R_{22}$ is selected from H;
in certain embodiments, $R_1$ is selected from deuterium;
in certain embodiments, $R_2$ and $R_3$ are selected from deuterium;
in certain embodiments, $R_6$ and $R_7$ are selected from deuterium;
in certain embodiments, $R_8$, $R_9$, $R_{10}$, $R_{11}$, $R_{12}$, $R_{13}$, $R_{14}$ and $R_{15}$ are selected from deuterium;
in certain embodiments, $R_{16}$ is selected from deuterium;
in certain embodiments, $R_{17}$ is selected from deuterium;
in certain embodiments, $R_{18}$, $R_{19}$, $R_{20}$ and $R_{21}$ are selected from deuterium;
in certain embodiments, $R_{23}$ and $R_{24}$ are selected from deuterium;
in certain embodiments, at least one of $R_{25}$, $R_{26}$ and $R_{27}$ is selected from deuterium;
in certain embodiments, $R_{25}$, $R_{26}$ and $R_{27}$ are selected from deuterium;
in certain embodiments, $R_{28}$, $R_{29}$ and $R_{30}$ are selected from deuterium;
in certain embodiments, $R_{23}$, $R_{24}$, $R_{25}$, $R_{26}$, $R_{27}$, $R_{28}$, $R_{29}$ and $R_{30}$ are selected from deuterium;
in certain embodiments, $R_8$, $R_9$, $R_{10}$, $R_{11}$, $R_{12}$, $R_{13}$, $R_{14}$, $R_{15}$, $R_{25}$, $R_{26}$ and $R_{27}$ are selected from deuterium;
in certain embodiments, $R_8$, $R_9$, $R_{10}$, $R_{11}$, $R_{12}$, $R_{13}$, $R_{14}$, $R_{15}$, $R_{23}$, $R_{24}$, $R_{25}$, $R_{26}$, $R_{27}$, $R_{28}$, $R_{29}$ and $R_{30}$ are selected from deuterium;
in certain embodiments, $R_8$, $R_9$, $R_{10}$, $R_{11}$, $R_{12}$, $R_{13}$, $R_{14}$, $R_{15}$, $R_{16}$ and $R_{17}$ are selected from deuterium;
in certain embodiments, $R_8$, $R_9$, $R_{10}$, $R_{11}$, $R_{12}$, $R_{13}$, $R_{14}$, $R_{15}$, $R_{18}$, $R_{19}$, $R_{20}$ and $R_{21}$ are selected from deuterium;
in certain embodiments, $R_{23}$, $R_{24}$, $R_{28}$, $R_{29}$, $R_{30}$, $R_{18}$, $R_{19}$, $R_{20}$ and $R_{21}$ are selected from deuterium;
in certain embodiments, $R_{25}$, $R_{26}$, $R_{27}$, $R_{18}$, $R_{19}$, $R_{20}$ and $R_{21}$ are selected from deuterium;
in certain embodiments, $R_1$, $R_{25}$, $R_{26}$ and $R_{27}$ are selected from deuterium;
in certain embodiments, $R_1$, $R_{23}$, $R_{24}$, $R_{28}$, $R_{29}$ and $R_{30}$ are selected from deuterium;
in certain embodiments, $R_6$, $R_7$, $R_{25}$, $R_{26}$ and $R_{27}$ are selected from deuterium;
in certain embodiments, $R_6$, $R_7$, $R_{23}$, $R_{24}$, $R_{28}$, $R_{29}$ and $R_{30}$ are selected from deuterium;
in certain embodiments, $R_2$, $R_3$, $R_{25}$, $R_{26}$ and $R_{27}$ are selected from deuterium; and
in certain embodiments, $R_2$, $R_3$, $R_{23}$, $R_{24}$, $R_{28}$, $R_{29}$ and $R_{30}$ are selected from deuterium.

The present invention provides the compound of formula (I) or the stereoisomer, solvate, prodrug, metabolite, pharmaceutically acceptable salt or eutectic thereof, wherein the compound has a structure selected from one of:

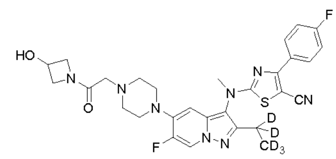
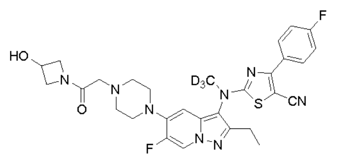
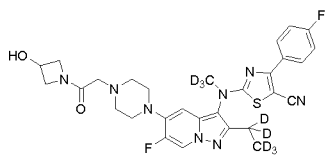

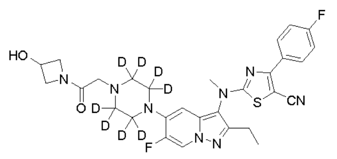
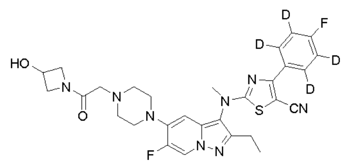
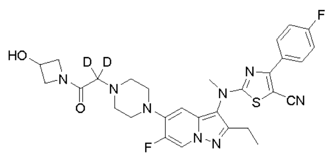

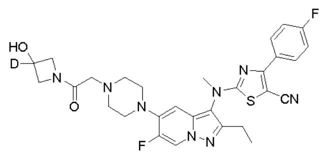
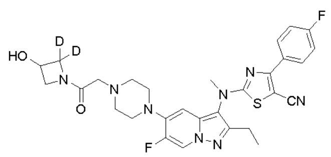

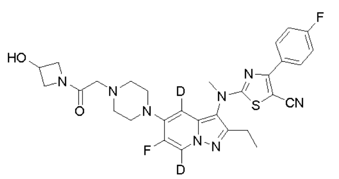
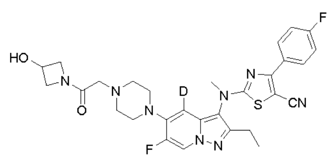
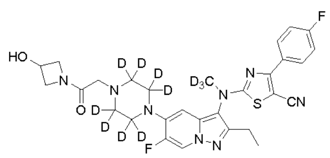

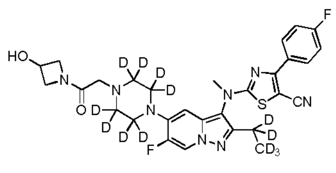
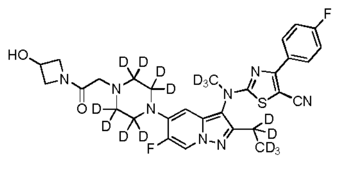
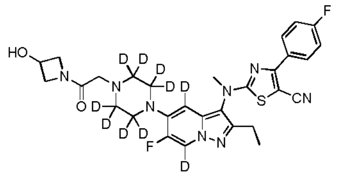

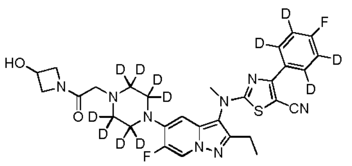
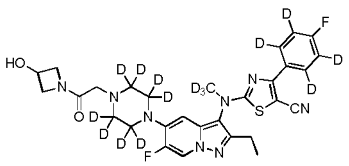
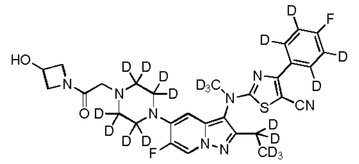

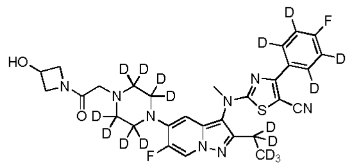
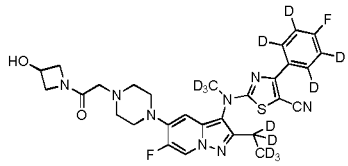
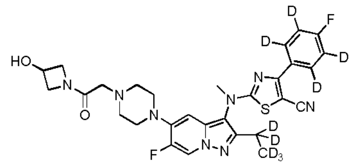

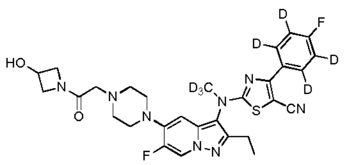
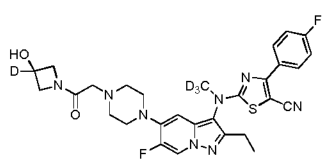

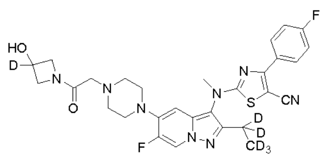
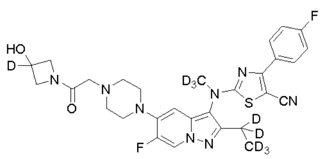
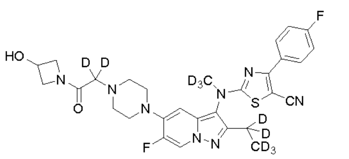

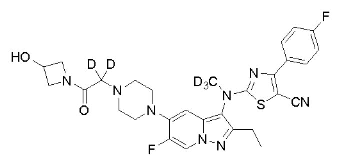
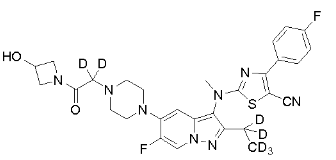
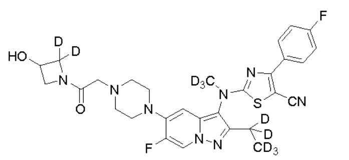

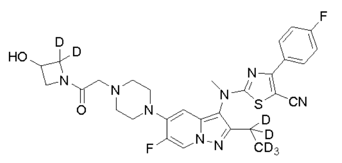
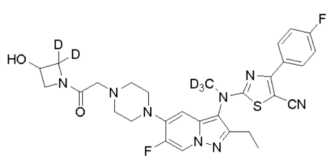
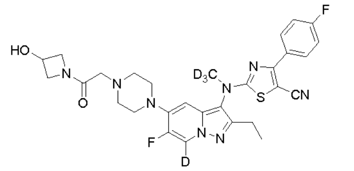

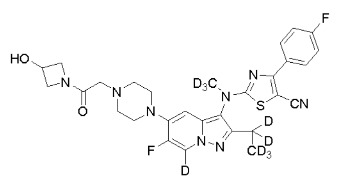
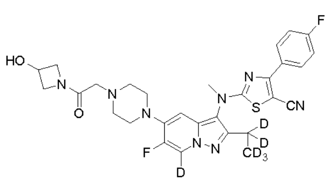
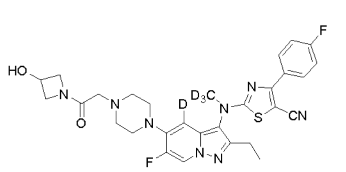

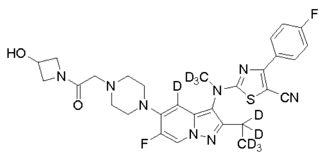
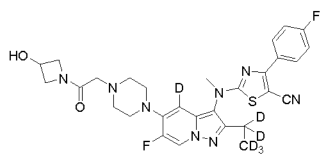
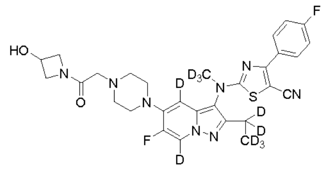

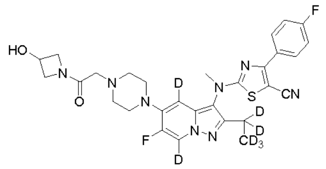
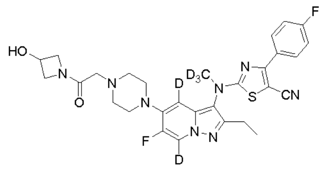
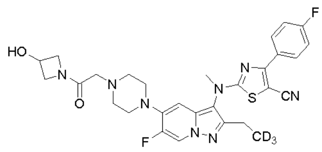

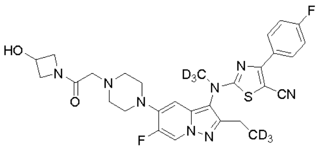
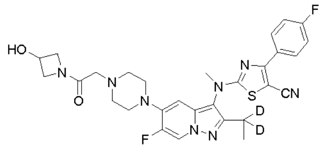
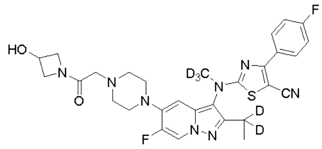
.

More specifically, the present invention further provides a crystal form I of the compound of formula (A). The crystal form has high purity, low hygroscopicity, good solubility, capabilities of resisting high temperature, high humidity and strong light, high stability, an appropriate particle size, easiness to filter and good fluidity, and is suitable for the preparation of drug dosage forms.

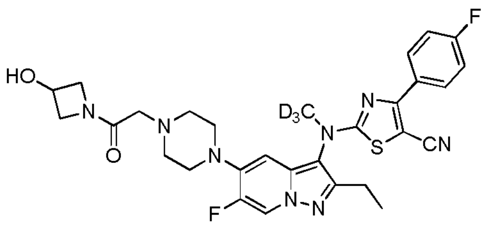

A

The crystal form I of the compound of formula (A) has an X-ray powder diffraction pattern comprising characteristic diffraction peaks at 9.35 ± 0.2°, 10.32 ± 0.2°, 12.08 ± 0.2° and 15.10 ± 0.2° 2$\theta$, as determined by using Cu-K$\alpha$ radiation.

Furthermore, the crystal form I of the compound of formula (A) has an X-ray powder diffraction pattern further comprising characteristic diffraction peaks at 17.29 ± 0.2°, 18.26 ± 0.2° and 20.58 ± 0.2° 2$\theta$.

Furthermore, the crystal form I of the compound of formula (A) has an X-ray powder diffraction pattern further comprising characteristic diffraction peaks at 6.92 ± 0.2°, 7.23 ± 0.2°, 13.46 ± 0.2°, 19.47 ± 0.2° and 24.85 ± 0.2° 2$\theta$.

Furthermore, the crystal form I of the compound of formula (A) has an X-ray powder diffraction pattern substantially as shown in Figure 1.

Furthermore, the crystal form I of the compound of formula (A) has an X-ray powder diffraction peak substantially as shown in Table 1.

Furthermore, the crystal form I of the compound of formula (A) has a TGA curve substantially as shown in Figure 2 and a DSC pattern substantially as shown in Figure 3.

Furthermore, with regard to the crystal form I of the compound of formula (A) of the present invention, the crystal has a particle size less than 100 $\mu$m; in some embodiments, the crystal has a particle size less than 90 $\mu$m; in some embodiments, the crystal has a particle size less than 80 $\mu$m; in some embodiments, the crystal has a particle size less than 70 $\mu$m; in some embodiments, the crystal has a particle size less than 60 $\mu$m; and in some embodiments, the crystal has a particle size less than 50 $\mu$m.

The present invention further provides a method for preparing a crystal form I of a compound of formula (A), wherein the method comprises: adding a crude product of the compound of formula (A) to an alcohol solvent, warming and stirring to dissolve same, then cooling to room temperature, and performing crystallization and filtration to obtain the crystal form I.

In some embodiments, according to the above-mentioned preparation method, the alcohol solvent is selected

from methanol, ethanol, propyl alcohol, isopropyl alcohol or a combination thereof.

In some embodiments, according to the above-mentioned preparation method, the ratio of the compound of formula (A) to the alcohol solvent is 1 : 5-8 g/mL. In some embodiments, the ratio of the compound of formula (A) to the alcohol solvent is 1 : 5-6 g/mL.

In some embodiments, according to the above-mentioned preparation method, the warming involves warming to 60°C-90°C. In some embodiments, the warming involves warming to 70°C-80°C. In some embodiments, the warming involves warming to 75°C.

The crystal form I of the compound of formula (A) of the present invention accounts for about 5 wt% to about 100 wt% of a bulk drug; in certain embodiments, the crystal form I of the compound of formula (A) accounts for about 10 wt% to about 100 wt% of a bulk drug; in certain embodiments, the crystal form I of the compound of formula (A) accounts for about 15 wt% to about 100 wt% of a bulk drug; in certain embodiments, the crystal form I of the compound of formula (A) accounts for about 20 wt% to about 100 wt% of a bulk drug; in certain embodiments, the crystal form I of the compound of formula (A) accounts for about 25 wt% to about 100 wt% of a bulk drug; in certain embodiments, the crystal form I of the compound of formula (A) accounts for about 30 wt% to about 100 wt% of a bulk drug; in certain embodiments, the crystal form I of the compound of formula (A) accounts for about 35 wt% to about 100 wt% of a bulk drug; in certain embodiments, the crystal form I of the compound of formula (A) accounts for about 40 wt% to about 100 wt% of a bulk drug; in certain embodiments, the crystal form I of the compound of formula (A) accounts for about 45 wt% to about 100 wt% of a bulk drug; in certain embodiments, the crystal form I of the compound of formula (A) accounts for about 50 wt% to about 100 wt% of a bulk drug; in certain embodiments, the crystal form I of the compound of formula (A) accounts for about 55 wt% to about 100 wt% of a bulk drug; in certain embodiments, the crystal form I of the compound of formula (A) accounts for about 60 wt% to about 100 wt% of a bulk drug; in certain embodiments, the crystal form I of the compound of formula (A) accounts for about 65 wt% to about 100 wt% of a bulk drug; in certain embodiments, the crystal form I of the compound of formula (A) accounts for about 70 wt% to about 100 wt% of a bulk drug; in certain embodiments, the crystal form I of the compound of formula (A) accounts for about 75 wt% to about 100 wt% of a bulk drug; in certain embodiments, the crystal form I of the compound of formula (A) accounts for about 80 wt% to about 100 wt% of a bulk drug; in certain embodiments, the crystal form I of the compound of formula (A) accounts for about 85 wt% to about 100 wt% of a bulk drug; in certain embodiments, the crystal form I of the compound of formula (A) accounts for about 90 wt% to about 100 wt% of a bulk drug; in certain embodiments, the crystal form I of the compound of formula (A) accounts for about 95 wt% to about 100 wt% of a bulk drug; in certain embodiments, the crystal form I of the compound of formula (A) accounts for about 98 wt% to about 100 wt% of a bulk drug; in certain embodiments, the crystal form I of the compound of formula (A) accounts for about 99 wt% to about 100 wt% of a bulk drug; and in certain embodiments, a bulk drug is basically composed of the crystal form I of the compound of formula (A) of the present invention, that is, the bulk drug is basically a phase-pure crystal.

The present invention further provides compounds of formula (II-A), formula (II-B) and formula (II-C) as shown below:

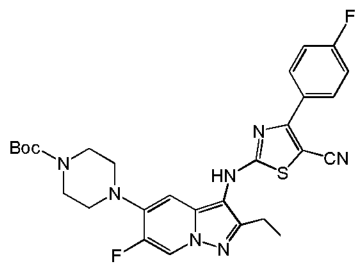
,
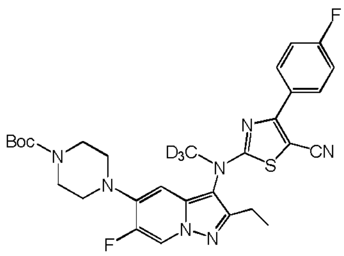
,
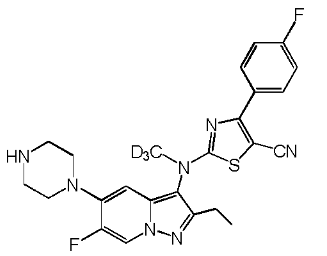

(II-A) (II-B) (II-C).

The compounds of formula (II-A), formula (II-B) and formula (II-C) are used as intermediates of the compound of formula (A).

The present invention further provides a method for preparing a compound of formula (A), wherein the method comprises the following steps:

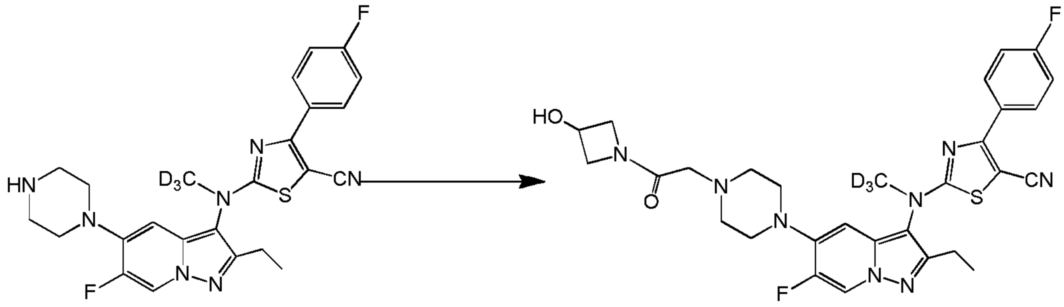

(II-C) (A)

dissolving a compound of formula (II-C) in an organic solvent A, and reacting the resulting solution with 2-chloro-1-(3-hydroxyazetidin-1-yl)ethanone and a base at 70°C-90°C to obtain the compound of formula (A).

In some embodiments, the above-mentioned preparation method involves dissolving a compound of formula (II-C) in an organic solvent A, and reacting the resulting solution with 2-chloro-1-(3-hydroxyazetidin-1-yl)ethanone and a base preferably at 72°C-88°C for 3-10 h, further preferably at 75°C-85°C for 4-8 h, and more preferably at 80°C for 6 h.

In some embodiments, according to the above-mentioned preparation method, the organic solvent A is selected from at least one of acetonitrile, 2-methyltetrahydrofuran, ethylbenzene, methyl and ethyl glyoxylate and diethylene glycol tert-butyl ether.

In some embodiments, according to the above-mentioned preparation method, the organic solvent A is selected from acetonitrile.

In some embodiments, according to the above-mentioned preparation method, the base is at least one of potassium carbonate and sodium carbonate.

In some embodiments, according to the above-mentioned preparation method, the base is potassium carbonate.

Further, the method for preparing the compound of formula (A) further comprises the following steps:

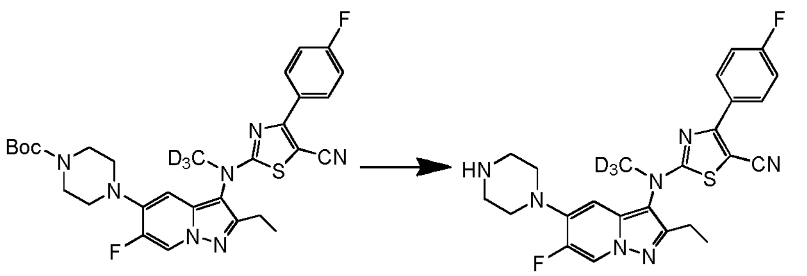

(II-B) (II-C)

dissolving the compound of formula (II-B) in an organic solvent C, adding trifluoroacetic acid, and carrying out a reaction to obtain the compound of formula (II-C).

Further, the method for preparing the compound of formula (A) further comprises the following steps:

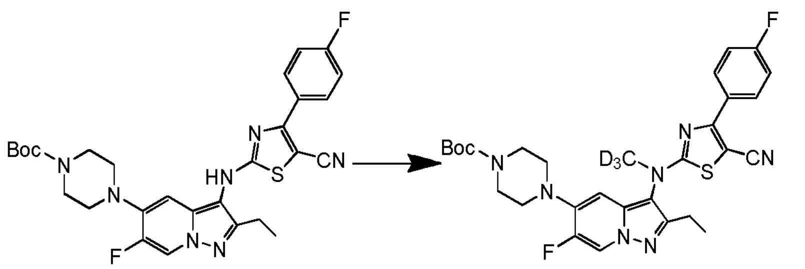

(II-A) (II-B)

dissolving the compound of formula (II-A) in an organic solvent B, cooling the resulting solution to 0°C under nitrogen protection, adding a reducing agent, and after a reaction is completed, adding methyl-$d_3$ iodide to obtain the compound

of formula (II-B).

Further, the method for preparing the compound of formula (A) further comprises the following steps:

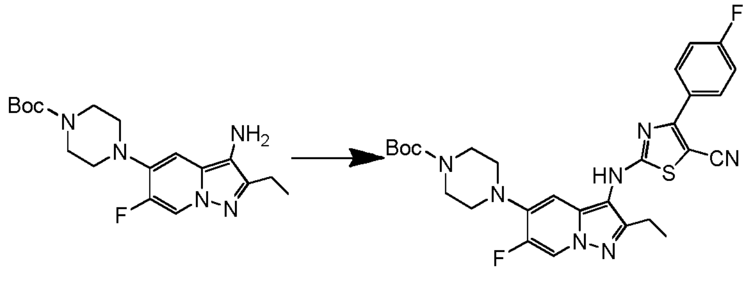

(II-D) (II-A)

reacting a compound of formula (II-D) with 2-chloro-4-(4-fluorophenyl)thiazole-5-cyano, 2,6-dimethylpyridine and an aprotic polar solvent at 60°C-80°C to obtain the compound of formula (II-A).

The method for preparing the compound of formula (A) of the present invention further comprises the following steps:

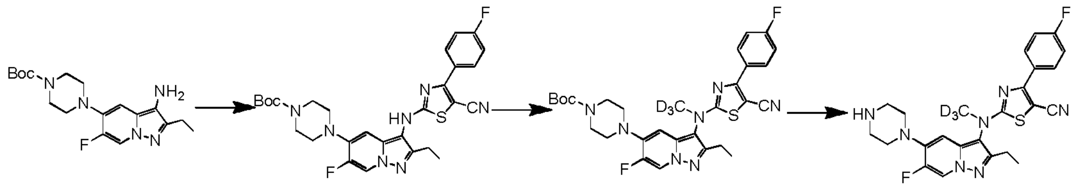

(II-D) (II-A) (II-B) (II-C)

i. reacting a compound of formula (II-D) with 2-chloro-4-(4-fluorophenyl)thiazole-5-cyano, 2,6-dimethylpyridine and an aprotic polar solvent at 60°C-80°C to obtain a compound of formula (II-A);
ii. dissolving the compound of formula (II-A) obtained in step i in an organic solvent B, cooling the resulting solution to 0°C under nitrogen protection, adding a reducing agent, and after a reaction is completed, adding methyl-$d_3$ iodide to obtain a compound of formula (II-B); and
iii. dissolving the compound of formula (II-B) obtained in step ii in an organic solvent C, adding trifluoroacetic acid, and carrying out a reaction to obtain the compound of formula (II-C).

The method for preparing the compound of formula (A) of the present invention comprises the following steps:

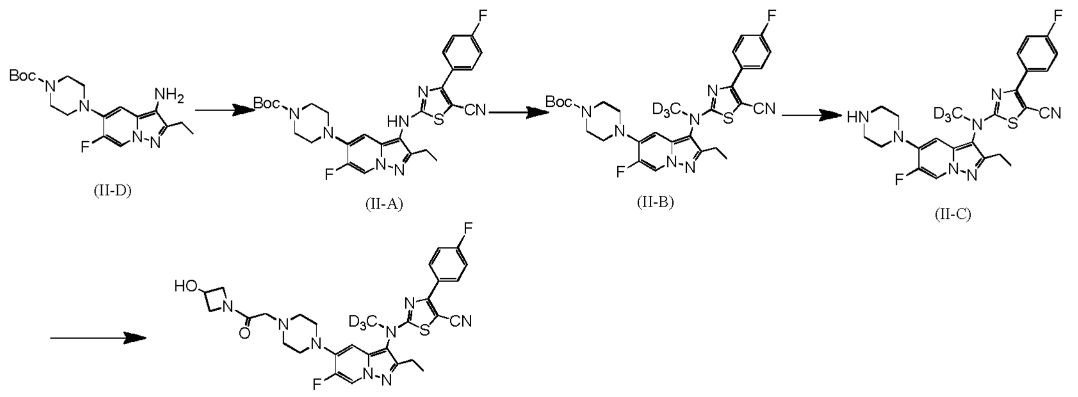

(II-D) (II-A) (II-B) (II-C)

(A)

.

Further, according to the above-mentioned preparation method, the aprotic polar solvent is at least one of N,N-dimethylacetamide, dimethylformamide, hexamethylphosphoramide, acetonitrile, acetone and dimethyl sulfoxide.

Furthermore, the aprotic polar solvent is N,N-dimethylacetamide.

Further, according to the above-mentioned preparation method, the organic solvent B is at least one of tetrahydrofuran, N,N-dimethylacetamide, dimethylformamide, acetonitrile and acetone.

Furthermore, the organic solvent B is tetrahydrofuran.

Further, according to the above-mentioned preparation method, the reducing agent is sodium hydride.

Further, according to the above-mentioned preparation method, the organic solvent C is at least one of dichloromethane, methyl acetate, dimethyl carbonate, propylene glycol methyl ether acetate and dimethyl nylon acid.

Furthermore, the organic solvent C is dichloromethane.

As another technical solution of the present invention, the present invention provides a pharmaceutical composition comprising the compound or the stereoisomer, solvate, prodrug, metabolite, pharmaceutically acceptable salt or eutectic thereof according to any one of the above-mentioned technical solutions, or the crystal form I of the compound of formula (A), and a pharmaceutically acceptable carrier and/or excipient.

As yet another technical solution of the present invention, the present invention provides the use of the compound or the stereoisomer, solvate, prodrug, metabolite, pharmaceutically acceptable salt or eutectic thereof according to any one of the above-mentioned technical solutions or the above-mentioned composition in the preparation of a drug for treating an ATX-mediated disease. The ATX-mediated disease is idiopathic pulmonary fibrosis.

It can be understood that, as is well known in the fields of thermogravimetric analysis (TGA) and differential scanning calorimetry (DSC), melting peak heights of a TGA curve and a DSC curve depend on many factors related to sample preparation and geometric shapes of instruments, while peak positions are relatively insensitive to experiment details. Therefore, in some embodiments, the crystallized compounds of the present invention have TGA and DSC patterns comprising characteristic peak positions, which have substantially the same properties as the TGA and DSC patterns provided in the drawings of the present invention, with an error tolerance of measured values within ± 5°C, which is generally required to be within ± 3°C.

It can be understood that the numerical values described and claimed in the present invention are approximate values. Changes in values may be attributed to device calibration, device errors, crystal purity, crystal size, sample size and other factors.

It can be understood that the crystal forms of the present invention are not limited to the characteristic patterns such as XRD, DSC, TGA, DVS and adsorption isotherm curve graphs which are completely identical to those described in the drawings disclosed by the present invention, and any crystal form having a characteristic pattern which is essentially or substantially the same as those described in the drawings falls within the scope of the present invention.

## Terms

The term "therapeutically effective amount" means an amount that causes a physiological or medical response in a tissue, system or subject and is a desirable amount, including the amount of a compound that is, when administered to a subject to be treated, sufficient to prevent occurrence of one or more symptoms of the disease or condition to be treated or to reduce the symptom(s) to a certain degree.

The term "room temperature" refers to: 10°C-30°C, > 30% RH.

The term "pharmaceutically acceptable salt" refers to a salt of the compound of the present invention, which salt maintains the biological effectiveness and characteristics of a free acid or a free base and is obtained by reacting the free acid with a non-toxic inorganic base or organic base, or reacting the free base with a non-toxic inorganic acid or organic acid.

The term "pharmaceutical composition" represents a mixture of one or more compounds or the stereoisomers, solvates, pharmaceutically acceptable salts, eutectics or deuterated products thereof of the present invention and other components comprising physiologically/pharmaceutically acceptable carriers and/or excipients.

The term "carrier" refers to a system that does not cause significant irritation to an organism and does not eliminate the biological activities and characteristics of the administered compound, and can change the way in which the drug enters the human body and the distribution of the drug in the body, control the release rate of the drug and delivery the drug to target organs. Non-limiting examples of carriers include a microcapsule, a microsphere, a nanoparticle, a liposome, etc.

The term "excipient" refers to a substance that is not a therapeutic agent per se, but used as a diluent, adjuvant, adhesive and/or vehicle for addition to a pharmaceutical composition, thereby improving the disposal or storage properties thereof, or allowing to or promoting the formation of a compound or a pharmaceutical composition into a unit dosage form for administration. As is known to a person skilled in the art, a pharmaceutically acceptable excipient can provide various functions and can be described as a wetting agent, a buffer, a suspending aid, a lubricant, an emulsifier, a disintegrating agent, an absorbent, a preservative, a surfactant, a colourant, a flavouring agent and a sweetening agent. Examples of pharmaceutically acceptable excipients include, but are not limited to: (1) sugars, such as lactose, glucose and sucrose; (2) starch, such as corn starch and potato starch; (3) cellulose and derivatives thereof, such as sodium carboxymethyl cellulose, ethyl cellulose, cellulose acetate, hydroxypropyl methylcellulose, hydroxypropyl cellulose, microcrystalline cellulose and croscarmellose (such as croscarmellose sodium); (4) tragacanth powder; (5) malt; (6) gelatine;

(7) talc; (8) excipients, such as cocoa butter or suppository wax; (9) oils, such as peanut oil, cottonseed oil, safflower oil, sesame oil, olive oil, corn oil and soybean oil; (10) diols, such as propylene glycol; (11) polyols, such as glycerol, sorbitol, mannitol and polyethylene glycol; (12) esters, such as ethyl oleate and ethyl laurate; (13) agar; (14) buffers, such as magnesium hydroxide and aluminium hydroxide; (15) alginic acid; (16) pyrogen-free water; (17) isotonic saline; (18) Ringer's solution; (19) ethanol; (20) pH buffered solution; (21) polyester, polycarbonate and/or polyanhydride; and (22) other non-toxic compatible substances used in a pharmaceutical preparation.

The term "stereoisomer" refers to an isomer produced as a result of different spatial arrangement of atoms in molecules, including cis-trans isomers, enantiomers and conformational isomers.

The term "solvate" refers to a substance formed by the compound of the present invention or the salt thereof and a stoichiometric or non-stoichiometric solvent bound by intermolecular non-covalent forces. When the solvent is water, the solvate is a hydrate.

The term "eutectic" refers to a crystal formed by the combination of an active pharmaceutical ingredient (API) and a co-crystal former (CCF) under the action of hydrogen bonds or other non-covalent bonds, wherein the pure states of API and CCF are both solid at room temperature, and there is a fixed stoichiometric ratio between the components. The eutectic is a multi-component crystal, which includes both a binary eutectic formed by means of two neutral solids and a multielement eutectic formed by means of a neutral solid and a salt or solvate.

Brief Description of the Drawings

Figure 1 shows an X-ray powder diffraction pattern of the crystal form I of compound A as determined by using Cu-K$\alpha$ radiation;
Figure 2 shows a TGA curve graph of the crystal form I of compound A;
Figure 3 shows a DSC curve graph of the crystal form I of compound A;
Figure 4 shows a PLM image of the crystal form I of compound A;
Figure 5 shows a DVS plot of the crystal form I of compound A.

Detailed Description of Embodiments

The content of the present invention is described in detail with reference to the following examples. If a specific condition is not indicated in the examples, a conventional condition is used in an experimental method. The listed examples are intended to better illustrate the content of the present invention, but should not be construed as limiting the content of the present invention. According to the above-mentioned content of the invention, a person skilled in the art can make unsubstantial modifications and adjustments to the embodiments, which still fall within the scope of protection of the present invention.

Unless otherwise specified, the raw materials are purchased from Titan Technology Co., Ltd., Energy Chemical Co., Ltd., Shanghai Demo Co., Ltd., Chengdu Kelong Chemical Co., Ltd., Accela ChemBio Co., Ltd., PharmaBlock Sciences (Nanjing), Inc., WuXi Apptec Co., Ltd., J&K Scientific Co., Ltd., etc.

Instrumental information and methods

The structures of the compounds are determined by nuclear magnetic resonance (NMR) or (and) mass spectrometry (MS). The NMR shift ($\delta$) is given in the unit of 10-6 (ppm). NMR is determined with Bruker Avance III 400 and Bruker Avance 300; the solvent for determination is deuterated dimethyl sulfoxide (DMSO-d6), deuterated chloroform ($CDCl_3$) and deuterated methanol ($CD_3OD$); and the internal standard is tetramethylsilane (TMS).

MS is measured with (Agilent 6120B (ESI) and Agilent 6120B (APCI));
HPLC determination is performed by using Agilent 1260DAD high pressure liquid chromatograph (Zorbax SB-C18 100 $\times$ 4.6 mm, 3.5 $\mu$M);
Yantai Huanghai HSGF254 or Qingdao GF254 silica gel plate is used as a silica plate for thin-layer chromatography, and the silica gel plate for the thin-layer chromatography (TLC) is of the specification of 0.15 mm-0.20 mm, and the specification used when a product is separated and purified product by means of thin-layer chromatography is 0.4 mm-0.5 mm.

For the column chromatography, 200-300 mesh Yantai Huanghai silica gel is generally used as a carrier.

X-ray powder diffraction

An XRPD pattern is obtained on an X-ray powder diffractometer produced by PANalytacal, and scanning parameters are as shown in the table below.

| Parameter | Set value |
|---|---|
| Model | X' Pert3 |
| X ray | Cu, Kα,<br>Kα1 (Å): 1.540598,<br>Kα2 (Å): 1.544426<br>Kα2/Kα1 intensity ratio: 0.50 |
| Settings of X-ray tube | 45 kV, 40 mA |
| Divergence slit | 1/8° |
| Scan mode | Continuous |
| Scan range (°2Theta) | 3-40 |
| Scanning time per step (s) | 46.7 |
| Step length of scanning (°2Theta) | 0.0263 |
| Test time | about 5 min |

Thermogravimetric analysis (TGA) and differential scanning calorimetry (DSC)

TGA and DSC patterns are obtained on a TA Q5000/5500 thermal gravimetric analyser and a TA 2500 differential scanning calorimeter, respectively, and test parameters are as shown in the table below.

| Parameter | TGA | DSC |
|---|---|---|
| Method | Linear temperature rise | Linear temperature rise |
| Sample tray | Aluminium tray, opened | Aluminium tray, capped/uncapped |
| Temperature range | Room temperature - set final temperature | 25°C - set final temperature |
| Scanning rate (°C/min) | 10 | 10 |
| Protective gas | Nitrogen gas | Nitrogen gas |

Dynamic vapor sorption (DVS)

A dynamic vapor sorption (DVS) curve is obtained on DVS Intrinsic of Surface Measurement Systems (SMS). The relative humidity at 25°C is corrected by using the deliquescence points of LiCl, $Mg(NO_3)_2$ and KCl. Test parameters for DVS are as shown in the table below.

| Parameter | Set value |
|---|---|
| Temperature | 25°C |
| Sample size | 10-20 mg |
| Protective gas and flow | $N_2$, 200 mL/min |
| dm/dt | 0.002%/min |
| Minimum dm/dt equilibration time | 10 min |
| Maximum equilibration time | 180 min |
| RH range | 0% RH-95% RH |

(continued)

| Parameter | Set value |
|---|---|
| RH gradient | 10% (0% RH - 90 %RH, 90% RH - 0% RH)<br>5% (90% RH - 95% RH, 95% RH - 90% RH) |

Polarized light microscope (PLM)

Data of polarized light microscope are collected by means of an Axio Lab. A1 upright microscope at room temperature.

Description of abbreviations:

NMP: N-methylpyrrolidone;
THF: tetrahydrofuran;
HATU: 2-(7-azabenzotriazole)-N,N,N',N'-tetramethyluronium hexafluorophosphate;
PE: petroleum ether;
EA: ethyl acetate;
DCM: dichloromethane;
MeOH: methanol;
Acetone: acetone;
EtOH: ethanol;
ACN: acetonitrile;
$H_2O$: water.

**Intermediate 1:**

(but-1-yn-1-yl-d5)trimethylsilane (**intermediate 1**)

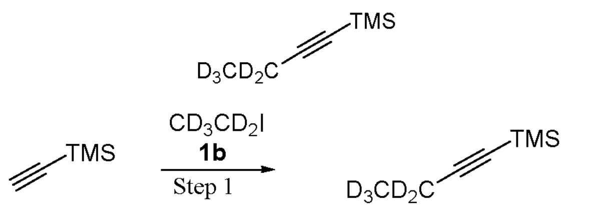


1a

Intermediate 1

Trimethylsilylacetylene (**1a**) (10.07 g, 102.5 mmol) was dissolved in dry tetrahydrofuran (45 mL) at room temperature and cooled to -78°C, and then n-butyllithium (45 mL, 2M n-hexane) was added dropwise. Upon completion of the dropwise addition, the resulting mixture was transferred to an ice-water bath, reacted for another 20 min, and then cooled to -78°C, and hexamethylphosphoramide (18.37 g, 102.5 mmol) was added dropwise and stirred for another 30 min under such condition. After that, ethyl-d5 iodide (**1b**) (15.00 g, 93.2 mmol) was added dropwise. Upon completion of the dropwise addition, the resulting mixture was naturally warmed to room temperature and reacted overnight. The reaction was quenched by adding water (20 mL) to the reaction liquid. Liquid separation was performed. The aqueous phase was extracted with ethyl ether (20 mL), and the organic phases were combined, washed with saturated brine (20 mL), dried over anhydrous sodium sulphate and filtered. The filtrate was distilled under normal pressure, and the fraction at 80°C to 100°C was collected to obtain the title compound (but-1-yn-1-yl-d5)trimethylsilane (**intermediate 1**) as a colourless transparent liquid (10.0 g, yield: 74%).

**Intermediate 2:**

2-chloro-1-(3-hydroxyazetidin-1-yl-3-d)ethan-1-one (**intermediate 2**)

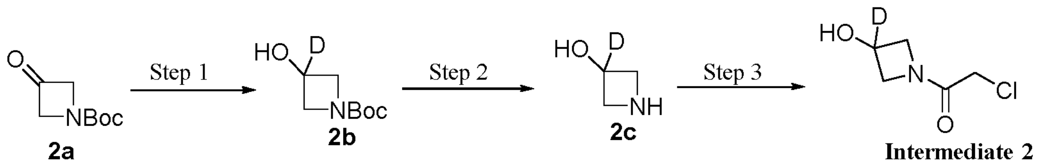

2a 2b 2c Intermediate 2

Step 1: tert-butyl 3-hydroxyazetidine-1-carboxylate-3-d (**2b**)

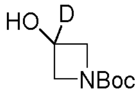

1-Boc-3-azetidinone (**2a**) (5.13 g, 30 mmol) was dissolved in tetrahydrofuran (50 mL) and stirred uniformly. Sodium borohydride-d4 (1.26 g, 30 mmol) was then added in portions, and the resulting mixture was reacted at room temperature for 1 h. The reaction was quenched by adding water (20 mL) and stirred for 10 min. Ethyl acetate (100 mL) and saturated sodium bicarbonate (100 mL) were then added, and extraction and layer separation were performed. The organic phases were washed with saturated brine (100 mL), dried over anhydrous sodium sulphate and filtered. The filtrate was concentrated under reduced pressure to obtain the target compound tert-butyl 3-hydroxyazetidine-1-carboxylate-3-d (**2b**) as a transparent oil (4.6 g, yield: 87%).

$^1H$ NMR (400 MHz, $CDCl_3$) $\delta$ 4.14 (d, 2H), 3.79 (d, 2H), 1.44 (s, 9H).

Step 2:

azetidin-3-d-3-ol (**2c**)

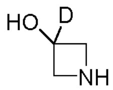

The compound tert-butyl 3-hydroxyazetidine-1-carboxylate-3-d (**2b**) (4.6 g, 26.3 mmol) was dissolved in dichloromethane (24 mL), and trifluoroacetic acid (8 mL) was added dropwise. Upon completion of the dropwise addition, the resulting mixture was reacted at room temperature for 1 h and concentrated under reduced pressure to obtain a trifluoroacetate of azetidin-3-d-3-ol (**2c**) as a brown oil (4.94 g, yield: 100%), and the crude product was directly used in the next reaction.

Step 3:

2-chloro-1-(3-hydroxyazetidin-1-yl-3-d)ethan-1-one (**intermediate 2**)

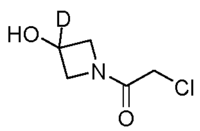

The trifluoroacetate (4.94 g, 26.3 mmol) of azetidin-3-d-3-ol (**2c**) was dissolved in water (40 mL), and stirred uniformly. Potassium carbonate (12.4 g, 60 mmol) was then added in portions, and after the system stopped bubbling, dichloromethane (40 mL) was added. Chloroacetyl chloride (3.39 g, 30 mmol) was added dropwise under an ice bath condition. Upon completion of the dropwise addition, the resulting mixture was reacted at room temperature for 2 h. Ethyl acetate (100 mL) and water (100 mL) were added, and extraction and layer separation were performed. The

aqueous phase was extracted with ethyl acetate (100 mL), and the organic phases were combined, washed with saturated brine (100 mL), dried over anhydrous sodium sulphate and filtered. The filtrate was concentrated under reduced pressure, and the residue was separated by column chromatography on silica gel to obtain the target compound 2-chloro-1-(3-hydroxyazetidin-1-yl-3-d)ethan-1-one (**intermediate 2**) as a white solid (0.85 g, yield: 21%).

$^{1}H$ NMR (400 MHz, $CDCl_3$) δ 4.49 (d, 1H), 4.30 (d, 1H), 4.15 (d, 1H), 3.94 (d, 1H), 3.90 (s, 2H), 3.01 (s, 1H).

**Intermediate 3:**

ethyl 2-bromoacetate-d2 (**intermediate 3**)

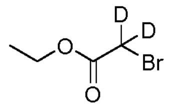

Intermediate 3

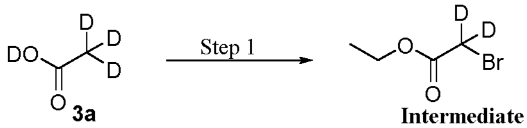


3a

Intermediate 3

Acetic acid-d4 (**3a**) (1.0 g, 15.6 mmol) was dissolved in trifluoroacetic anhydride (6 mL) and stirred uniformly. After that, 4-dimethylaminopyridine (30 mg, 0.25 mmol) was added and heated to 60°C, and bromine (0.98 mL) was slowly added dropwise under such condition for 1 h. Upon completion of the dropwise addition, the resulting mixture was reacted under such condition for 1 h and then cooled to room temperature, and nitrogen gas was introduced until the volume of the system was reduced to about 1 mL. Ethanol (20 mL) was added and reacted at 60°C overnight. The resultant was then cooled to room temperature, and concentrated under reduced pressure. Ethyl ether (100 mL) was added, and the resulting mixture was washed with saturated brine (100 mL), dried over anhydrous sodium sulphate and filtered. The filtrate was concentrated under reduced pressure to obtain ethyl 2-bromoacetate-d2 (**intermediate 3**) as a pale yellow liquid (1.65 g, yield: 62.6%).

$^{1}H$ NMR (400 MHz, $CDCl_3$) δ 4.24 (q, $J$ = 7.1 Hz, 1H), 1.30 (t, $J$ = 7.1 Hz, 2H).

**Intermediate 4:**

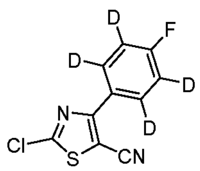

Intermediate 4

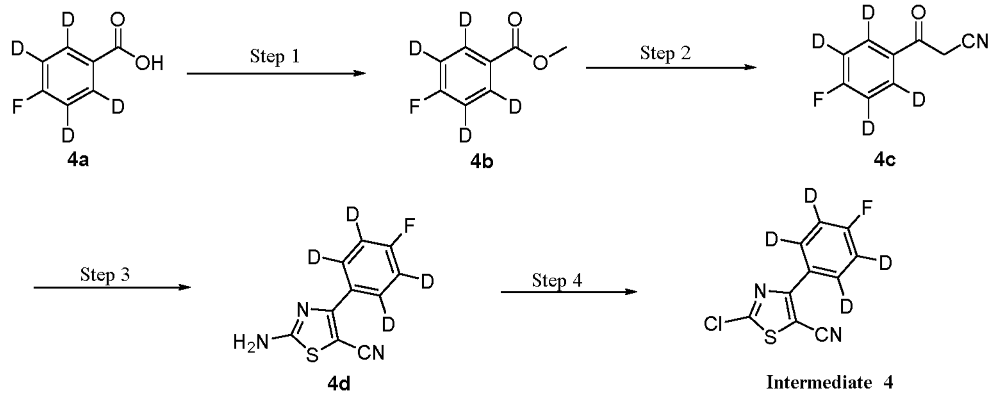


4a 4b 4c 4d Intermediate 4

Step 1:

methyl 4-fluorobenzoate-2,3,5,6-d4 (**4b**)

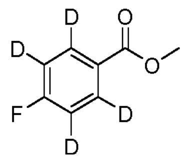

4-fluorobenzoic acid-2,3,5,6-d4 (**4a**) (1.0 g, 6.94 mmol) was dissolved in N,N-dimethylformamide (10 mL) and cooled to 0°C. Potassium carbonate (1.92 g, 13.9 mmol) was added and reacted under such condition for 5 min. Dimethyl sulphate (1.05 g, 8.33 mmol) was then added dropwise. Upon completion of the addition, the resulting mixture was reacted at room temperature for 1 h. The reaction was quenched by adding water (20 mL) and extracted with ethyl acetate (50 mL × 2). The organic phases were combined, washed with saturated brine (50 mL × 1), dried over anhydrous sodium sulphate and filtered. The filtrate was concentrated under reduced pressure, and the residue was separated by column chromatography on silica gel (petroleum ether : ethyl acetate (v/v) = 8 : 1) to obtain the target compound methyl 4-fluorobenzoate-2,3,5,6-d4 (**4b**) as a transparent liquid (0.96 g, yield: 87%).

$^1$H NMR (400 MHz, $CDCl_3$) $\delta$ 3.91 (s, 3H).

Step 2:

3-(4-fluorophenyl-2,3,5,6-d4)-3-oxopropanenitrile (**4c**)

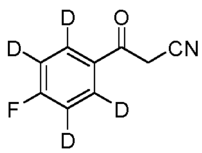

Methyl 4-fluorobenzoate-2,3,5,6-d4 (**4b**) (0.96 g, 6.1 mmol) was dissolved in methylbenzene (8 mL) and cooled to -10°C under nitrogen protection. Acetonitrile (1.46 g, 36.3 mmol) was added, and sodium bis(trimethylsilyl)amide (6 mL, 12 mmol, 2M in THF) was slowly added dropwise. Upon completion of the dropwise addition, the reaction was quenched with hydrochloric acid (20 mL, 1N aqueous solution). The reaction product was warmed to room temperature and extracted with ethyl acetate (50 mL × 2). The organic phases were combined, washed with saturated brine (50 mL × 1), dried over anhydrous sodium sulphate and filtered. The filtrate was concentrated under reduced pressure to obtain the target compound 3-(4-fluorophenyl-2,3,5,6-d4)-3-oxopropanenitrile (**4c**) as a pale yellow solid (0.92 g, yield: 91%).

$^1$H NMR (400 MHz, $CDCl_3$) $\delta$ 4.05 (s, 2H).

Step 3:

2-amino-4-(4-fluorophenyl-2,3,5,6-d4)thiazole-5-carbonitrile (**4d**)

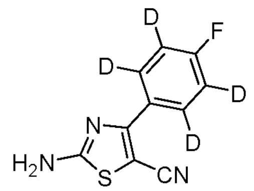

3-(4-fluorophenyl-2,3,5,6-d4)-3-oxopropanenitrile (**4c**) (0.92 g, 5.5 mmol) was dissolved in ethanol (12 mL). Pyridine (0.44 g, 5.5 mmol) was added, and then the resulting mixture was warmed to 70°C, reacted for 15 min and cooled to room temperature, and a suspension (8 mL) of thiourea (0.84 g, 11 mmol) and iodine (1.4 g, 5.5 mmol) in ethanol was slowly added dropwise. Upon completion of the dropwise addition, the resulting mixture was reacted at room temperature for 1 h. The reaction was quenched by adding sodium thiosulphate (10 mL, 1N aqueous solution) and filtered. The filter cake was dried to obtain the target compound 2-amino-4-(4-fluorophenyl-2,3,5,6-d4)thiazole-5-carbonitrile (**4d**) as a white solid (0.82 g, yield: 67%).

Step 4:

2-chloro-4-(4-fluorophenyl-2,3,5,6-d4)thiazole-5-carbonitrile (**intermediate 4**)

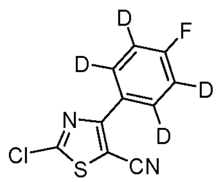

Copper chloride (0.60 g, 4.5 mmol) was dissolved in acetonitrile (8 mL), tert-butyl nitrite (0.67 g, 5.6 mmol) was added dropwise, and the mixture was stirred at room temperature for 30 min. After that, 2-amino-4-(4-fluorophenyl-2,3,5,6-d4)thiazole-5-carbonitrile (**4d**) (0.82 g, 3.67 mmol) was added in portions. Upon completion of the addition, the resulting mixture was reacted at room temperature for another 1 h. The reaction was quenched with hydrochloric acid (10 mL, 1N aqueous solution). Ethyl acetate (80 mL) was added, and the resulting mixture was washed with saturated brine (50 mL), dried over anhydrous sodium sulphate and filtered. The filtrate was concentrated under reduced pressure, and the residue was separated by column chromatography on silica gel (dichloromethane) to obtain the target compound 2-chloro-4-(4-fluorophenyl-2,3,5,6-d4)thiazole-5-carbonitrile (**intermediate 4**) as a pale yellow solid (0.68 g, yield: 76%).

**Example 1:**

2-((2-(ethyl-d5)-6-fluoro-5-(4-(2-(3-hydroxyazetidin-1-yl)-2-oxoethyl)piperazin-1-yl)pyrazolo[1,5-a]pyridin-3-yl)(methyl)amino)-4-(4-fluorophenyl)thiazole-5-carbonitrile (**compound 1**)

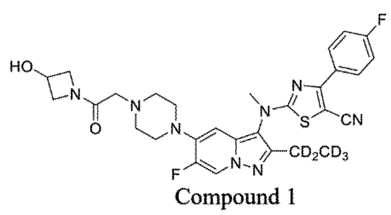

Compound 1

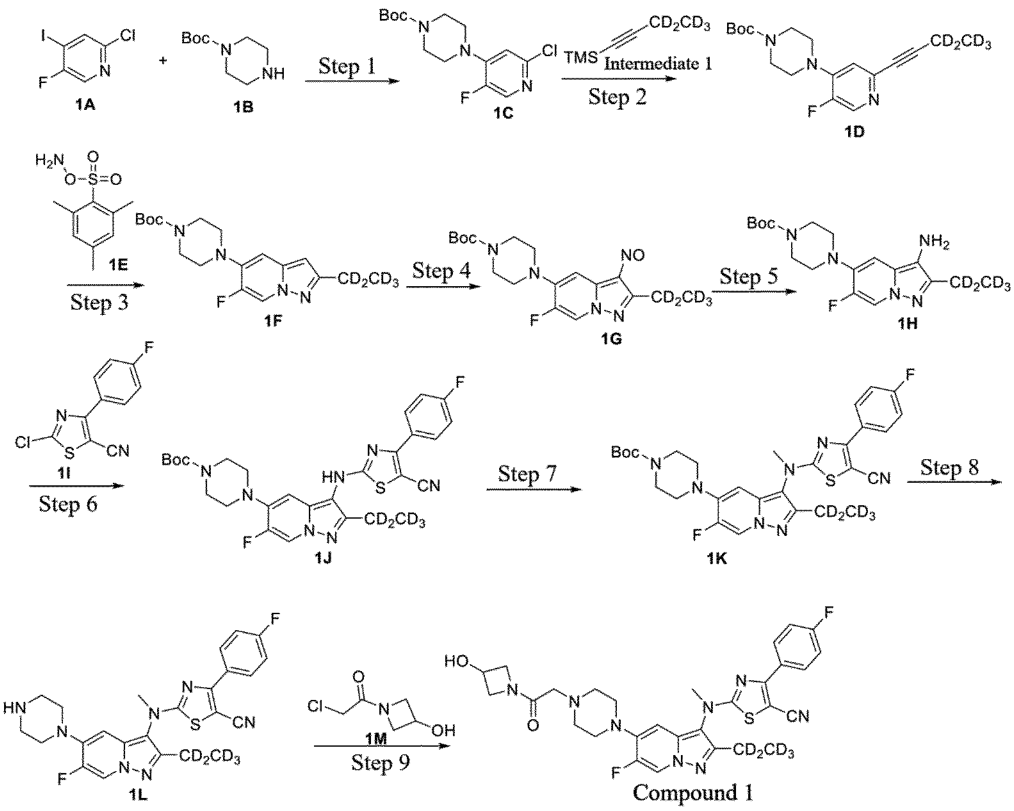


Step 1:

tert-butyl 4-(2-chloro-5-fluoropyridin-4-yl)piperazine-1-carboxylate (**1C**)

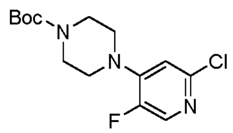

NMP (220 mL) was added to a 500 mL reaction flask and stirred, and then 2-chloro-5-fluoro-4-iodopyridine (**1A**) (44.00 g, 170.9 mmol), 1-Boc-piperazine (**1B**) (47.75 g, 256.4 mmol) and potassium carbonate (47.24 g, 341.8 mmol) were added successively. The resulting mixture was warmed to 150°C and reacted for 3 h, and then the reaction stopped. The reaction liquid was cooled to room temperature, the reactant was slowly added to ice water (500 mL), and a white solid was precipitated out. Stirring was performed for 0.5 h, and then filtration was performed. The filter cake was washed with water, slurried with n-hexane (200 mL), filtered and dried to obtain the target compound tert-butyl 4-(2-chloro-5-fluoropyridin-4-yl)piperazine-1-carboxylate (**1C**) as a white solid (50 g, yield: 64%).
LCMS m/z =316.2[M +1]$^+$.

Step 2:

tert-butyl 4-(2-(but-1-yn-1-yl-d5)-5-fluoropyridin-4-yl)piperazine-1-carboxylate (**1D**)

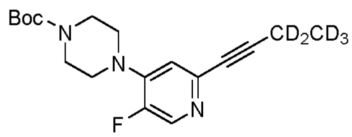

Tert-butyl 4-(2-chloro-5-fluoropyridin-4-yl)piperazine-1-carboxylate (**1C**) (23.18 g, 71.6 mmo), (but-1-yn-1-yl-d5)trimethylsilane (**intermediate 1**) (9.4 g, 131.3 mmol), bistriphenylphosphine palladium dichloride (5.03 g, 7.2 mmol), 1,3-bis(diphenylphosphino)propane (4.43 g, 10.7 mmol) and caesium fluoride (22.8 g, 143.3 mmol) were successively added to dimethyl sulfoxide (184 mL). The system was subjected to nitrogen replacement three times and reacted at 95°C for 4 h. After the reaction was completed, the reaction product was cooled to room temperature, and water (200 mL) was added. The aqueous phase was extracted with ethyl acetate (200 mL × 3). The organic phases were combined, washed with a saturated aqueous sodium chloride solution (200 mL), dried over anhydrous sodium sulphate and filtered. The filtrate was concentrated under reduced pressure. The residue was separated by column chromatography on silica gel (PE : EA = 20 : 1 - 5 : 1) to obtain tert-butyl 4-(2-(but-1-yn-1-yl-d5)-5-fluoropyridin-4-yl)piperazine-1-carboxylate (**1D**) as a pale brown viscous liquid (10.15 g, yield: 41.9%).

LCMS m/z =339.2[M +1]$^+$.

Step 3:

tert-butyl 4-(2-(ethyl-d5)-6-fluoropyrazolo[1,5-a]pyridin-5-yl)piperazine-1-carboxylate (**1F**)

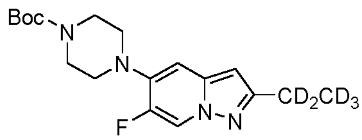

Ethanol (100 mL) and tert-butyl 4-(2-(but-1-yn-1-yl-d5)-5-fluoropyridin-4-yl)piperazine-1-carboxylate (**1D**) (10.15 g, 30.0 mmol) were added to a 250 mL reaction flask and cooled to 0°C. 2-[(aminooxy)sulfonyl]-1,3,5-trimethylbenzene (**1E**) (11.00 g, 51.1 mmol) was added in portions, and then sodium bicarbonate (5.04 g, 60.0 mmol) was added to the reaction. Upon completion of the addition, the mixture was reacted at room temperature for 2 h. After that, potassium carbonate (8.29 g, 60.0 mmol) was added to the reaction and reacted at room temperature overnight. Water (150 mL) was added to the reaction liquid, and the aqueous phase was extracted with ethyl acetate (200 mL × 2). The organic phases were combined, washed with saturated sodium chloride (100 mL), dried over anhydrous sodium sulphate and filtered. The filtrate was concentrated under reduced pressure, and the residue was separated and purified by column chromatography on silica gel to obtain the target compound tert-butyl 4-(2-(ethyl-d5)-6-fluoropyrazolo[1,5-a]pyridin-5-yl)piperazine-1-carboxylate (**1F**) as a white solid (7.53 g, yield: 71%).

LCMS m/z =354.3 [M +1]$^+$.

Step 4:

tert-butyl 4-(2-(ethyl-d5)-6-fluoro-3-nitrosopyrazolo[1,5-a]pyridin-5-yl)piperazine-1-carboxylate (**1G**)

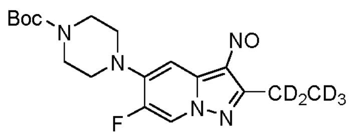

Tert-butyl 4-(2-(ethyl-d5)-6-fluoropyrazolo[1,5-a]pyridin-5-yl)piperazine-1-carboxylate (**1F**) (7.50 g, 21.2 mmol) was dissolved in methanol (37.5 mL) and acetic acid (7.5 mL), and the resulting solution was cooled to 5°C. After that, sodium nitrite (2.93 g, 42.4 mmol) was dissolved in water (10 mL) and then added dropwise to the reaction liquid. Upon completion of the addition, the resulting mixture was reacted at room temperature for 16 h. Water (30 mL) was added

dropwise to the reaction liquid. Upon completion of the addition, filtration was performed. The filter cake was washed with water (10 mL × 2) and dried to obtain a crude product of the target compound tert-butyl 4-(2-(ethyl-d5)-6-fluoro-3-nitrosopyrazolo[1,5-a]pyridin-5-yl)piperazine-1-carboxylate (**1G**) as a dark green solid (11.5 g).

LCMS m/z =383.3 [M +1]+.

Step 5:

tert-butyl 4-(3-amino-2-(ethyl-d5)-6-fluoropyrazolo[1,5-a]pyridin-5-yl)piperazine-1-carboxylate (**1H**)

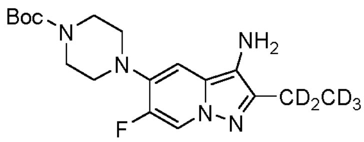

Tert-butyl 4-(2-(ethyl-d5)-6-fluoro-3-nitrosopyrazolo[1,5-a]pyridin-5-yl)piperazine-1-carboxylate (**1G**) (11.5 g, crude) was dissolved in ethanol (40 mL) and water (20 mL). After that, ammonium chloride (15.78 g, 295 mmol) and iron powder (8.23 g, 147 mmol) were added to the reaction, reacted at 65°C for 20 min and filtered. The filtrate was extracted with dichloromethane (50 mL × 3). The organic phases were combined, washed with saturated brine (100 mL × 1), dried over anhydrous sodium sulphate and filtered. The filtrate was concentrated under reduced pressure to obtain a crude product of the target compound tert-butyl 4-(3-amino-2-(ethyl-d5)-6-fluoropyrazolo[1,5-a]pyridin-5-yl)piperazine-1-carboxylate (**1H**) as a yellow solid (7.85 g).

LCMS m/z =369.2[M +1]+.

Step 6:

tert-butyl 4-(3-((5-cyano-4-(4-fluorophenyl)thiazol-2-yl)amino)-2-(ethyl-d5)-6-fluoropyrazolo[1,5-a]pyridin-5-yl)piperazine-1-carboxylate (**1J**)

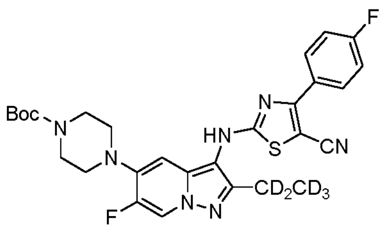

Tert-butyl 4-(3-amino-2-(ethyl-d5)-6-fluoropyrazolo[1,5-a]pyridin-5-yl)piperazine-1-carboxylate (**1H**) (7.85 g, 21.3 mmol), 2-chloro-4-(4-fluorophenyl)thiazole-5-cyano (**1I**) (6.1 g, 25.6 mmol) (reference document: J. Med. Chem. 2017, 60, 3580-3590, DOI: 10.1021/acs.jmedchem.7b00032), 2,6-dimethylpyridine (3.42 g, 31.9 mmol) and N,N-dimethylacetamide (40 mL) were added. The resulting mixture was warmed to 70°C and then reacted for 4 h. The reaction was quenched by adding a 10% sodium chloride solution (100 mL) and extracted with ethyl acetate (150 mL × 3). The organic phases were combined, washed with saturated brine (100 mL × 1), dried over anhydrous sodium sulphate and filtered. The filtrate was concentrated under reduced pressure to obtain a crude product of the target compound tert-butyl 4-(3-((5-cyano-4-(4-fluorophenyl)thiazol-2-yl)amino)-2-(ethyl-d5)-6-fluoropyrazolo[1,5-a]pyridin-5-yl)piperazine-1-carboxylate (**1J**) as a yellowish-brown viscous substance (15.50 g).

LCMS m/z =571.2[M +1]+.

Step 7:

tert-butyl 4-(3-((5-cyano-4-(4-fluorophenyl)thiazol-2-yl)(methyl)amino)-2-ethyl-6-fluoropyrazolo[1,5-a]pyridin-5-yl)piperazine-1-carboxylate (**1K**)

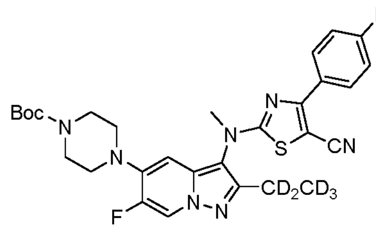

The crude product (15.50 g) of tert-butyl 4-(3-((5-cyano-4-(4-fluorophenyl)thiazol-2-yl)amino)-2-(ethyl-d5)-6-fluoropyrazolo[1,5-a]pyridin-5-yl)piperazine-1-carboxylate (**1J**) was dissolved in tetrahydrofuran (60 mL) and cooled to 0°C under nitrogen protection. Sodium hydride (1.3 g, 32.6 mmol, 60 wt%) was added in portions. Upon completion of the addition, the mixture was reacted under such condition for 10 min, and then methyl iodide (4.63 g, 32.6 mmol) was added dropwise. Upon completion of the addition, the resulting mixture was reacted at room temperature for 30 min. The reaction was quenched by adding water (100 mL) and extracted with ethyl acetate (100 mL × 2). The organic phases were combined, washed with saturated brine (100 mL × 1), dried over anhydrous sodium sulphate and filtered. The filtrate was concentrated under reduced pressure, and the residue was separated by column chromatography on silica gel to obtain the target compound tert-butyl 4-(3-((5-cyano-4-(4-fluorophenyl)thiazol-2-yl)(methyl)amino)-2-ethyl-6-fluoropyrazolo[1,5-a]pyridin-5-yl)piperazine-1-carboxylate (**1K**) as a yellow solid (11.56 g, four-step yield: 93%).

Step 8:

2-((2-(ethyl-d5)-6-fluoro-5-(piperazin-1-yl)pyrazolo[1,5-a]pyridin-3-yl)(methyl)amino)-4-(4-fluorophenyl)thiazole-5-carbonitrile hydrochloride (**1L**)

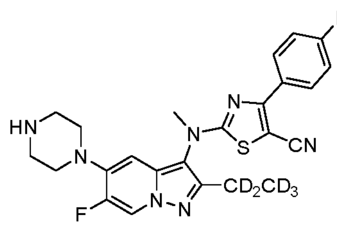

The compound tert-butyl 4-(3-((5-cyano-4-(4-fluorophenyl)thiazol-2-yl)(methyl)amino)-2-ethyl-6-fluoropyrazolo[1,5-a]pyridin-5-yl)piperazine-1-carboxylate (**1K**) (11.56 g, 19.8 mmol) was dissolved in dichloromethane (110 mL), and trifluoroacetic acid (35 mL) was added dropwise. The resulting mixture was reacted for about 3 h and then concentrated under reduced pressure. Dichloromethane (200 mL) was added to the residue, and the pH was adjusted to 8 with saturated sodium bicarbonate. Liquid separation was performed. The aqueous phase was extracted with dichloromethane (200 mL × 2). The organic phases were combined, washed with saturated brine (300 mL × 1), dried over anhydrous sodium sulphate and filtered. The filtrate was concentrated under reduced pressure to obtain the target compound 2-((2-(ethyl-d5)-6-fluoro-5-(piperazin-1-yl)pyrazolo[1,5-a]pyridin-3-yl)(methyl)amino)-4-(4-fluorophenyl)thiazole-5-carbonitrile (**1L**) as a yellow solid (10.7 g).

Step 9:

2-((2-(ethyl-d5)-6-fluoro-5-(4-(2-(3-hydroxyazetidin-1-yl)-2-oxoethyl)piperazin-1-yl)pyrazolo[1,5-a]pyridin-3-yl)(methyl)amino)-4-(4-fluorophenyl)thiazole-5-carbonitrile (**compound 1**)

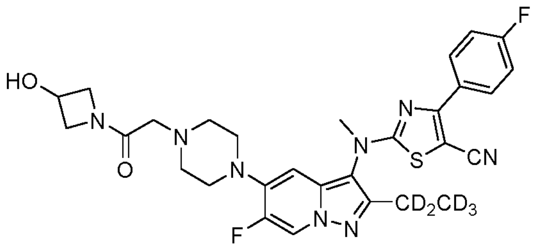

2-((2-(ethyl-d5)-6-fluoro-5-(piperazin-1-yl)pyrazolo[1,5-a]pyridin-3-yl)(methyl)amino)-4-(4-fluorophenyl)thiazole-5-carbonitrile (**1L**) (10.7 g) was dissolved in acetonitrile (100 mL), and 2-chloro-1-(3-hydroxyazetidin-1-yl)ethanone (**1M**) (3.63 g, 24.3 mmol, synthesized by the method in reference document: J. Med. Chem. 2017, 60, 3580-3590) and potassium carbonate (6.11 g, 44.2 mmol) were successively added. The resulting mixture was warmed to 80°C and then reacted overnight. The reaction was quenched by adding water (100 mL) and extracted with ethyl acetate (150 mL × 3). The organic phases were combined, washed with saturated brine (100 mL × 1), dried over anhydrous sodium sulphate and filtered. The filtrate was concentrated under reduced pressure, and the residue was separated by column chromatography on silica gel (dichloromethane/methanol = 20 : 1) to obtain the title compound 2-((2-(ethyl-d5)-6-fluoro-5-(4-(2-(3-hydroxyazetidin-1-yl)-2-oxoethyl)piperazin-1-yl)pyrazolo[1,5-a]pyridin-3-yl)(methyl)amino)-4-(4-fluorophenyl)thiazole-5-carbonitrile (**compound 1**) (6.2 g, two-step yield: 53%).

$^1H$ NMR (400 MHz, $CDCl_3$) δ 8.27 (d, 1H), 8.18-8.10 (m, 2H), 7.20-7.11 (m, 2H), 6.45 (d, 1H), 4.67 (s, 1H), 4.50-4.40 (m, 1H), 4.32-4.23 (m, 1H), 4.15-4.07 (m, 1H), 3.93-3.85 (m, 1H), 3.58 (s, 3H), 3.30-3.20 m, 4H), 3.19-3.06 (m, 2H), 2.85-2.67 (m, 5H).

LCMS m/z =598.1[M + 1t.

**Example 2:**

2-((2-(ethyl-d5)-6-fluoro-5-(4-(2-(3-hydroxyazetidin-1-yl)-2-oxoethyl)piperazin-1-yl)pyrazolo[1,5-a]pyridin-3-yl)(methyl-d3)amino)-4-(4-fluorophenyl)thiazole-5-carbonitrile (**compound 2**)

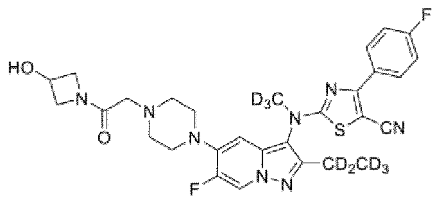

Compound 2

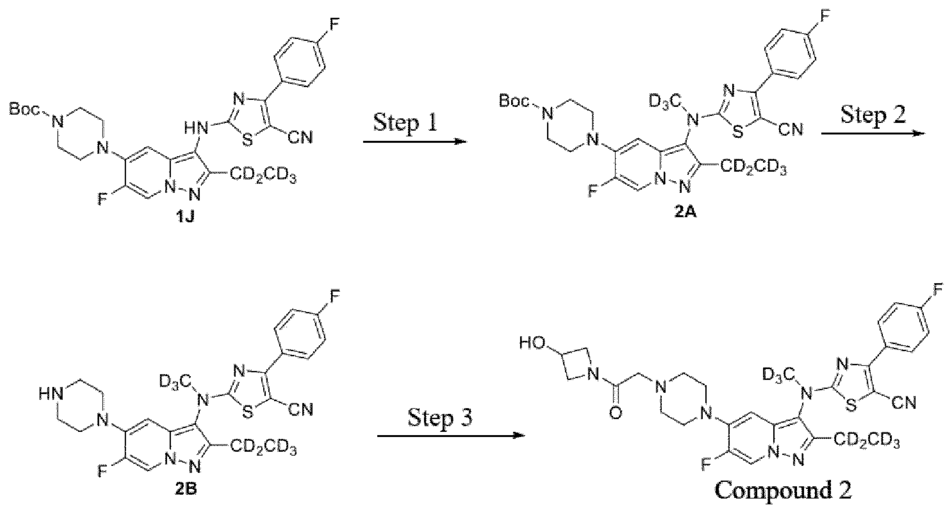


1J 2A 2B Compound 2

Step 1:

tert-butyl 4-(3-((5-cyano-4-(4-fluorophenyl)thiazol-2-yl)(methyl-d3)amino)-2-(ethyl-d5)-6-fluoropyrazolo[1,5-a]pyridin-5-yl)piperazine-1-carboxylate (**2A**)

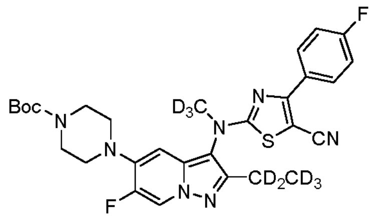

The crude product (9.2 g, 16.1 mmol) of tert-butyl 4-(3-((5-cyano-4-(4-fluorophenyl)thiazol-2-yl)amino)-2-(ethyl-d5)-6-fluoropyrazolo[1,5-a]pyridin-5-yl)piperazine-1-carboxylate (**1J**) was dissolved in tetrahydrofuran (60 mL) and cooled to 0°C under nitrogen protection. Sodium hydride (0.77 g, 19.3 mmol, 60 wt%) was added in portions. Upon completion of the addition, the resulting mixture was reacted under such condition for 10 min, and then methyl-d3 iodide (2.80 g, 19.3 mmol) was added dropwise. Upon completion of the addition, the resulting mixture was reacted at room temperature for 30 min. The reaction was quenched by adding water (100 mL) and extracted with ethyl acetate (100 mL × 2). The organic phases were combined, washed with saturated brine (100 mL × 1), dried over anhydrous sodium sulphate and filtered. The filtrate was concentrated under reduced pressure, and the residue was separated by column chromatography on silica gel to obtain the target compound tert-butyl 4-(3-((5-cyano-4-(4-fluorophenyl)thiazol-2-yl)(methyl-d3)amino)-2-(ethyl-d5)-6-fluoropyrazolo[1,5-a]pyridin-5-yl)piperazine-1-carboxylate (**2A**) as a yellow solid (8.0 g, yield: 84.5%).

Step 2:

2-((2-(ethyl-d5)-6-fluoro-5-(piperazin-1-yl)pyrazolo[1,5-a]pyridin-3-yl)(methyl-d3)amino)-4-(4-fluorophenyl)thiazole-5-carbonitrile (**2B**)

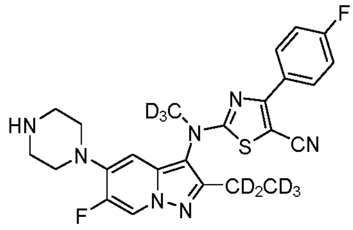

The compound tert-butyl 4-(3-((5-cyano-4-(4-fluorophenyl)thiazol-2-yl)(methyl-d3)amino)-2-(ethyl-d5)-6-fluoropyrazolo[1,5-a]pyridin-5-yl)piperazine-1-carboxylate (**2A**) (8.0 g, 13.6 mmol) was dissolved in dichloromethane (90 mL), and trifluoroacetic acid (30 mL) was added dropwise. The resulting mixture was reacted for about 3 h and then concentrated under reduced pressure. Dichloromethane (100 mL) was added to the residue, and the pH was adjusted to 8 with saturated sodium bicarbonate. Liquid separation was performed. The aqueous phase was extracted with dichloromethane (100 mL × 3). The organic phases were combined, washed with saturated brine (100 mL × 1), dried over anhydrous sodium sulphate and filtered. The filtrate was concentrated under reduced pressure to obtain the target compound 2-((2-(ethyl-d5)-6-fluoro-5-(piperazin-1-yl)pyrazolo[1,5-a]pyridin-3-yl)(methyl-d3)amino)-4-(4-fluorophenyl)thiazole-5-carbonitrile (**2B**) as a yellow solid (6.40 g, yield: 96.3%).

Step 3:

2-((2-(ethyl-d5)-6-fluoro-5-(4-(2-(3-hydroxyazetidin-1-yl)-2-oxoethyl)piperazin-1-yl)pyrazolo[1,5-a]pyridin-3-yl)(methyl-d3)amino)-4-(4-fluorophenyl)thiazole-5-carbonitrile (**compound 2**)

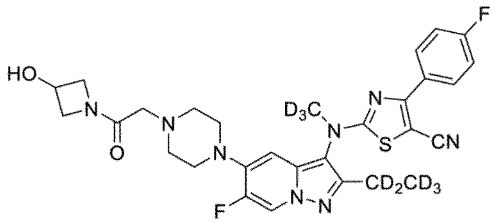

Compound 2

2-((2-(ethyl-d5)-6-fluoro-5-(piperazin-1-yl)pyrazolo[1,5-a]pyridin-3-yl)(methyl-d3)amino)-4-(4-fluorophenyl)thiazole-5-carbonitrile (**2B**) (6.40 g, 13.1 mmol) was dissolved in acetonitrile (100 mL). 2-chloro-1-(3-hydroxyazetidin-1-yl)ethanone (**1M**) (2.16 g, 14.4 mmol) and potassium carbonate (3.62 g, 26.2 mmol) were successively added, and the resulting mixture was warmed to 80°C and reacted for 5 h. The reaction was quenched by adding water (60 mL) and extracted with ethyl acetate (100 mL × 3). The organic phases were combined, washed with saturated brine (100 mL × 1), dried over anhydrous sodium sulphate and filtered. The filtrate was concentrated under reduced pressure, and the residue was separated by column chromatography on silica gel (dichloromethane/methanol = 20 : 1) to obtain the title compound 2-((2-(ethyl-d5)-6-fluoro-5-(4-(2-(3-hydroxyazetidin-1-yl)-2-oxoethyl)piperazin-1-yl)pyrazolo[1,5-a]pyridin-3-yl)(methyl-d3)amino)-4-(4-fluorophenyl)thiazole-5-carbonitrile (**compound 2**) (5.1 g, two-step yield: 64.8%).

$^1$H NMR (400 MHz, $CDCl_3$) δ 8.27 (d, 1H), 8.20-8.11 (m, 2H), 7.20-7.11 (m, 2H), 6.45 (d, 1H), 4.67 (s, 1H), 4.50-4.40( m, 1H), 4.32-4.24 (m, 1H), 4.15-4.07 (m, 1H), 3.94-3.86 (m, 1H), 3.31-3.06 (m, 6H), 2.86-2.66 (m, 5H).

LCMS m/z = 601.2[M +1]$^+$.

**Example 3:**

2-((2-ethyl-6-fluoro-5-(4-(2-(3-hydroxyazetidin-1-yl)-2-oxoethyl)piperazin-1-yl)pyrazolo[1,5-a]pyridin-3-yl)(methyl-d3)amino)-4-(4-fluorophenyl)thiazole-5-carbonitrile (**compound 3**)

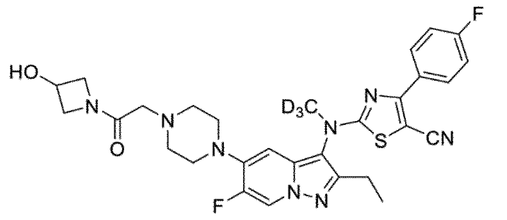

Compound 3

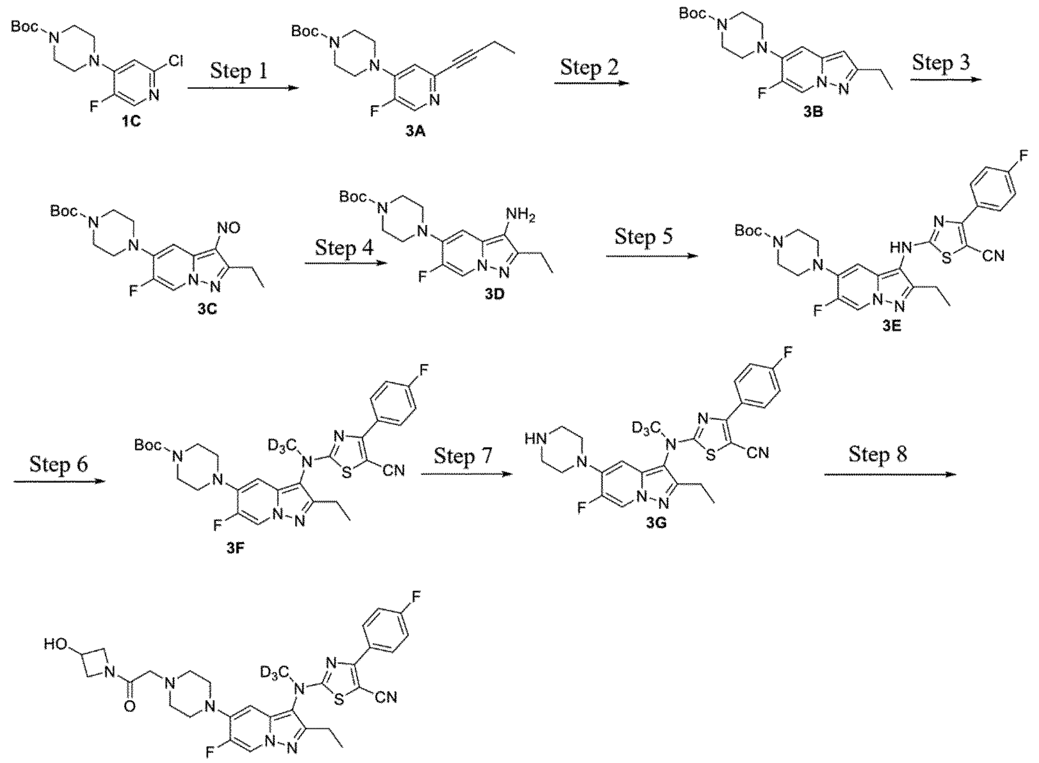


Compound 3

Step 1:

tert-butyl 4-(2-(but-1-yn-1-yl)-5-fluoropyridin-4-yl)piperazine-1-carboxylate (**3A**)

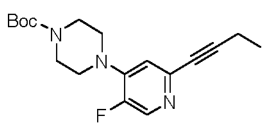

Tert-butyl 4-(2-chloro-5-fluoropyridin-4-yl)piperazine-1-carboxylate (**1C**) (20.1 g, 62 mmo), (but-1-yn-1-yl)trimethylsilane (9.3 g, 74 mmol), bistriphenylphosphine palladium dichloride (4.4 g, 6.3 mmol), 1,3-bis(diphenylphosphino)propane (3.84 g, 9.3 mmol) and caesium fluoride (19.0 g, 125 mmol) were successively added to dimethyl sulfoxide (160 mL). The system was subjected to nitrogen replacement three times and reacted at 95°C for 4 h. After the reaction was completed, the reaction product was cooled to room temperature, and water (160 mL) was added. The aqueous phase was extracted with ethyl acetate (200 mL × 3). The organic phases were combined, washed with a saturated aqueous sodium chloride solution (200 mL), dried over anhydrous sodium sulphate and filtered. The filtrate was concentrated under reduced pressure. The residue was separated by column chromatography on silica gel (PE : EA = 20 : 1 - 5 : 1) to obtain tert-butyl 4-(2-(but-1-yn-1-yl)-5-fluoropyridin-4-yl)piperazine-1-carboxylate (**3A**) as a pale brown viscous liquid (11.0 g, yield: 53%).

LCMS m/z =334.2[M +1]+.

Step 2:

tert-butyl 4-(2-ethyl-6-fluoropyrazolo[1,5-a]pyridin-5-yl)piperazine-1-carboxylate (**3B**)

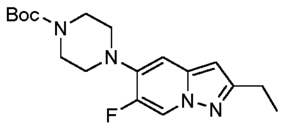

Ethanol (100 mL) and tert-butyl 4-(2-(but-1-yn-1-yl)-5-fluoropyridin-4-yl)piperazine-1-carboxylate (**3A**) (11.0 g, 33 mmol) were added to a 250 mL reaction flask and cooled to 0°C. 2-[(aminooxy)sulfonyl]-1,3,5-trimethylbenzene (**1E**) (10.7 g, 49.5 mmol) was added in portions, and then sodium bicarbonate (5.5 g, 66 mmol) was added to the reaction. Upon completion of the addition, the mixture was reacted at room temperature for 2 h. After that, potassium carbonate (9.12 g, 66 mmol) was added to the reaction and reacted at room temperature overnight. Water (200 mL) was added to the reaction liquid, and the aqueous phase was extracted with ethyl acetate (200 mL × 2). The organic phases were combined, washed with saturated sodium chloride (100 mL), dried over anhydrous sodium sulphate, filtered, concentrated and separated by column chromatography on silica gel to obtain the target compound tert-butyl 4-(2-ethyl-6-fluoropyrazolo[1,5-a]pyridin-5-yl)piperazine-1-carboxylate (**3B**) as a white solid (7.8 g, yield: 68%).

LCMS m/z =349.2[M +1]+.

Step 3:

tert-butyl 4-(2-ethyl-6-fluoro-3-nitrosopyrazolo[1,5-a]pyridin-5-yl)piperazine-1-carboxylate (3C)

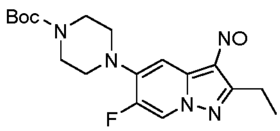

Tert-butyl 4-(2-ethyl-6-fluoropyrazolo[1,5-a]pyridin-5-yl)piperazine-1-carboxylate (**3B**) (7.40 g, 22.4 mmol) was dissolved in methanol (40 mL) and acetic acid (8 mL), and the resulting solution was cooled to 5°C. After that, sodium nitrite (3.09 g, 44.8 mmol) was dissolved in water (10 mL) and slowly added dropwise to the reaction liquid. Upon completion of the addition, the resulting mixture was reacted at room temperature for 16 h. Water (30 mL) was added dropwise to the reaction liquid. Upon completion of the dropwise addition, filtration was performed. The filter cake was washed with water (10 mL × 2) and dried to obtain a crude product of the target compound tert-butyl 4-(2-ethyl-6-fluoro-3-nitrosopyrazolo[1,5-a]pyridin-5-yl)piperazine-1-carboxylate (**3C**) as a dark green solid (10.8 g).

LCMS m/z =378.3 [M +1]+.

Step 4:

tert-butyl 4-(3-amino-2-ethyl-6-fluoropyrazolo[1,5-a]pyridin-5-yl)piperazine-1-carboxylate (**3D**)

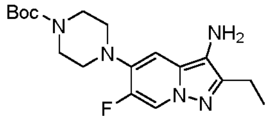

Tert-butyl 4-(2-ethyl-6-fluoro-3-nitrosopyrazolo[1,5-a]pyridin-5-yl)piperazine-1-carboxylate (**3C**) (10.8 g, crude)

was dissolved in ethanol (40 mL) and water (20 mL). After that, ammonium chloride (14.98 g, 0.28 mol) and iron powder (7.84 g, 0.14 mol) were added to the reaction, reacted at 65°C for 20 min and filtered. The filtrate was extracted with dichloromethane (60 mL × 3). The organic phases were combined, washed with saturated brine (100 mL × 1), dried over anhydrous sodium sulphate and filtered. The filtrate was concentrated under reduced pressure to obtain a crude product of the target compound tert-butyl 4-(3-amino-2-ethyl-6-fluoropyrazolo[1,5-a]pyridin-5-yl)piperazine-1-carboxylate (**3D**) as a yellow solid (7.12 g, two-step yield: 87.5%).

LCMS m/z =364.2[M +1]$^+$.

Step 5:

tert-butyl 4-(3-((5-cyano-4-(4-fluorophenyl)thiazol-2-yl)amino)-2-ethyl-6-fluoropyrazolo[1,5-a]pyridin-5-yl)piperazine-1-carboxylate (**3E**)

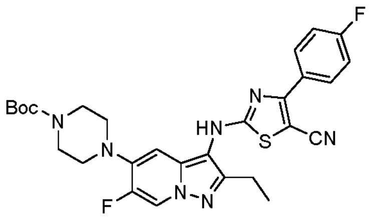

Tert-butyl 4-(3-amino-2-ethyl-6-fluoropyrazolo[1,5-a]pyridin-5-yl)piperazine-1-carboxylate (**3D**) (7.12 g, 19.6 mmol), 2-chloro-4-(4-fluorophenyl)thiazole-5-cyano (**1I**) (5.61 g, 23.5 mmol), 2,6-dimethylpyridine (3.15 g, 29.4 mmol) and N,N-dimethylacetamide (40 mL) were added, and the resulting mixture was warmed to 70°C and reacted for 5 h. The reaction was quenched by adding a 10% sodium chloride solution (100 mL) and extracted with ethyl acetate (150 mL × 3). The organic phases were combined, washed with saturated brine (100 mL × 1), dried over anhydrous sodium sulphate and filtered. The filtrate was concentrated under reduced pressure, and the residue was separated by column chromatography on silica gel to obtain the target compound tert-butyl 4-(3-((5-cyano-4-(4-fluorophenyl)thiazol-2-yl)amino)-2-ethyl-6-fluoropyrazolo[1,5-a]pyridin-5-yl)piperazine-1-carboxylate (**3E**) as a yellowish-brown solid (10.0 g, yield: 90.3%).

LCMS m/z =566.2[M +1]$^+$.

Step 6:

tert-butyl 4-(3-((5-cyano-4-(4-fluorophenyl)thiazol-2-yl)(methyl-d3)amino)-2-ethyl-6-fluoropyrazolo[1,5-a]pyridin-5-yl)piperazine-1-carboxylate (**3F**)

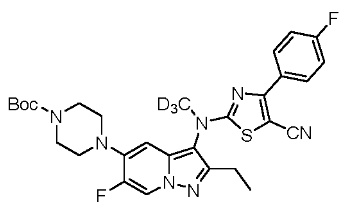

Tert-butyl 4-(3-((5-cyano-4-(4-fluorophenyl)thiazol-2-yl)amino)-2-ethyl-6-fluoropyrazolo[1,5-a]pyridin-5-yl)piperazine-1-carboxylate (**3E**) (10.0 g, 17.7 mmol) was dissolved in tetrahydrofuran (60 mL) and cooled to 0°C under nitrogen protection. Sodium hydride (0.85 g, 21.2 mmol, 60 wt%) was added in portions. Upon completion of the addition, the resulting mixture was reacted under such condition for 10 min, and then methyl-d3 iodide (3.07 g, 21.2 mmol) was added dropwise. Upon completion of the addition, the resulting mixture was reacted at room temperature for 30 min. The reaction was quenched by adding water (100 mL) and extracted with ethyl acetate (100 mL × 2). The organic phases were combined, washed with saturated brine (100 mL × 1), dried over anhydrous sodium sulphate and filtered. The filtrate was concentrated under reduced pressure to obtain a crude product of the target compound tert-butyl 4-(3-((5-cyano-4-(4-fluorophenyl)thiazol-2-yl)(methyl-d3)amino)-2-ethyl-6-fluoropyrazolo[1,5-a]pyridin-5-yl)piperazine-1-carboxylate (**3F**) as a yellow solid (11.7 g).

LCMS m/z =583.2[M +1]$^+$.

Step 7:

2-((2-ethyl-6-fluoro-5-(piperazin-1-yl)pyrazolo[1,5-a]pyridin-3-yl)(methyl-d3)amino)-4-(4-fluorophenyl)thiazole-5-carbonitrile hydrochloride (**3G**)

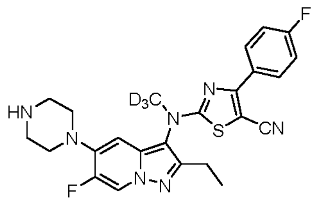

The crude product (11.7 g) of the compound tert-butyl 4-(3-((5-cyano-4-(4-fluorophenyl)thiazol-2-yl)(methyl-d3)amino)-2-ethyl-6-fluoropyrazolo[1,5-a]pyridin-5-yl)piperazine-1-carboxylate (**3F**) was dissolved in dichloromethane (100 mL), and trifluoroacetic acid (33 mL) was added dropwise. The resulting mixture was reacted for about 3 h and then concentrated under reduced pressure. Dichloromethane (200 mL) was added to the residue, and the pH was adjusted to 8 with saturated sodium bicarbonate. Liquid separation was performed. The aqueous phase was extracted with dichloromethane (200 mL × 2). The organic phases were combined, washed with saturated brine (300 mL × 1), dried over anhydrous sodium sulphate and filtered. The filtrate was concentrated under reduced pressure to obtain a crude product of the target compound 2-((2-ethyl-6-fluoro-5-(piperazin-1-yl)pyrazolo[1,5-a]pyridin-3-yl)(methyl-d3)amino)-4-(4-fluorophenyl)thiazole-5-carbonitrile (**3G**) as a yellow solid (10.0 g).

LCMS m/z =483.2[M +1]$^+$.

Step 8:

2-((2-ethyl-6-fluoro-5-(4-(2-(3-hydroxyazetidin-1-yl)-2-oxoethyl)piperazin-1-yl)pyrazolo[1,5-a]pyridin-3-yl)(methyl-d3)amino)-4-(4-fluorophenyl)thiazole-5-carbonitrile (**compound 3**)

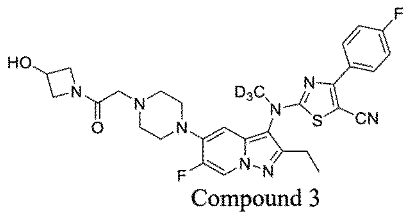

Compound 3

The crude product (10.0 g) of 2-((2-ethyl-6-fluoro-5-(piperazin-1-yl)pyrazolo[1,5-a]pyridin-3-yl)(methyl-d3)amino)-4-(4-fluorophenyl)thiazole-5-carbonitrile (**3G**) was dissolved in acetonitrile (100 mL). 2-chloro-1-(3-hydroxyazetidin-1-yl)ethanone (**1M**) (3.35 g, 22.4 mmol) and potassium carbonate (5.55 g, 40.1 mmol) were successively added, and the resulting mixture was warmed to 80°C and reacted for 6 h. The reaction was quenched by adding water (100 mL) and extracted with ethyl acetate (150 mL × 3). The organic phases were combined, washed with saturated brine (100 mL × 1), dried over anhydrous sodium sulphate and filtered. The filtrate was concentrated under reduced pressure, and the residue was separated by column chromatography on silica gel (dichloromethane/methanol = 20 : 1) to obtain the title compound 2-((2-ethyl-6-fluoro-5-(4-(2-(3-hydroxyazetidin-1-yl)-2-oxoethyl)piperazin-1-yl)pyrazolo[1,5-a]pyridin-3-yl)(methyl-d3)amino)-4-(4-fluorophenyl)thiazole-5-carbonitrile (**compound 3**) (7.6 g, three-step yield: 72.1%).

$^1$H NMR (400 MHz, $CDCl_3$) δ 8.27 (d, 1H), 8.20-8.10 (m, 2H), 7.20-7.11 (m, 2H), 6.45 (d, 1H), 4.67 (s, 1H), 4.50-4.40 (m, 1H), 4.32-4.24 (m, 1H), 4.16-4.08 (m, 1H), 3.94-3.86 (m, 1H), 3.29-3.07 (m, 6H), 2.78( s, 4H), 2.72 (q, 2H), 2.58 (s, 1H), 1.32 (t, 3H).

LCMS m/z =596.3[M +1]$^+$.

**Example 4:**

2-((2-ethyl-6-fluoro-5-(4-(2-(3-hydroxyazetidin-1-yl)-2-oxoethyl)piperazin-1-yl-2,2,3,3,5,5,6,6-d8)pyrazolo[1,5-a]pyridin-3-yl)(methyl)amino)-4-(4-fluorophenyl)thiazole-5-carbonitrile (**compound 4**)

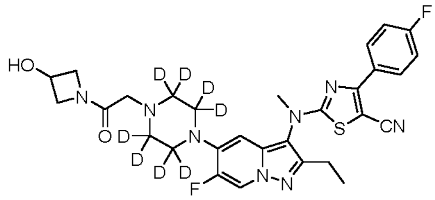

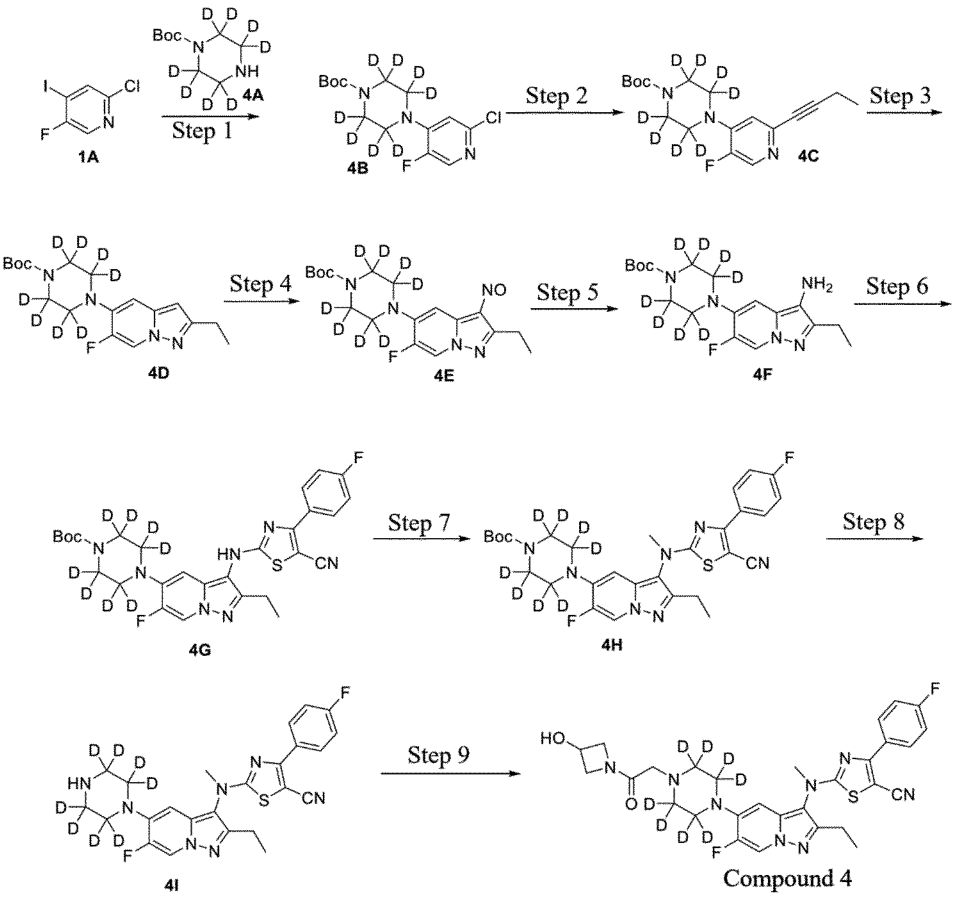


Step 1:

tert-butyl 4-(2-chloro-5-fluoropyridin-4-yl)piperazine-1-carboxylate-2,2,3,3,5,5,6,6-d8 (**4B**)

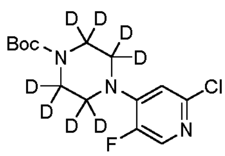

To a 250 mL reaction flask, NMP (93 mL) was added, and then 2-chloro-5-fluoro-4-iodopyridine (**1A**) (9.3 g,

36.2 mmol), piperazin-1-tert-butyl carbonate-2,2,3,3,5,5,6,6-d8 (**4A**) (4.5 g, 24.2 mmol) and potassium carbonate (6.7 g, 48.3 mmol) were added successively. The resulting mixture was warmed to 150°C and reacted for 3 h, and then the reaction stopped. The reaction liquid was cooled to room temperature, the reactant was slowly added to ice water (200 mL), and a white solid was precipitated out. Stirring was performed for 0.5 h, and then filtration was performed. The filter cake was washed with water, slurried with n-hexane (100 mL), filtered and dried to obtain the target compound tert-butyl 4-(2-chloro-5-fluoropyridin-4-yl)piperazine-1-carboxylate-2,2,3,3,5,5,6,6-d8 (**4B**) as a white solid (10.01 g, yield: 85%).
LCMS m/z =324.3[M +1]$^+$.

Step 2:

tert-butyl 4-(2-(but-1-yn-1-yl)-5-fluoropyridin-4-yl)piperazine-1-carboxylate-2,2,3,3,5,5,6,6-d8 (**4C**)

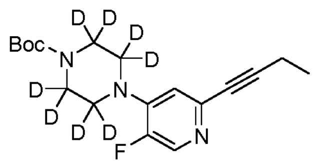

Tert-butyl 4-(2-chloro-5-fluoropyridin-4-yl)piperazine-1-carboxylate (**4B**) (2.00 g, 6.2 mmol), (but-1-yn-1-yl)trimethylsilane (0.94 g, 7.4 mmol), bistriphenylphosphine palladium dichloride (0.44 g, 0.6 mmol), 1,3-bis(diphenylphosphino)propane (0.38 g, 0.9 mmol) and caesium fluoride (1.89 g, 12.4 mmol) were successively added to dimethyl sulfoxide (16 mL). The system was subjected to nitrogen replacement three times and reacted at 95°C for 5 h. After the reaction was completed, the reaction product was cooled to room temperature, and water (20 mL) was added. The aqueous phase was extracted with ethyl acetate (20 mL × 3). The organic phases were combined, washed with a saturated aqueous sodium chloride solution (20 mL), dried over anhydrous sodium sulphate and filtered. The filtrate was concentrated under reduced pressure. The residue was separated by column chromatography on silica gel (PE : EA = 15 : 1 - 5 : 1) to obtain **4C** (1.20 g, yield: 57%).
LCMS m/z =342.3[M +1]$^+$.

Step 3:

tert-butyl 4-(2-ethyl-6-fluoropyrazolo[1,5-a]pyridin-5-yl)piperazine-1-carboxylate-2,2,3,3,5,5,6,6-d8 (**4D**)

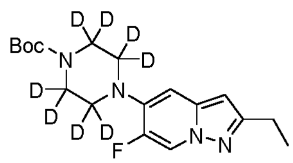

Ethanol (12 mL) and **4C** (1.20 g, 3.5 mmol) were added to a 100 mL reaction flask and cooled to 0°C. 2-[(aminooxy)sulfonyl]-1,3,5-trimethylbenzene (**1E**) (1.14 g, 5.3 mmol) was added in portions, and then sodium bicarbonate (0.59 g, 7.0 mmol) was added to the reaction. Upon completion of the addition, the mixture was reacted at room temperature for 2 h. After that, potassium carbonate (0.97 g, 7.0 mmol) was added to the reaction and reacted at room temperature overnight. Water (150 mL) was added to the reaction liquid. The aqueous phase was extracted with ethyl acetate (200 mL × 2). The organic phases were combined, washed with saturated sodium chloride (100 mL), dried over anhydrous sodium sulphate, filtered and concentrated to obtain a crude product (2.0 g) of **4D.**
LCMS m/z =357.2[M +1]$^+$.

Step 4:

tert-butyl 4-(2-ethyl-6-fluoro-3-nitrosopyrazolo[1,5-a]pyridin-5-yl)piperazine-1-carboxylate-2,2,3,3,5,5,6,6-d8 (**4E**)

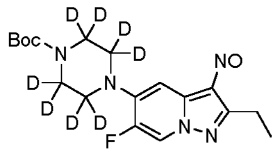

The crude product (2.0 g, about 5.6 mmol) of **4D** obtained in the previous step was dissolved in methanol (10.0 mL) and acetic acid (2.0 mL), and the resulting solution was cooled to 5°C-10°C. After that, an aqueous solution (2.0 mL) of sodium nitrite (0.77 g, 11.2 mmol) was added dropwise to the reaction liquid. Upon completion of the addition, the resulting mixture was reacted at room temperature for 16 h. Water (20 mL) was added dropwise to the reaction liquid. Upon completion of the addition, filtration was performed. The filter cake was washed with water (5 mL × 2) and dried with suction to obtain a crude product (2.00 g) of **4E.**

Step 5:

tert-butyl 4-(3-amino-2-ethyl-6-fluoropyrazolo[1,5-a]pyridin-5-yl)piperazine-1-carboxylate-2,2,3,3,5,5,6,6-d8 (**4F**)

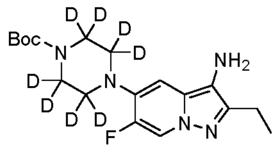

The crude product (2.00 g, about 5.2 mmol) of **4E** obtained in the previous step was dissolved in ethanol (10 mL) and water (10 mL). After that, ammonium chloride (2.78 g, 52.0 mmol) and iron powder (1.46 g, 26.0 mmol) were added to the reaction, reacted at 65°C for 2 h and filtered. The filtrate was extracted with EA (10 mL × 3). The organic phases were combined, washed with saturated brine (10 mL × 1), dried over anhydrous sodium sulphate and filtered. The filtrate was concentrated under reduced pressure, and then the residue was purified by column chromatography (DCM : MeOH = 30 : 1) to obtain **4F** (0.92 g, yield: 48%).

LCMS m/z =372.3$[M+1]^+$.

Step 6:

tert-butyl 4-(3-((5-cyano-4-(4-fluorophenyl)thiazol-2-yl)amino)-2-ethyl-6-fluoropyrazolo[1,5-a]pyridin-5-yl)piperazine-1-carboxylate-2,2,3,3,5,5,6,6-d8 (**4G**)

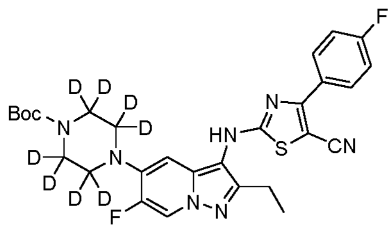

**4F** (0.92 g, 2.5 mmol), 2-chloro-4-(4-fluorophenyl)thiazole-5-carbonitrile (**1I**) (0.72 g, 3.0 mmol), 2,6-dimethylpyridine (0.40 g, 3.8 mmol) and N,N-dimethylacetamide (5 mL) were added, and the resulting mixture was warmed to 70°C and reacted overnight. The reaction was quenched by adding a 10% sodium chloride solution (20 mL) and extracted

with ethyl acetate (20 mL × 3). The organic phases were combined, washed with saturated brine (20 mL × 1),dried over anhydrous sodium sulphate and filtered. The filtrate was concentrated under reduced pressure, and then the residue was subjected to column chromatography (DCM : MeOH(v/v) = 30 : 1) to obtain **4G** (1.00 g, yield: 70%).

LCMS m/z =574.3[M +1]$^+$.

Step 7:

tert-butyl 4-(3-((5-cyano-4-(4-fluorophenyl)thiazol-2-yl)(methyl)amino)-2-ethyl-6-fluoropyrazolo[1,5-a]pyridin-5-yl)piperazine-1-carboxylate-2,2,3,3,5,5,6,6-d8 (**4H**)

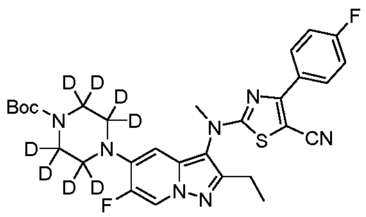

**4G** (15.50 g) was dissolved in tetrahydrofuran (10 mL) and cooled to 0°C under nitrogen protection. Sodium hydride (0.10 g, 2.1 mmol, 60 wt%) was added in portions. Upon completion of the addition, the mixture was reacted under such condition for 10 min, and then iodomethane (0.16 mL, 2.1 mmol) was added dropwise. Upon completion of the addition, the resulting mixture was naturally warmed to room temperature and reacted for 1 h. The reaction was quenched by adding 10 mL of methanol, and the reaction liquid was subjected to rotary evaporation to obtain a crude product (1.20 g) of **4H,** which was directly used in the next reaction without purification.

LCMS m/z = 588.3[M +1]$^+$.

**Compound 4H may also be synthesized via the following method.**

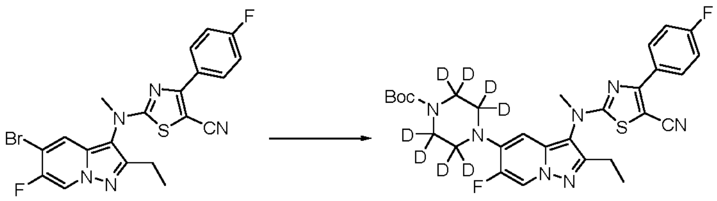

4J 4H

Raw materials **4J** (see WO 2019228403 A1 for synthesis of intermediate 3) (948 mg, 2.0 mmol), N-Boc-piperazine-d8 (580 mg, 3.0 mmol), tris(dibenzylideneacetone)dipalladium (183 mg, 0.2 mmol), 4,5-bis(diphenylphosphino)-9,9-dimethylxanthene (230 mg, 0.4 mmol) and sodium tert-butoxide (576 mg, 6.0 mmol) were added to dry 1,4-dioxane (20 mL) at room temperature. The system was subjected to nitrogen replacement three times, warmed to 105°C and reacted for 2 h. The resultant was cooled to room temperature, subjected to rotary evaporation and directly purified by column chromatography (PE : EA = 5 : 1 - 2 : 1) to obtain **4H** as a brown foamy solid (530 mg, yield: 45%).

LCMS m/z = 588.3[M +1]$^+$.

Step 8: 2-((2-ethyl-6-fluoro-5-(piperazin-1-yl-2,2,3,3,5,5,6,6-d8)pyrazolo[1,5-a]pyridin-3-yl)(methyl)amino)-4-(4-fluorophenyl)thiazole-5-carbonitrile (**4I**)

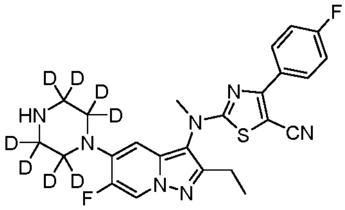

Trifluoroacetic acid (5.0 mL) was dropwise added to a solution of compound **4H** (1.20 g, crude, obtained in the previous step) in dichloromethane (15.0 mL) at 0°C under nitrogen protection, and then the resulting mixture was naturally warmed to room temperature and stirred for another 2 h. After the reaction liquid was subjected to rotary evaporation, 10 mL of dichloromethane was added, and then the resulting mixture was neutralized to pH = 8 with saturated sodium carbonate, extracted with dichloromethane (10 mL × 2), washed with water (10 mL × 1), washed with saturated sodium chloride (10 mL × 1), dried over anhydrous sodium sulphate, filtered and concentrated under reduced pressure to obtain a crude product (0.90 g) of **4I,** which was directly used in the next reaction without purification.

LCMS m/z = 488.3[M +1]$^+$.

Step 9:

2-((2-ethyl-6-fluoro-5-(4-(2-(3-hydroxyazetidin-1-yl)-2-oxoethyl)piperazin-1-yl-2,2,3,3,5,5,6,6-d8)pyrazolo[1,5-a]pyridin-3-yl)(methyl)amino)-4-(4-fluorophenyl)thiazole-5-carbonitrile (**compound 4**)

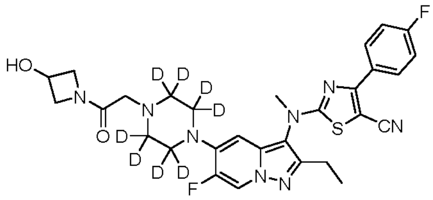

The crude product (860 mg, about 1.8 mmol) of compound **4I,** 2-chloro-1-(3-hydroxyacridin-1-yl)ethan-1-one (296 mg, 2.0 mmol) and potassium carbonate (500 mg, 3.6 mmol) were added to acetonitrile (50 mL) at room temperature, and then the resulting mixture was warmed to 80°C and reacted for 5 h. The reaction product was cooled to room temperature and then filtered by suction. The filter cake was washed with dichloromethane (30 mL × 2). The filtrate was concentrated, and then the residue was subjected to column chromatography (DCM : MeOH = 30 : 1) to obtain compound **4** (880 mg, yield: 81%).

$^1$H NMR (400 MHz, $CDCl_3$) δ 8.27 (d, 1H), 8.19-8.11 (m, 2H), 7.20-7.12 (m, 2H), 6.44 (d,1H), 4.67 (s, 1H), 4.50-4.40 (m, 1H), 4. 32-4.24 (m, 1H), 4.16- 4.09 (m, 1H), 3.94-3.86 (m, 1H), 3.58 (s, 3H), 3.24-3.06 (m, 2H), 2.72 (q, 2H), 2.59 (s, 1H), 1.32 (t, 3H).

LCMS m/z = 601.2[M +1]$^+$.

**Example 5:**

2-((2-ethyl-6-fluoro-5-(4-(2-(3-hydroxyazetidin-1-yl-3-d)-2-oxoethyl)piperazin-1-yl)pyrazolo[1,5-a]pyridin-3-yl)(methyl)amino)-4-(4-fluorophenyl)thiazole-5-carbonitrile (**compound 5**)

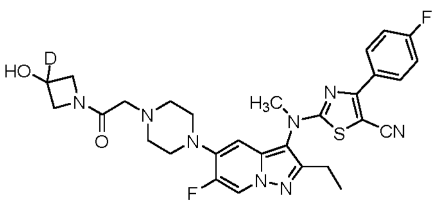

**Compound 5**

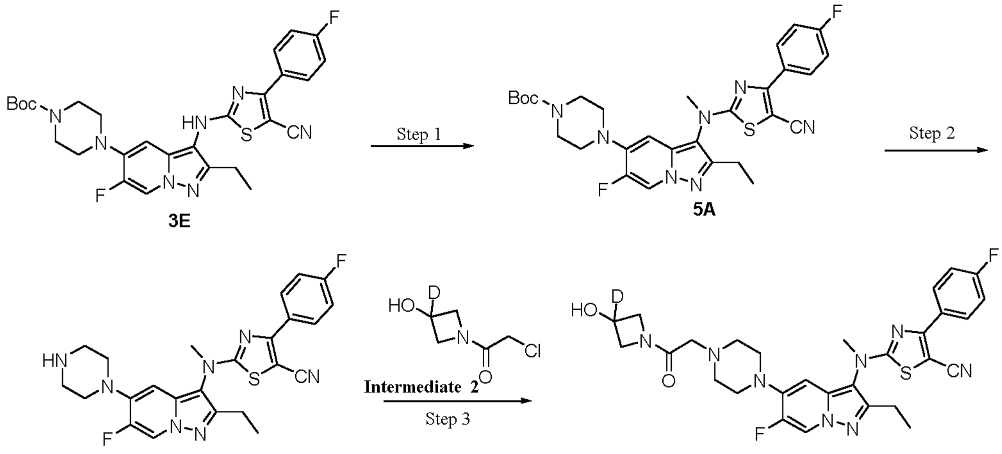


3E 5A 5B Compound 5

Step 1:

tert-butyl 4-(3-((5-cyano-4-(4-fluorophenyl)thiazol-2-yl)(methyl)amino)-2-ethyl-6-fluoropyrazolo[1,5-a]pyridin-5-yl)piperazine-1-carboxylate (**5A**)

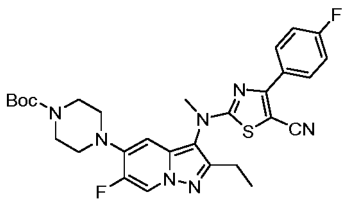

Tert-butyl 4-(3-((5-cyano-4-(4-fluorophenyl)thiazol-2-yl)amino)-2-ethyl-6-fluoropyrazolo[1,5-a]pyridin-5-yl)piperazine-1-carboxylate (**3E**) (2.8 g, 5.0 mmol) was dissolved in tetrahydrofuran (30 mL) and cooled to 0°C under nitrogen protection. Sodium hydride (0.24 g, 6.0 mmol, 60 wt%) was added in portions. Upon completion of the addition, the resulting mixture was reacted under such condition for 10 min, and then methyl iodide (1.42 g, 10 mmol) was added dropwise. Upon completion of the addition, the resulting mixture was reacted at room temperature for 30 min. The reaction was quenched by adding water (50 mL) and extracted with ethyl acetate (50 mL × 2). The organic phases were combined, washed with saturated brine (100 mL × 1), dried over anhydrous sodium sulphate and filtered. The filtrate was concentrated under reduced pressure to obtain a crude product of the target compound tert-butyl 4-(3-((5-cyano-4-(4-fluorophenyl)thiazol-2-yl)(methyl)amino)-2-ethyl-6-fluoropyrazolo[1,5-a]pyridin-5-yl)piperazine-1-carboxylate (**5A**) as a yellow solid (2.8 g).

LCMS m/z = 580.3[M +1]$^+$.

Step 2:

2-((2-ethyl-6-fluoro-5-(piperazin-1-yl)pyrazolo[1,5-a]pyridin-3-yl)(methyl)amino)-4-(4-fluorophenyl)thiazole-5-carbonitrile (**5B**)

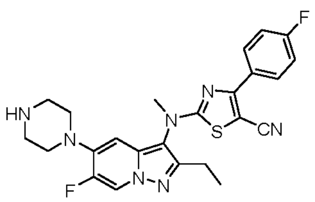

The crude product (2.8 g) of tert-butyl 4-(3-((5-cyano-4-(4-fluorophenyl)thiazol-2-yl)(methyl)amino)-2-ethyl-6-fluoropyrazolo[1,5-a]pyridin-5-yl)piperazine-1-carboxylate (**5A**) was dissolved in dichloromethane (30 mL), and trifluoroacetic acid (15 mL) was added dropwise. The resulting mixture was reacted for about 3 h and then concentrated under reduced pressure. Dichloromethane (100 mL) was added to the residue, and the pH was adjusted to 8 with saturated sodium bicarbonate. Liquid separation was performed. The aqueous phase was extracted with dichloromethane (100 mL × 2). The organic phases were combined, washed with saturated brine (100 mL × 1), dried over anhydrous sodium sulphate and filtered. The filtrate was concentrated under reduced pressure to obtain the target compound 2-((2-ethyl-6-fluoro-5-(piperazin-1-yl)pyrazolo[1,5-a]pyridin-3-yl)(methyl)amino)-4-(4-fluorophenyl)thiazole-5-carbonitrile (**5B**) as a yellow solid (2.0 g, two-step yield: 83.3%).

LCMS m/z = 480.1[M +1]$^+$.

Step 3:

2-((2-ethyl-6-fluoro-5-(4-(2-(3-hydroxyazetidin-1-yl-3-d)-2-oxoethyl)piperazin-1-yl)pyrazolo[1,5-a]pyridin-3-yl)(methyl)amino)-4-(4-fluorophenyl)thiazole-5-carbonitrile (**compound 5**)

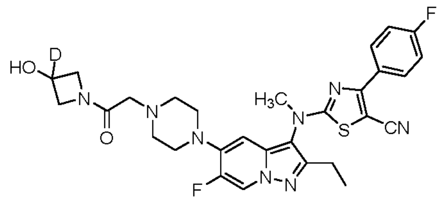

Compound 5

2-((2-ethyl-6-fluoro-5-(piperazin-1-yl)pyrazolo[1,5-a]pyridin-3-yl)(methyl)amino)-4-(4-fluorophenyl)thiazole-5-carbonitrile (0.31 g, 0.65 mmol) was dissolved in acetonitrile (10 mL). 2-chloro-1-(3-hydroxyazetidin-1-yl-3-d)ethan-1-one (**intermediate 2**) (0.15 g, 1.0 mmol) and potassium carbonate (0.27 g, 2.0 mmol) were successively added, and the resulting mixture was warmed to 80°C and reacted for 6 h. The reaction was quenched by adding water (50 mL) and extracted with dichloromethane (50 mL × 3). The organic phases were combined, washed with saturated brine (50 mL × 1), dried over anhydrous sodium sulphate and filtered. The filtrate was concentrated under reduced pressure, and the residue was separated by column chromatography on silica gel (dichloromethane/methanol = 20 : 1) to obtain the title compound 2-((2-ethyl-6-fluoro-5-(4-(2-(3-hydroxyazetidin-1-yl-3-d)-2-oxoethyl)piperazin-1-yl)pyrazolo[1,5-a]pyridin-3-yl)(methyl)amino)-4-(4-fluorophenyl)thiazole-5-carbonitrile (**compound 5**) (90 mg, yield: 23%).

$^1$H NMR (400 MHz, $CDCl_3$) δ 8.27 (d, 1H), 8.18-8.10 (m, 2H), 7.20-7.10 (m, 2H), 6.45 (d, 1H), 4.43 (d, 1H), 4.25 (d, 1H), 4.11 (dd, 1H), 3.89 (d, 1H), 3.58 (s, 3H), 3.28-3.06 (m, 7H), 2.81-2.66 (m, 6H), 1.31 (t, 3H).

LCMS m/z = 594.2[M +1]$^+$.

**Example 6:**

2-((2-ethyl-6-fluoro-5-(4-(2-(3 -hydroxyazetidin-1 -yl)-2-oxoethyl-1,1-d2)piperazin-1-yl)pyrazolo[1,5-a]pyridin-3-yl)(methyl)amino)-4-(4-fluorophenyl)thiazole-5-carbonitrile (**compound 6**)

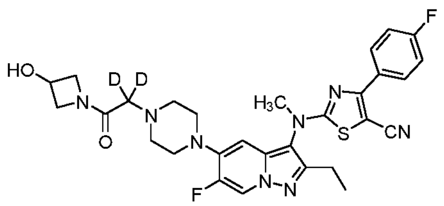

Compound 6

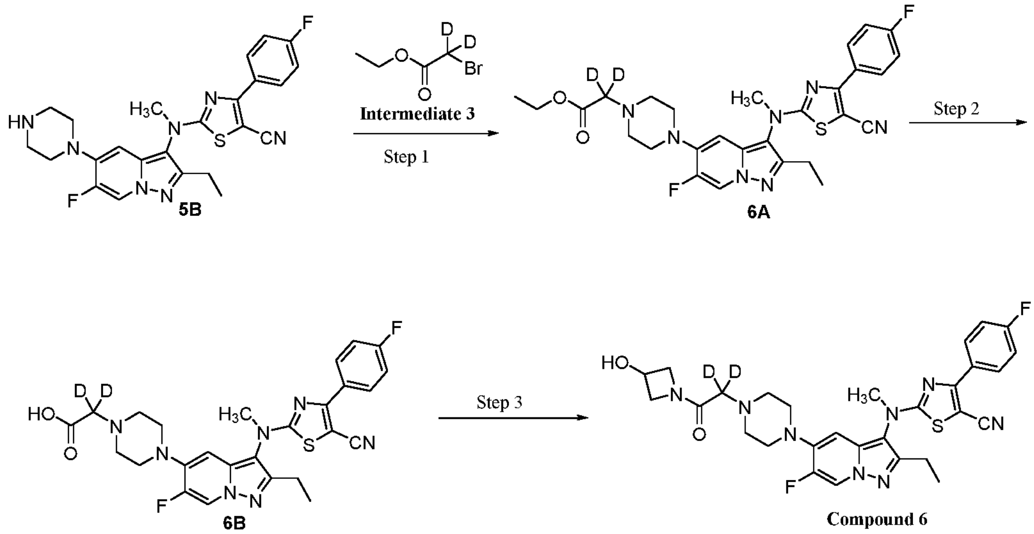


5B

6A

6B

Compound 6

Step 1:

ethyl 2-(4-(3-((5-cyano-4-(4-fluorophenyl)thiazol-2-yl)(methyl)amino)-2-ethyl-6-fluoropyrazolo[1,5-a]pyridin-5-yl)piperazin-1-yl)acetate-d2 (**6A**)

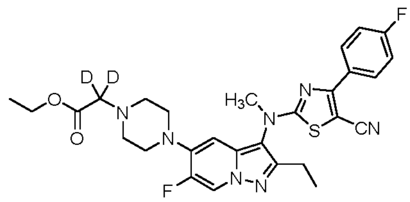

Potassium carbonate (0.14 g, 1 mmol) and ethyl 2-bromoacetate-d2 (**intermediate 3**) (0.17 g, 1 mmol) were successively added to a solution of 2-((2-ethyl-6-fluoro-5-(piperazin-1-yl)pyrazolo[1,5-a]pyridin-3-yl)(methyl)amino)-4-(4-fluorophenyl)thiazole-5-carbonitrile (**5B**) (0.31 g, 0.65 mmol) in acetonitrile (10 mL), and the mixture was stirred at room temperature for 1 h. After the reaction was completed, the mixture was diluted with saturated brine (30 mL) and extracted with ethyl acetate (30 mL × 2). The organic phases were dried over anhydrous sodium sulphate, filtered and concentrated. The residue was purified by column chromatography on silica gel (ethyl acetate : petroleum ether = 1 : 1) to obtain ethyl 2-(4-(3-((5-cyano-4-(4-fluorophenyl)thiazol-2-yl)(methyl)amino)-2-ethyl-6-fluoropyrazolo[1,5-a]pyridin-5-yl)piperazin-1-yl)acetate-d2 (**6A**) as a pale yellow solid (0.32 g, 86.7%).

LC-MS(ESI) : m/z =568.2[M+H]$^+$.

Step 2:

2-(4-(3-((5-cyano-4-(4-fluorophenyl)thiazol-2-yl)(methyl)amino)-2-ethyl-6-fluoropyrazolo[1,5-a]pyridin-5-yl)piperazin-1-yl)acetic-2,2-d2 acid (**6B**)

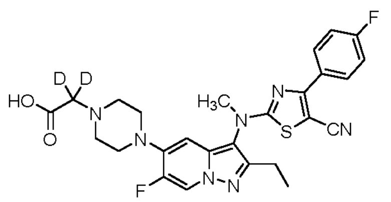

Ethyl 2-(4-(3-((5-cyano-4-(4-fluorophenyl)thiazol-2-yl)(methyl)amino)-2-ethyl-6-fluoropyrazolo[1,5-a]pyridin-5-yl)piperazin-1-yl)acetate-d2 (**6A**) (0.32 g, 0.56 mmol) was dissolved in tetrahydrofuran (6 mL), and methanol (2 mL) and water (2 mL) were added. The mixture was stirred uniformly, and then lithium hydroxide monohydrate (84 mg, 2 mmol) was added. The resulting mixture was reacted at room temperature for 1 h, adjusted to pH 5 with hydrochloric acid (1N aqueous solution) and extracted with ethyl acetate (50 mL × 2). The organic phases were combined, dried over anhydrous sodium sulphate and filtered. The filtrate was concentrated under reduced pressure to obtain 2-(4-(3-((5-cyano-4-(4-fluorophenyl)thiazol-2-yl)(methyl)amino)-2-ethyl-6-fluoropyrazolo[1,5-a]pyridin-5-yl)piperazin-1-yl)acetic-2,2-d2 acid (**6B**) as a pale yellow solid (0.24 g, yield: 79%).

LCMS m/z =540.2 $[M+1]^+$.

Step 3:

2-((2-ethyl-6-fluoro-5-(4-(2-(3 -hydroxyazetidin-1 -yl)-2-oxoethyl-1,1-d2)piperazin-1-yl)pyrazolo[1,5-a]pyridin-3-yl)(methyl)amino)-4-(4-fluorophenyl)thiazole-5-carbonitrile (**compound 6**)

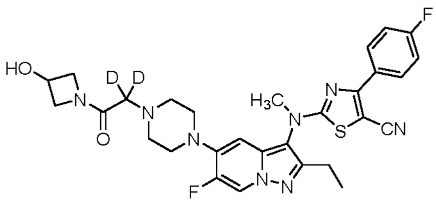

2-(4-(3-((5-cyano-4-(4-fluorophenyl)thiazol-2-yl)(methyl)amino)-2-ethyl-6-fluoropyrazolo[1,5-a]pyridin-5-yl)piperazin-1-yl)acetic-2,2-d2 acid (**6B**) (0.24 g, 0.45 mmol) was dissolved in N,N-dimethylformamide (5 mL). Triethylamine (0.15 g, 1.5 mmol) and HATU (0.19 g, 0.5mmol) were successively added, and the resulting mixture was stirred for 10 min. After that, a solution of 4-hydroxyazetidine hydrochloride (0.7 mmol, 76 mg) in N,N-dimethylformamide (5 mL) was added, and the resulting mixture was reacted at room temperature for 1 h. The reaction was quenched by adding water (30 mL) and extracted with dichloromethane (50 mL × 3). The organic phases were combined, washed with saturated brine (50 mL × 1), dried over anhydrous sodium sulphate and filtered. The filtrate was concentrated under reduced pressure, and the residue was separated by column chromatography on silica gel (dichloromethane/methanol = 20 : 1) to obtain the title compound 2-((2-ethyl-6-fluoro-5-(4-(2-(3-hydroxyazetidin-1-yl)-2-oxoethyl-1,1-d2)piperazin-1-yl)pyrazolo[1,5-a]pyridin-3 - yl)(methyl)amino)-4-(4-fluorophenyl)thiazole-5-carbonitrile (**compound 6**) (0.12 g, yield: 45%).

$^1$H NMR (400 MHz, $CDCl_3$) δ 8.27 (d, 1H), 8.19-8.10 (m, 2H), 7.20-7.12 (m, 2H), 6.45 (d, 1H), 4.67 (s, 1H), 4.49-4.40 (m, 1H), 4.30-4.24 (m, 1H), 4.16-4.08 (m, 1H), 3.96-3.87 (m, 1H), 3.58 (s, 3H), 3.26 (s, 4H), 2.84 (s, 4H), 2.72 (q, 2H), 2.59 (s, 1H), 1.32 (t, 3H).

LCMS m/z = 595.3$[M +1]^+$.

**Example 7:**

2-((2-ethyl-6-fluoro-5-(4-(2-(3-hydroxyazetidin-1-yl-3-d)-2-oxoethyl)piperazin-1-yl)pyrazolo[1,5-a]pyridin-3-yl)(methyl-d3)amino)-4-(4-fluorophenyl)thiazole-5-carbonitrile (**compound 7**)

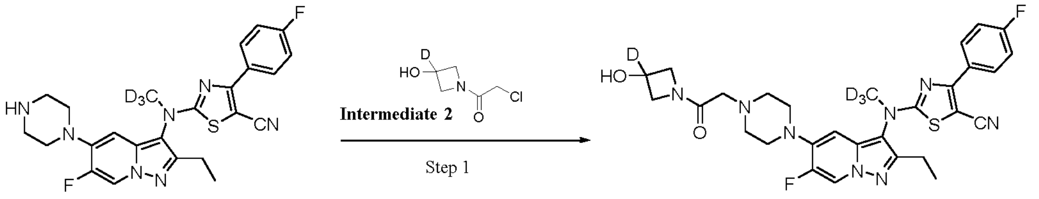


3G

Compound 7

The crude product (300 mg) of 2-((2-ethyl-6-fluoro-5-(piperazin-1-yl)pyrazolo[1,5-a]pyridin-3-yl)(methyl-d3)amino)-4-(4-fluorophenyl)thiazole-5-carbonitrile (**3G**) was dissolved in acetonitrile (5 mL). **Intermediate 2** (103 mg, 0.7 mmol) and potassium carbonate (172 mg, 1.3 mmol) were successively added, and the resulting mixture was warmed to 80°C and reacted for 6 h. The reaction was quenched by adding water (10 mL) and extracted with ethyl acetate (10 mL × 3). The organic phases were combined, washed with saturated brine (10 mL × 1), dried over anhydrous sodium sulphate and filtered. The filtrate was concentrated under reduced pressure, and the residue was separated by column chromatography on silica gel (dichloromethane/methanol (v/v) = 20 : 1) to obtain **compound 7** as a white solid (200 mg, 54%).

$^1$H NMR (400 MHz, $CDCl_3$) δ 8.27 (d, 1H), 8.17-8.13 (m, 2H), 7.18-7.14 (m, 2H), 6.45 (d, 1H), 4.46-4.44 (m, 1H), 4.29-4.26 (m, 1H), 4.14-4.10 (m, 1H), 3.92-3.89 (m, 1H), 3.26-3.15 (m, 6H), 2.83( s, 4H), 2.75-2.69 (m, 2H), 2.54( s, 1H), 1.32 (t, 3H).

LCMS m/z = 597.3[M +1]$^+$.

**Example 8:**

2-((2-ethyl-6-fluoro-5-(4-(2-(3 -hydroxyazetidin-1 -yl)-2-oxoethyl-1,1-d2)piperazin-1-yl)pyrazolo[1,5-a]pyridin-3-yl)(methyl-d3)amino)-4-(4-fluorophenyl)thiazole-5-carbonitrile (**compound 8**)

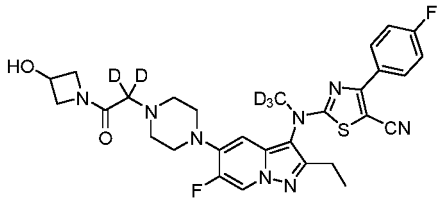

Compound 8

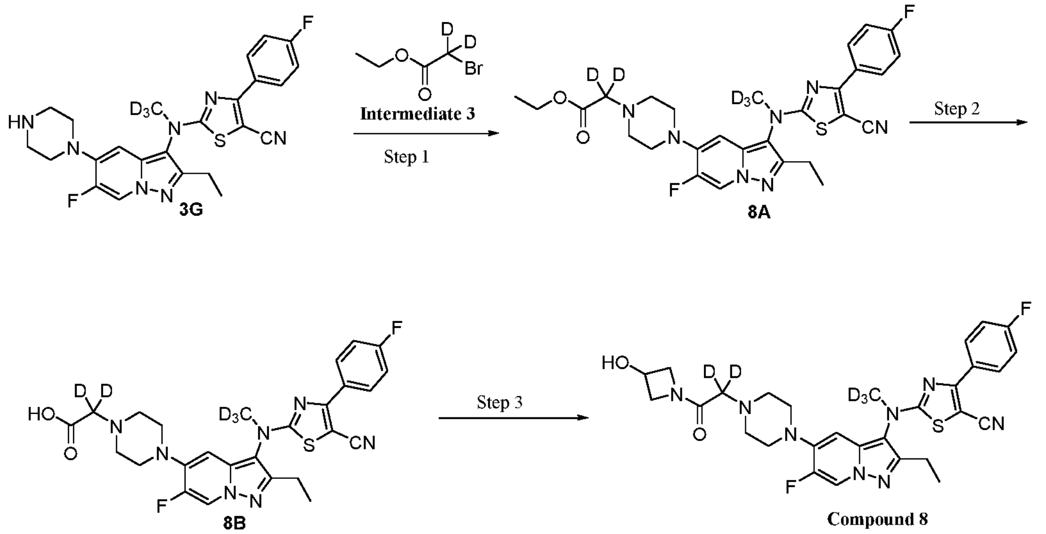


3G

8A

8B

Compound 8

Step 1:

ethyl 2-(4-(3-((5-cyano-4-(4-fluorophenyl)thiazol-2-yl)(methyl-d3)amino)-2-ethyl-6-fluoropyrazolo[1,5-a]pyridin-5-yl)piperazin-1-yl)acetate-d2 (**8A**)

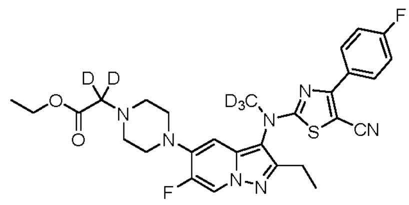

Potassium carbonate (0.21 g, 1.5 mmol) and ethyl 2-bromoacetate-d2 (**intermediate 3**) (0.17 g, 1 mmol) were added successively to a solution of compound **3G** (0.35 g, 0.73 mmol) in acetonitrile (10 mL), and the mixture was stirred at room temperature for 1 h. After the reaction was completed, the mixture was diluted with saturated brine (30 mL) and extracted with ethyl acetate (30 mL × 2). The organic phases were dried over anhydrous sodium sulphate, filtered and concentrated. The residue was purified by column chromatography on silica gel (ethyl acetate : petroleum ether = 1 : 1) to obtain a pale yellow solid (0.38 g, yield: 92%).

Step 2:

2-(4-(3-((5-cyano-4-(4-fluorophenyl)thiazol-2-yl)(methyl-d3)amino)-2-ethyl-6-fluoropyrazolo[1,5-a]pyridin-5-yl)piperazin-1-yl)acetic-2,2-d2 acid (**8B**)

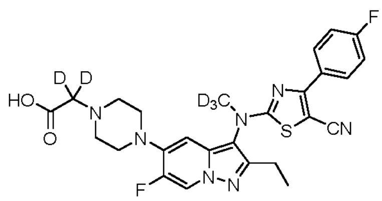

Ethyl 2-(4-(3-((5-cyano-4-(4-fluorophenyl)thiazol-2-yl)(methyl-d3)amino)-2-ethyl-6-fluoropyrazolo[1,5-a]pyridin-5-yl)piperazin-1-yl)acetate-d2 (**8A**) (0.38 g, 0.67 mmol) was dissolved in tetrahydrofuran (9 mL), and methanol (3 mL) and water (3 mL) were added. The mixture was stirred uniformly, and then lithium hydroxide monohydrate (126 mg, 3 mmol) was added. The resulting mixture was reacted at room temperature for 1 h, adjusted to pH 5 with hydrochloric acid (1N aqueous solution) and extracted with ethyl acetate (50 mL × 2). The organic phases were combined, dried over anhydrous sodium sulphate and concentrated to obtain 2-(4-(3-((5-cyano-4-(4-fluorophenyl)thiazol-2-yl)(methyl-d3)amino)-2-ethyl-6-fluoropyrazolo[1,5-a]pyridin-5-yl)piperazin-1-yl)acetic-2,2-d2 acid (**8B**) as a pale yellow solid (0.32 g, yield: 88%).

Step 3:

2-((2-ethyl-6-fluoro-5-(4-(2-(3 -hydroxyazetidin-1 -yl)-2-oxoethyl-1,1-d2)piperazin-1-yl)pyrazolo[1,5-a]pyridin-3-yl)(methyl-d3)amino)-4-(4-fluorophenyl)thiazole-5-carbonitrile (**compound 8**)

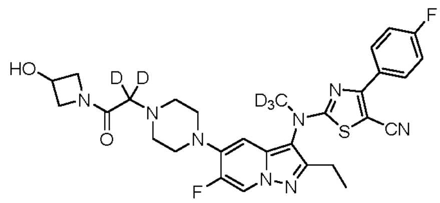

2-(4-(3-((5-cyano-4-(4-fluorophenyl)thiazol-2-yl)(methyl-d3)amino)-2-ethyl-6-fluoropyrazolo[1,5-a]pyridin-5-yl)piperazin-1-yl)acetic-2,2-d2 acid (**8B**) (0.32 g, 0.59 mmol) was dissolved in N,N-dimethylformamide (10 mL). Triethylamine (0.20 g, 2.0 mmol) and HATU (0.27 g, 0.7 mmol) were successively added, and the mixture was stirred for 10 min. After that, a solution of 4-hydroxyazetidine hydrochloride (1 mmol, 0.11 g) in N,N-dimethylformamide (5 mL) was

added, and the resulting mixture was reacted at room temperature for 1 h. The reaction was quenched by adding water (30 mL) and extracted with dichloromethane (50 mL × 3). The organic phases were combined, washed with saturated brine (50 mL × 1), dried over anhydrous sodium sulphate and filtered. The filtrate was concentrated under reduced pressure, and the residue was separated by column chromatography on silica gel (dichloromethane/methanol = 20 : 1) to obtain 2-((2-ethyl-6-fluoro-5-(4-(2-(3-hydroxyazetidin-1-yl)-2-oxoethyl-1,1-d2)piperazin-1-yl)pyrazolo[1,5-a]pyridin-3-yl)(methyl-d3)amino)-4-(4-fluorophenyl)thiazole-5-carbonitrile (**compound 8**) (0.28 g, 79%).

$^1H$ NMR (400 MHz, $CDCl_3$) δ 8.27 (d, 1H), 8.19-8.11 (m, 2H), 7.20-7.11 (m, 2H), 6.44 (d, 1H), 4.67 (s, 1H), 4.49-4.41 (m, 1H), 4.32- 4.24 (m, 1H), 4.15-4.08 (m, 1H), 3.94-3.84 (m, 1H), 3.23 (s, 4H), 2.79- 2.66 (m, 6H), 2.62 (s, 1H), 1.32 (t, 3H).

LCMS m/z = 598.2[M +1]$^+$.

**Example 9:**

2-((2-ethyl-6-fluoro-5-(4-(2-(3-hydroxyazetidin-1-yl)-2-oxoethyl)piperazin-1-yl)pyrazolo[1,5-a]pyridin-3-yl)(methyl)amino)-4-(4-fluorophenyl-2,3,5,6-d4)thiazole-5-carbonitrile (**compound 9**)

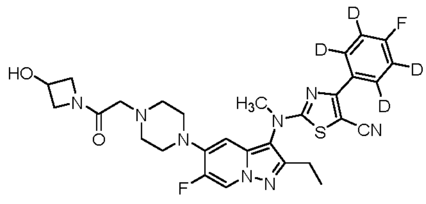

Compound 9

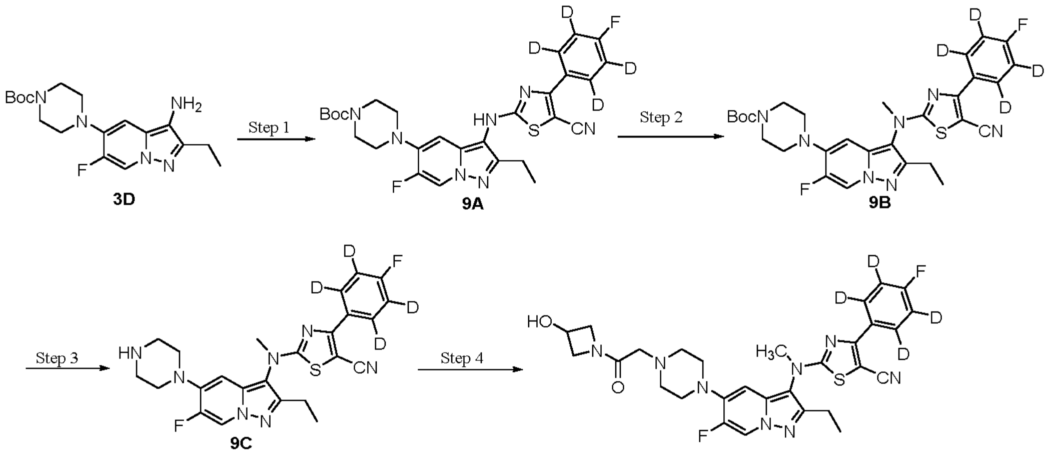


3D 9A 9B

9C

Compound 9

Step 1:

tert-butyl 4-(3-((5-cyano-4-(4-fluorophenyl-2,3,5,6-d4)thiazol-2-yl)amino)-2-ethyl-6-fluoropyrazolo[1,5-a]pyridin-5-yl)piperazine-1-carboxylate (9A)

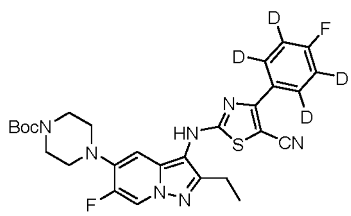

Tert-butyl 4-(3-amino-2-ethyl-6-fluoropyrazolo[1,5-a]pyri din-5-yl)piperazine-1-carboxylate **(3D)** (1.02 g, 2.8 mmol), 2-chloro-4-(4-fluorophenyl-2,3,5,6-d4)thiazole-5-carbonitrile **(intermediate 4)** (0.68 g, 2.8 mmol) and 2,6-dimethylpyridine (0.60 g, 5.6 mmol) were successively added to N,N-dimethylacetamide (20 mL), and the resulting mixture was warmed to 70°C and reacted for 5 h. The reaction was quenched by adding a 10% sodium chloride solution (60 mL) and extracted with ethyl acetate (60 mL × 3). The organic phases were combined, washed with saturated brine (60 mL × 1), dried over anhydrous sodium sulphate and filtered. The filtrate was concentrated under reduced pressure, and the residue was separated by column chromatography on silica gel (petroleum ether : ethyl acetate (v/v) = 3 : 1) to obtain the target compound tert-butyl 4-(3-((5-cyano-4-(4-fluorophenyl-2,3, 5,6-d4)thiazol-2-yl)amino)-2-ethyl-6-fluoropyrazolo[1,5-a]pyridin-5-yl)piperazine-1-carboxylate **(9A)** as a yellowish-brown solid (1.02 g, yield: 64%).

LCMS m/z = 570.2[M +1]+.

Step 2:

tert-butyl 4-(3-((5-cyano-4-(4-fluorophenyl-2,3,5,6-d4)thiazol-2-yl)(methyl)amino)-2-ethyl-6-fluoropyrazolo[1,5-a]pyridin-5-yl)piperazine-1-carboxylate **(9B)**

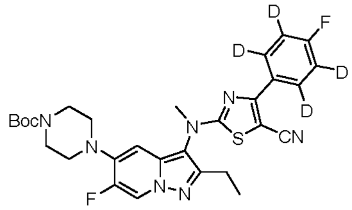

Tert-butyl 4-(3-((5-cyano-4-(4-fluorophenyl-2,3,5,6-d4)thiazol-2-yl)amino)-2-ethyl-6-fluoropyrazolo[1,5-a]pyridin-5-yl)piperazine-1-carboxylate **(9A)** (0.51 g, 0.89 mmol) was dissolved in N,N-dimethylformamide (10 mL). Potassium carbonate (0.27 g, 2 mmol) was added. Methyl iodide (0.19 g, 1.34 mmol) was added dropwise. Upon completion of the addition, the resulting mixture was reacted at room temperature for 30 min. The reaction was quenched by adding water (50 mL) and extracted with ethyl acetate (50 mL × 2). The organic phases were combined, washed with saturated brine (100 mL × 1), dried over anhydrous sodium sulphate and filtered. The filtrate was concentrated under reduced pressure to obtain the target compound tert-butyl 4-(3-((5-cyano-4-(4-fluorophenyl-2,3,5,6-d4)thiazol-2-yl)(methyl)amino)-2-ethyl-6-fluoropyrazolo[1,5-a]pyridin-5-yl)piperazine-1-carboxylate **(9B)** as a yellow solid (0.52 g, yield: 100%).

LCMS m/z = 584.2[M +1]+.

Step 3:

2-((2-ethyl-6-fluoro-5-(piperazin-1-yl)pyrazolo[1,5-a]pyridin-3-yl)(methyl)amino)-4-(4-fluorophenyl-2,3,5,6-d4)thiazole-5-carbonitrile **(9C)**

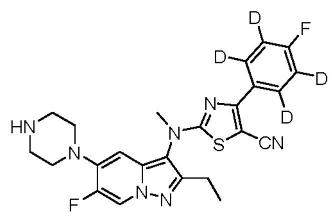

The compound (3-((5-cyano-4-(4-fluorophenyl-2,3,5,6-d4)thiazol-2-yl)(methyl)amino)-2-ethyl-6-fluoropyrazolo[1,5-a]pyridin-5-yl)piperazine-1-carboxylate **(9B)** (0.52 g, 0.89 mmol) was dissolved in dichloromethane (10 mL), and trifluoroacetic acid (3 mL) was added dropwise. The resulting mixture was reacted for about 2 h and then concentrated under reduced pressure to obtain a trifluoroacetate (0.58 g, a brown viscous substance) of the target compound **9C,** and the crude product was directly used in the next reaction.

LCMS m/z = 484.2[M +1]+.

Step 4:

2-((2-ethyl-6-fluoro-5-(4-(2-(3-hydroxyazetidin-1-yl)-2-oxoethyl)piperazin-1-yl)pyrazolo[1,5-a]pyridin-3-yl)(methyl)amino)-4-(4-fluorophenyl-2,3,5,6-d4)thiazole-5-carbonitrile **(compound 9)**

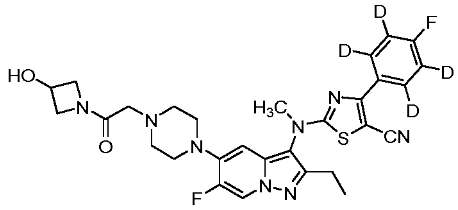

The trifluoroacetate (0.58 g) of the target compound **9C** was dissolved in acetonitrile (15 mL). Potassium carbonate (0.41 g, 3 mmol) and 2-chloro-1-(3-hydroxyazetidin-1-yl)ethanone **(1M)** (0.22 g, 1.5 mmol) were successively added, and the resulting mixture was warmed to 80°C and reacted for 3 h. The reaction was quenched by adding water (50 mL) and extracted with ethyl acetate (50 mL × 3). The organic phases were combined, washed with saturated brine (50 mL × 1), dried over anhydrous sodium sulphate and filtered. The filtrate was concentrated under reduced pressure, and the residue was separated by column chromatography on silica gel (dichloromethane/methanol = 20 : 1) to obtain the title compound 2-((2-ethyl-6-fluoro-5-(4-(2-(3-hydroxyazetidin-1-yl)-2-oxoethyl)piperazin-1-yl)pyrazolo[1,5-a]pyridin-3-yl)(methyl)amino)-4-(4-fluorophenyl-2,3,5,6-d4)thiazole-5-carbonitrile **(compound 9)** (0.39 g, two-step yield: 73%).

$^1$H NMR (400 MHz, $CDCl_3$) δ 8.27 (d, 1H), 6.45 (d, 1H), 4.67 (s, 1H), 4.50-4.41 (m, 1H), 4.32-4.24 (m, 1H), 4.15-4.08 (m, 1H), 3.94-3.86 (m, 1H), 3.58 (s, 3H), 3.22 (s, 4H), 3.17-3.10 (m, 2H), 2.78 (s, 4H), 2.72 (q, 2H), 2.65 (s, 1H), 1.32 (t, 3H).

LCMS m/z = 597.3[M +1]$^+$.

**Example 10:**

2-((2-ethyl-6-fluoro-5-(4-(2-(3-hydroxyazetidin-1-yl)-2-oxoethyl)piperazin-1-yl)pyrazolo[1,5-a]pyridin-3-yl)(methyl-d3)amino)-4-(4-fluorophenyl-2,3,5,6-d4)thiazole-5-carbonitrile **(compound 10)**

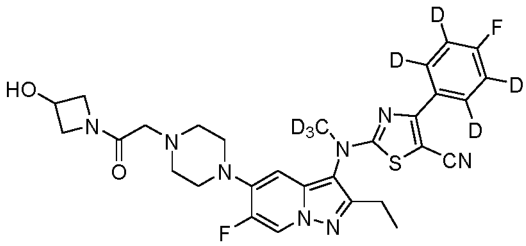

**Compound 10**

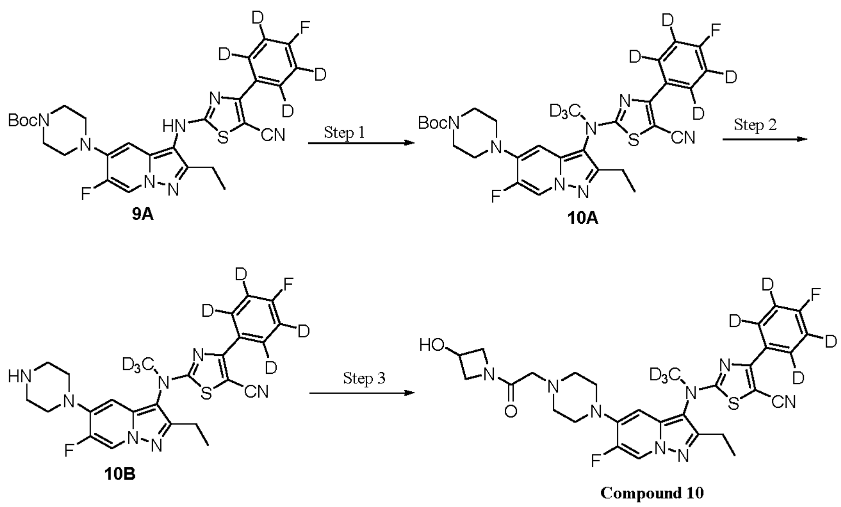

9A 10A 10B Compound 10

Step 1:

tert-butyl4-(3-((5-cyano-4-(4-fluorophenyl-2,3,5,6-d4)thiazol-2-yl)(methyl-d3)amino)-2-ethyl-6-fluoropyrazolo[1,5-a]pyridin-5-yl)piperazine-1-carboxylate **(10A)**

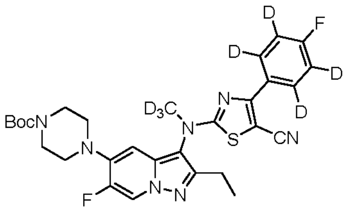

Tert-butyl 4-(3-((5-cyano-4-(4-fluorophenyl-2,3,5,6-d4)thiazol-2-yl)amino)-2-ethyl-6-fluoropyrazolo[1,5-a]pyridin-5-yl)piperazine-1-carboxylate (9A) (0.51 g, 0.89 mmol) was dissolved in N,N-dimethylformamide (10 mL). Potassium carbonate (0.27 g, 2 mmol) was added, and methyl-d3 iodide (0.19 g, 1.34 mmol) was added dropwise. Upon completion of the addition, the resulting mixture was reacted at room temperature for 30 min. The reaction was quenched by adding water (50 mL) and extracted with ethyl acetate (50 mL × 2). The organic phases were combined, washed with saturated brine (100 mL × 1), dried over anhydrous sodium sulphate and filtered. The filtrate was concentrated under reduced pressure to obtain the target compound tert-butyl 4-(3-((5-cyano-4-(4-fluorophenyl-2,3,5,6-d4)thiazol-2-yl)(methyl-d3)amino)-2-ethyl-6-fluoropyrazolo[1,5-a]pyridin-5-yl)piperazine-1-carboxylate **(10A)** as a yellow solid (0.51 g, yield: 98%).

LCMS m/z = 587.2[M +1]$^+$.

Step 2:

2-((2-ethyl-6-fluoro-5-(piperazin-1-yl)pyrazolo[1,5-a]pyridin-3-yl)(methyl-d3)amino)-4-(4-fluorophenyl-2,3,5,6-d4)thiazole-5-carbonitrile **(10B)**

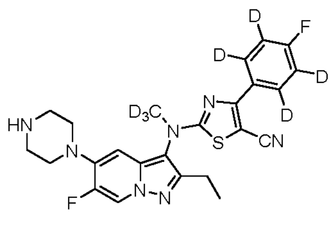

Tert-butyl 4-(3-((5-cyano-4-(4-fluorophenyl-2,3,5,6-d4)thiazol-2-yl)(methyl-d3)amino)-2-ethyl-6-fluoropyrazolo[1,5-a]pyridin-5-yl)piperazine-1-carboxylate **(10A)** (0.51 g, 0.88 mmol) was dissolved in dichloromethane (10 mL), and trifluoroacetic acid (3 mL) was added dropwise. The resulting mixture was reacted for about 2 h and then concentrated under reduced pressure to obtain a trifluoroacetate (0.62 g, a brown viscous substance) of the target compound **10B,** and the crude product was directly used in the next reaction.

LCMS m/z = 487.3[M +1]$^+$.

Step 3:

2-((2-ethyl-6-fluoro-5-(4-(2-(3-hydroxyazetidin-1-yl)-2-oxoethyl)piperazin-1-yl)pyrazolo[1,5-a]pyridin-3-yl)(methyl-d3)amino)-4-(4-fluorophenyl-2,3,5,6-d4)thiazole-5-carbonitrile **(compound 10)**

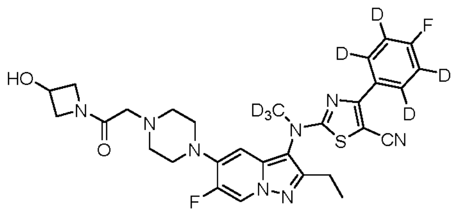

The trifluoroacetate (0.62 g) of compound **10B** was dissolved in acetonitrile (15 mL). Potassium carbonate (0.46 g, 3.3 mmol) and 2-chloro-1-(3-hydroxyazetidin-1-yl)ethanone **(1M)** (0.22 g, 1.5 mmol) were successively added, and the resulting mixture was warmed to 80°C and reacted for 4 h. The reaction was quenched by adding water (60 mL) and extracted with ethyl acetate (60 mL×3). The organic phases were combined, washed with saturated brine (60 mL×1), dried over anhydrous sodium sulphate and filtered. The filtrate was concentrated under reduced pressure, and the residue was separated by column chromatography on silica gel (dichloromethane/methanol (v/v) = 20 : 1) to obtain the title compound 2-((2-ethyl-6-fluoro-5-(4-(2-(3-hydroxyazetidin-1-yl)-2-oxoethyl)piperazin-1-yl)pyrazolo[1,5-a]pyridin-3-yl)(methyl-d3)amino)-4-(4-fluorophenyl-2,3,5,6-d4)thiazole-5-carbonitrile **(compound 10)** (0.35 g, two-step yield: 66%).

$^1$H NMR (400 MHz, $CDCl_3$) $\delta$ 8.27 (d, 1H), 6.44 (d, 1H), 4.66 (s, 1H), 4.50-4.40 (m, 1H), 4.30-4.22 (m, 1H), 4.15-4.05 (m, 1H), 3.93-3.85 (m, 1H), 3.21 (s, 4H), 3.18-3.04 (m, 2H), 2.90 (s, 1H), 2.77-2.66 (m, 6H), 1.32 (t, 3H).

LCMS m/z = 600.3[M +1]$^+$.

**Example 11: Preparation of crystal form I of compound A**

A free base (9.1 g, 15.3 mmol) of 2-((2-ethyl-6-fluoro-5-(4-(2-(3-hydroxyazetidin-1-yl)-2-oxoethyl)piperazin-1-yl)pyrazolo[1,5-a]pyridin-3-yl)(methyl-d3)amino)-4-(4-fluorophenyl)thiazole-5-carbonitrile (compound A, i.e., compound 3) prepared in example 3 was added to ethanol (50 mL), warmed to 75°C, stirred for 2 h and then dissolved. The mixture was gradually cooled to room temperature under slow stirring, with a large amount of white solids precipitated out, and filtered by suction. The filter cake was washed with ethanol (10 mL), and then dried under vacuum at 40°C for 24 h to obtain a crystal form I of compound A as a white solid (6.2 g, yield: 68%). The XRPD pattern thereof was as shown in Figure 1, and XRPD peaks were listed in Table 1. The TGA graph was as shown in Figure 2, and the DSC graph was as shown in Figure 3. Results showed that the sample has a weight loss of 1.3% when heated to 150°C and has one endothermic peak at 178.7°C (initial temperature). The dynamic vapor sorption (DVS) plot was as shown in Figure 4, wherein the water adsorption at 25°C/80% RH was 0.20%. The PLM image was as shown in Figure 5, and the results showed that the crystal has a particle size less than 50 μm.

Table 1 List of XRPD peaks of crystal form I of compound A

| Pos. [°2θ] | Height [cts] | FWHM Left [°2θ] | d-spacing [Å] | Rel. Int. [%] |
|---|---|---|---|---|
| 3.53 | 177.68 | 0.1279 | 25.02 | 1.86 |
| 6.92 | 802.27 | 0.1279 | 12.78 | 8.38 |
| 7.23 | 658.95 | 0.1279 | 12.22 | 6.88 |
| 9.35 | 4733.35 | 0.1535 | 9.46 | 49.45 |

(continued)

| Pos. [°2θ] | Height [cts] | FWHM Left [°2θ] | d-spacing [Å] | Rel. Int. [%] |
|---|---|---|---|---|
| 10.32 | 7358.94 | 0.1535 | 8.57 | 76.87 |
| 12.08 | 9572.89 | 0.1535 | 7.33 | 100.00 |
| 12.58 | 138.70 | 0.1279 | 7.04 | 1.45 |
| 13.05 | 243.53 | 0.1023 | 6.78 | 2.54 |
| 13.46 | 720.73 | 0.1023 | 6.58 | 7.53 |
| 13.72 | 396.86 | 0.1279 | 6.46 | 4.15 |
| 15.10 | 4416.66 | 0.1535 | 5.87 | 46.14 |
| 16.12 | 167.99 | 0.1279 | 5.50 | 1.75 |
| 16.52 | 129.50 | 0.1279 | 5.36 | 1.35 |
| 17.29 | 1497.63 | 0.2558 | 5.13 | 15.64 |
| 18.26 | 2183.47 | 0.1535 | 4.86 | 22.81 |
| 18.63 | 505.17 | 0.1279 | 4.76 | 5.28 |
| 19.20 | 442.98 | 0.1023 | 4.62 | 4.63 |
| 19.47 | 637.77 | 0.1279 | 4.56 | 6.66 |
| 20.58 | 1838.31 | 0.1535 | 4.32 | 19.20 |
| 21.15 | 44.85 | 0.1023 | 4.20 | 0.47 |
| 21.50 | 175.74 | 0.1535 | 4.13 | 1.84 |
| 21.77 | 444.84 | 0.1279 | 4.08 | 4.65 |
| 22.54 | 451.51 | 0.1279 | 3.95 | 4.72 |
| 22.81 | 260.72 | 0.1023 | 3.90 | 2.72 |
| 23.37 | 343.00 | 0.1023 | 3.81 | 3.58 |
| 24.06 | 422.01 | 0.1791 | 3.70 | 4.41 |
| 24.60 | 392.04 | 0.1023 | 3.62 | 4.10 |
| 24.85 | 840.89 | 0.1279 | 3.58 | 8.78 |
| 25.45 | 177.45 | 0.1279 | 3.50 | 1.85 |
| 26.19 | 192.60 | 0.2047 | 3.40 | 2.01 |
| 26.49 | 290.80 | 0.1279 | 3.36 | 3.04 |
| 27.08 | 127.66 | 0.1023 | 3.29 | 1.33 |
| 27.50 | 267.21 | 0.1279 | 3.24 | 2.79 |
| 27.84 | 440.46 | 0.1535 | 3.20 | 4.60 |
| 28.26 | 398.61 | 0.1279 | 3.16 | 4.16 |
| 28.73 | 346.74 | 0.2558 | 3.11 | 3.62 |
| 29.28 | 84.30 | 0.1535 | 3.05 | 0.88 |
| 29.86 | 121.78 | 0.2558 | 2.99 | 1.27 |
| 31.68 | 189.25 | 0.1023 | 2.82 | 1.98 |
| 32.39 | 56.06 | 0.1791 | 2.76 | 0.59 |
| 33.49 | 113.35 | 0.1535 | 2.68 | 1.18 |
| 34.50 | 95.57 | 0.1535 | 2.60 | 1.00 |

(continued)

| Pos. [°2θ] | Height [cts] | FWHM Left [°2θ] | d-spacing [Å] | Rel. Int. [%] |
|---|---|---|---|---|
| 35.14 | 186.14 | 0.2047 | 2.55 | 1.94 |
| 36.71 | 62.86 | 0.2558 | 2.45 | 0.66 |
| 37.60 | 113.45 | 0.1535 | 2.39 | 1.19 |
| 38.45 | 85.25 | 0.1791 | 2.34 | 0.89 |
| 39.68 | 74.00 | 0.1791 | 2.27 | 0.77 |

## Test examples

### 1. Solubility test

The dynamic solubility of the crystal form I of compound A (example 11) in water and three biological vehicles was evaluated. The dynamic solubility (1 h, 2 h, 4 h and 24 h) of samples in four solvent systems, i.e., water, SGF, FaSSIF and FeSSIF, was measured by means of rotational blend (25 rpm) at a feeding concentration of 5-10 mg/mL at 37°C. The samples at each time point were centrifuged (12000 rpm, 5 min) and filtered (0.45 μm PTFE filter), and the UPLC concentration and pH value of the filtrates were measured. The solubility test results were shown in Table 2.

Table 2 Summary of dynamic solubility test results

| Raw material | Vehicle | 1 h | | 2 h | | 4 h | | 24 h | |
|---|---|---|---|---|---|---|---|---|---|
| | | S | pH | S | pH | S | pH | S | pH |
| Compound A Crystal form I | $H_2O$ | > 5.1 | 2.0 | > 5.2 | 2.2 | > 5.3 | 2.0 | > 5.3 | 2.1 |
| | SGF | 0.015 | 6.4 | 0.015 | 6.5 | 0.014 | 6.4 | 0.014 | 6.4 |
| | FaSSIF | 0.32 | 4.9 | 0.27 | 5.0 | 0.36 | 4.9 | 0.21 | 4.9 |
| | FeSSIF | <LOQ | 8.5 | <LOQ | 8.5 | <LOQ | 8.6 | <LOQ | 8.5 |
| S: Solubility (mg/mL);<br>LOQ: 0.03 μg/mL | | | | | | | | | |

The initial pH values of $H_2O$, SGF, FaSSIF and FeSSIF were 6.9, 1.8, 6.5 and 5.0, respectively.

Preparation of biological vehicle

Preparation of simulated gastric fluid (SGF): 0.1 g of NaCl and 0.05 g of Triton X-100 were weighted to a 50 mL volumetric flask, and purified water was added to obtain a clear solution. 67.5 μL of concentrated hydrochloric acid (12 M) was added, and the pH was adjusted to 1.8 with 1 M hydrochloric acid or a 1 M NaOH solution. Purified water was added to a constant volume.

Preparation of simulated intestinal fluid (FaSSIF) in a fasted state: 0.17 g of anhydrous $NaH_2PO_4$, 0.021 g of NaOH and 0.31 g of NaCl were weighed to a 50 mL volumetric flask. About 48 mL of purified water was added to obtain a clear solution, and the pH was adjusted to 6.5 with 1 M hydrochloric acid or a 1 M NaOH solution. Purified water was added to a constant volume, and 0.11 g of SIF powder was weighed to obtain a clear solution.

Preparation of simulated intestinal fluid (FeSSIF) in a feeding state: 0.41 mL of glacial acetic acid, 0.20 g of NaOH and 0.59 g of NaCl were weighed to a 50 mL volumetric flask. About 48 mL of purified water was added to obtain a clear solution, and the pH was adjusted to 5.0 with 1 M hydrochloric acid or a 1 M NaOH solution. Purified water was added to a constant volume, and 0.56 g of SIF powder was weighed to obtain a clear solution.

### 2. Stability test

The crystal form I (example 11) was placed at 25°C/60% RH and 40°C/75% RH for 12 days; and then the sample purity was detected, and the crystal form change was observed. The results were as shown in Table 3.

Table 3 Evaluation of crystal form stability

| Initial sample | Condition | Time point | Purity (area%) | Initial purity (area%) | Crystal form change |
|---|---|---|---|---|---|
| Crystal form I of compound A | 25°C/ 60%RH | 12 days | 99.41 | 99.76 | No |
| | 40°C/ 75%RH | 12 days | 99.44 | 99.76 | No |

Conclusion: the crystal form I of compound A had relatively good stability.

**Bioexperiments**

**1. In vitro bioexperiment**

Autocrine motility factor is a phosphodiesterase in plasma that converts lysophosphatidylcholine (LPC) to lysophosphatidic acid (LPA). Therefore, the formation of LPA can be used to evaluate the potency of an autocrine motility factor inhibitor. The potency of the compound in mixed ex vivo human plasma was evaluated.

Whole blood was anticoagulated with heparin and centrifuged, and then plasma was collected. 5 μL of gradient-diluted test compounds or DMSO was added to 95 μL of plasma and incubated at 37°C for 2 h. After that, a stop buffer (40 mM disodium hydrogen phosphate buffer, containing 30 mM of citric acid, pH = 4) was added. LPA in plasma was detected by LC-MSMS before and after incubation. In order to determine the concentration of LPA 18 : 2 or LPA 20 : 4 in the plasma in study, calibration standard substances at 20000 ng/mL, 10000 ng/mL, 5000 ng/mL, 2000 ng/mL, 1000 ng/mL, 500 ng/mL, 200 ng/mL, 100 ng/mL, 50 ng/mL, 20 ng/mL and 10 ng/mL for LPA 18 : 2 or LPA 20 : 4 were prepared by performing serial dilution in butanol. 3 μL of calibration standard solution was added to 27.0 μL of blank plasmas in each 1.5 mL microcentrifuge tube to produce 1X calibration standard substances. 30.0 μL of standard substances or plasma in study was added to each 1.5 mL microcentrifuge tube. 200 μL of butanol (containing 25.0 ng/mL LPA 17 : 0 for internal control) was added to each 1.5 mL microcentrifuge tube containing the plasma in study or calibration standard substance. Vortex oscillation was performed for 1 min, and centrifugation was performed at 10000 rpm for 10 min. 180 μL of the supernatant was transferred to a 96-well plate, and LC/MS/MS was used together with the standard substance to quantify the concentration of LPA 18 : 2 in the plasma. In short, 8 μL of the solution was injected, and LC-MS/MS analysis using an ACQUITY UPLC BEH C18 column (2.1 × 50 mm, 1.7 μm) was performed, wherein mobile phase A [20 mM $NH_4OAC$ aqueous solution (0.1% FA)] and mobile phase B [5 mM $NH_4OAC$ aqueous solution/0.2% FA in ACN = 5 : 95] were used. The parameters of a mass spectrometer for LPA 18 : 2 were optimized by means of deprotonation of molecular ions. For LPA 18 : 2 at m/z 433.2([M-H]-), LPA 20 : 4 at 457.2, and LPA 18 : 2 and LPA 20 : 4 both at m/z 152.8, abundant product ions were obtained. Quantitative data were obtained in a multiple reaction monitoring (MRM) negative electrospray ionization mode.

The inhibition rates of compounds at different concentrations on LPA formation were determined by comparing the changes of LPA levels in incubated and unincubated plasma. The $IC_{50}$ of the compounds was calculated as a calculation inhibition rate, and the relative concentration at each well = the concentration at each well - the average concentration at baseline.

Inhibition rate% = (average relative concentration at control well - average relative concentration at test well) / average relative concentration at control well × 100%. The curve was plotted with the inhibition rates as a y axis and the compound concentrations as an x axis, and fitting was performed by GraphPad Prism7.0 using log (inhibitor) and normalized response (variable slope).

Table 4 $IC_{50}$ (nM) of test compounds for inhibiting LPA formation

| Examples | LPA formation (18 : 2) | LPA formation (20 : 4) |
|---|---|---|
| 1 | 46.05 | 48.51 |
| 2 | 51.96 | 55.27 |
| 3 | 42.66 | 44.47 |
| 4 | 51.79 | 54.81 |

## 2. Pharmacokinetic test

### 2.1 Pharmacokinetic test in rats

Test objective: test compounds were given to SD rats via single-dose intravenous and intragastric administration, the concentrations of the test compounds in plasma of rats were measured, and the pharmacokinetic characteristics and bioavailability of test compounds in rats were evaluated.

Test subject: the compound in example 3 of the present invention.

Test animals: male SD rats, about 220 g, 6-8 weeks old, six rats/compound, purchased from Chengdu Ddossy Experimental Animals Co., Ltd.

Test method: on the day of the test, 6 SD rats were randomly grouped according to their body weights. The rats were fasted with water available for 12-14 h one day before the administration, and were fed 4 h after the administration. The administration was performed according to Table 5.

Table 5 Administration information

| Group | Number | Administration information | | | | | | |
|---|---|---|---|---|---|---|---|---|
| | Male | Test compound | Administration dosage* (mg/kg) | Administration concentration (mg/mL) | Administration volume (mL/kg) | Collected sample | Mode of administration | Vehicle |
| G1 | 3 | Compound | 1 | 0.2 | 5 | Plasma | Intravenously | 5% DMA + 5% Solutol + 90% Saline |
| G2 | 3 | Compound | 5 | 0.5 | 10 | Plasma | Intragastrically | 0.5% MC |

*Dosage is calculated on the basis of free base.

**2.2 Biological sample collection**

Before and after the administration, 0.1 mL of blood was taken from the orbits of the rats under isoflurane anaesthesia, and placed in an EDTAK2 centrifuge tube. Centrifugation was performed at 5000 rpm at 4°C for 10 min, and plasma was collected.
Time points for sample collection in group G1 comprise 0 min, 5 min, 15 min, 30 min, 1 h, 2 h, 4h, 6 h, 8 h and 24 h.
Time points for sample collection in group G2 comprise 0 min, 15 min, 30 min, 1 h, 2h, 4h, 6 h, 8 h and 24 h.
Before analysis and detection, all samples were stored at -80°C.

**2.3 Sample pretreatment**

30 μL of each of plasma samples, standard curve samples and quality control samples was taken, and 200 μL of acetonitrile solution containing an internal standard was added, and the resulting mixture was homogeneously mixed by vortex and centrifuged at 4°C at 12000 rpm for 10 min. 170 μL of the supernatant was taken to a 96-well plate, and LC-MS/MS analysis was performed, wherein the sample size was 2 μL.
The main pharmacokinetic parameters were analysed by a non-compartmental model using WinNonlin 8.0 software. The test results were as shown in Table 6.

Table 6 Pharmacokinetics in rats

| Example no. | Mode of administration | $C_0$ or $C_{max}$ (ng/ml) | AUC (h*ng/ml) | $T_{1/2}$ (h) | $T_{max}$ (h) | F% |
|---|---|---|---|---|---|---|
| Compound I-1 | IV 1 mg/kg | 870 ± 25 | 1491 ± 203 | 2.69 ± 0.48 | - | - |
| | PO 5 mg/kg | 268 ± 76 | 2048 ± 884 | 3.29 ± 0.25 | 2.83 ± 2.8 | 29.3 ± 8.0 |
| Compound I-2 | IV 1 mg/kg | 841 ± 123 | 1024 ± 43 | 1.79 ± 0.22 | - | - |
| | PO 5 mg/kg | 149 ± 26 | 1395 ± 537 | 2.62 ± 1.1 | 5.33 ± 1.2 | 27.2 ± 10 |
| Compound 1 | IV 1 mg/kg | 967 ± 106 | 1061 ± 124 | 2.60 ± 0.61 | - | - |
| | PO 5 mg/kg | 181 ± 10 | 1173 ± 46 | 6.40 ± 2.4 | 4.00 ± 2.0 | 22.1 ± 0.87 |
| Compound 2 | IV 1 mg/kg | 1032 ± 62 | 1332 ± 83 | 2.40 ± 0.35 | - | - |
| | PO 5 mg/kg | 297 ± 105 | 2570 ± 449 | 2.87 ± 0.15 | 4.33 ± 2.9 | 38.6 ± 6.7 |
| Compound 9 | IV 1 mg/kg | 748 ± 187 | 943 ± 109 | 1.93 ± 0.072 | - | - |
| | PO 5 mg/kg | 399 ± 137 | 2418 ± 860 | 2.43 ± 0.33 | 1.17 ± 0.76 | 51.3 ± 18 |
| Compound 10 | IV 1 mg/kg | 813 ± 85 | 1053 ± 84 | 2.05 ± 0.29 | - | - |
| | PO 5 mg/kg | 234 ± 136 | 1961 ± 858 | 2.63 ± 0.071 | 2.83 ± 2.0 | 37.3 ± 6 |

Conclusion: the compounds of the present invention, particularly compounds 2, 9 and 10, had good pharmacokinetics and high bioavailability in rats; and compounds 5, 6 and 7 had rapid onset.

**2.2 Pharmacokinetic test in mice**

Test objective: the pharmacokinetic characteristics and bioavailability of test compounds in mice were evaluated.
Test animals: thirty-six C57 male mice, about 25 g, 6-8 weeks old, purchased from Chengdu Ddossy Experimental Animals Co., Ltd., with the production license number of SCXK (CHUAN) 2020-030.
Test subject: compound I-1 (compound 101 in WO 2019228403 A1) and compound 3.

Test design:

Table 7 Administration information

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected sample | Mode of administration |
| G1 | 9 | Positive compound I-1 | 1 | 0.2 | 5 | Plasma | Intravenously |
| G2 | 9 | Example compound 3 | 1 | 0.2 | 5 | Plasma | |
| G3 | 9 | Positive compound I-1 | 10 | 1 | 10 | Plasma | Intragastrically |
| G4 | 9 | Example compound 3 | 10 | 1 | 10 | Plasma | |

Vehicle for intravenous administration: 5% DMA + 5% Solutol + 90% Saline; vehicle for intragastric administration: 0.5% MC

Before and after the administration, 0.06 mL of blood was taken from the orbits under isoflurane anaesthesia and placed in an EDTAK2 centrifuge tube. The blood was centrifuged at 5000 rpm at 4°C for 10 min, and plasma was collected. Time points for blood collection: 0 min, 5 min, 15 min, 30 min, 1 h, 2 h, 4 h, 6 h, 8 h, 10 h and 24 h. Before analysis and detection, all plasma samples were stored at - 80°C. Test results were shown in Table 8.

Table 8 Pharmacokinetics in mice

| Example no. | Mode of administration | $C_0$ or $C_{max}$ (ng/ml) | AUC (h*ng/ml) | $T_{1/2}$ (h) | $T_{max}$ (h) | F% |
|---|---|---|---|---|---|---|
| Compound I-1 | IV 1 mg/kg | 1383 | 1594 | 1.78 | - | - |
| | PO 10 mg/kg | 928 | 6768 | 2.14 | 2.00 | 42.5 |
| Compound 3 | IV 1 mg/kg | 1108 | 1490 | 1.56 | - | - |
| | PO 10 mg/kg | 1296 | 9604 | 2.14 | 0.25 | 64.5 |

Conclusion: the compounds of the present invention, particularly compound 3, had good pharmacokinetics and high bioavailability in mice.

**2.3 Pharmacokinetic test in dogs**

Test objective: the pharmacokinetic characteristics and bioavailability of test compounds in dogs were evaluated.

Test animals: twelve male beagles, 8-10 kg, 0.5-1.5 years old, purchased from Beijing Marshall Biotechnology Co. Ltd., with the production license number of SCXK (JING) 2016-001.

Test subject: compound I-1 (compound 101 in WO 2019228403 A1) and compound 3.

Test design:

Table 9 Administration information

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected sample | Mode of administration |
| G1 | 3 | Positive compound I-1 | 1 | 1 | 1 | Plasma | Intravenously |
| G2 | 3 | Example compound 3 | 1 | 1 | 1 | Plasma | |
| G3 | 3 | Positive compound I-1 | 2 | 0.4 | 5 | Plasma | Intragastrically |
| G4 | 3 | Example compound 3 | 2 | 0.4 | 5 | Plasma | |

Vehicle for intravenous administration: 5% DMSO + 5% Solutol + 90% Saline;
vehicle for intragastric administration: 1% DMSO + 1% Solutol + 98% (0.5%MC);

Before and after the administration, 1.0 mL of blood was taken from the veins of the forelimbs and placed in an EDTAK2 centrifuge tube. The blood was centrifuged at 5000 rpm at 4°C for 10 min, and plasma was collected. Time points for blood collection: 0 min, 5 min, 15 min, 30 min, 1 h, 2 h, 4 h, 6 h, 8 h, 10 h, 12 h and 24 h. Before analysis and detection, all plasma samples were stored at -80°C. Test results were shown in Table 10.

Table 10 Pharmacokinetics in dogs

| Example no. | Mode of administration | $C_0$ or $C_{max}$ (ng/ml) | AUC (h*ng/ml) | $T_{1/2}$ (h) | $T_{max}$ (h) | F% |
|---|---|---|---|---|---|---|
| Compound | IV 1 mg/kg | 1374 ± 72 | 6850 ± 1812 | 5.68 ± 1.0 | - | - |
| I-1 | PO 2 mg/kg | 1081 ± 149 | 9124 ± 1303 | 7.25 ± 0.45 | 2.00 ± 0.0 | 66.6 ± 9.5 |
| Compound 3 | IV 1 mg/kg | 1486 ± 451 | 6838 ± 883 | 6.96 ± 1.5 | - | - |
| | PO 2 mg/kg | 1618 ± 176 | 15929 ± 2977 | 7.19 ± 0.75 | 1.67 ± 0.58 | 116 ± 22 |

Conclusion: the compounds of the present invention, particularly compound 3, had good pharmacokinetics and high bioavailability in dogs.

**2.4 Pharmacokinetic test in monkeys**

Test objective: the pharmacokinetic characteristics and bioavailability of test compounds in monkeys were evaluated.

Test animals: twelve male Cynomolgus macaques, 2.4-5.9 kg, 3-5.5 years old, purchased from Suzhou Xishan Zhongke Laboratory Animal Co., Ltd., with the production license number of SCXK (SU) 2018-0001.

Test subject: compound I-1 (compound 101 in WO 2019228403 A1) and compound 3.

Test design:

Table 11 Administration information

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected sample | Mode of administration |
| G1 | 3 | Positive compound I-1 | 1 | 1 | 1 | Plasma | Intravenously |
| G2 | 3 | Example compound 3 | 1 | 1 | 1 | Plasma | |
| G3 | 3 | Positive compound I-1 | 5 | 1 | 5 | Plasma | Intragastrically |
| G4 | 3 | Example compound 3 | 5 | 1 | 5 | Plasma | |

Vehicle for intravenous administration: 5% DMSO + 5% Solutol + 90% Saline;
vehicle for intragastric administration: 1% DMSO + 1% Solutol + 98% (0.5% CMC-Na)

Before and after the administration, 1.0 mL of blood was taken from the veins of the forelimbs and placed in an EDTAK2 centrifuge tube. The blood was centrifuged at 5000 rpm at 4°C for 10 min, and plasma was collected. Time points for blood collection: 0 min, 5 min, 15 min, 30 min, 1 h, 2 h, 4 h, 6 h, 8 h, 10 h, 12 h and 24 h. Before analysis and detection, all plasma samples were stored at -80°C. Test results were shown in Table 12.

Table 12 Pharmacokinetics in monkeys

| Example no. | Mode of administration | $C_0$ or $C_{max}$ (ng/ml) | AUC (h*ng/ml) | $T_{1/2}$ (h) | $T_{max}$ (h) |
|---|---|---|---|---|---|
| Compound I-1 | IV 1 mg/kg | 1002 ± 92 | 1453 ± 374 | 1.74 ± 0.23 | - |
| | PO 5 mg/kg | 516 ± 60 | 2624 ± 23 | 3.18 ± 1.04 | 1.33 ± 0.58 |
| Compound 3 | IV 1 mg/kg | 1037 ± 116 | 2009 ± 621 | 2.96 ± 1.0 | - |
| | PO 5 mg/kg | 657 ± 170 | 3121 ± 492 | 4.02 f 0.59 | 1.33 ± 0.58 |

Conclusion: the compounds of the present invention, particularly compound 3, had good pharmacokinetics and high bioavailability in monkeys.

**2.5 Test of effects on hERG potassium channel**

Test compounds: compound I-1 (compound 101 in WO 2019228403 A1) and example compound 3.

The effect of the example compounds on the current of an hERG potassium channel (human *Ether-à-go-go* Related Gene potassium channel) was tested by means of an electrophysiological manual patch-clamp method.

Table 13 Test materials and instruments

| **Test material** | **Provider (cat. no.)** |
|---|---|
| 6 cm cell culture dish | Shanghai Yes Service Biotech, Inc. (150288) |
| 3.5 cm cell culture dish | Shanghai Yes Service Biotech, Inc. (153066) |
| Dialysed fetal bovine serum | Shanghai BioSun Sci&Tech Co., Ltd. (BS-0005-500) |
| DMSO | Merck KGaA (102952) |
| DMEM medium | Thermo Fisher Scientific (China) Co., Ltd. (10569) |
| HEPES | Thermo Fisher Scientific (China) Co., Ltd. (15630080) |
| Trypsin | Thermo Fisher Scientific (China) Co., Ltd. (12604) |
| Phosphate buffer solution (without calcium and magnesium ions) | Thermo Fisher Scientific (China) Co., Ltd. (14190) |
| Penicillin-streptomycin solution | Thermo Fisher Scientific (China) Co., Ltd. (15140-122) |
| MEM non-essential amino acid solution | Thermo Fisher Scientific (China) Co., Ltd. (11140) |
| Geneticin (G418) | Thermo Fisher Scientific (China) Co., Ltd. (11811031) |
| Blasticidin | Thermo Fisher Scientific (China) Co., Ltd. (R21001) |
| Polylysine | Thermo Fisher Scientific (China) Co., Ltd. (P4832) |
| Dofetilide | Beijing Express Technology Co., Ltd. (D525700) |
| Doxycycline | Sigma-Aldrich Shanghai Trading Co., Ltd. (D9891) |
| **Test instrument** | **Provider (model)** |
| Carbon dioxide incubator | Thermo Fisher Scientific (China) Co., Ltd. (371) |
| Glass microelectrode puller | Sutter Instrument Company, United States (P-97) |
| Micromanipulator | Siskiyou Corporation, United States (MC1000e) |

(continued)

| Test material | Provider (cat. no.) |
|---|---|
| | Sutter Instrument Company, United States (ROE-200; MP285) |
| Amplifier | HEKA, German (EPC10) |
| Amplifier | AXON, United States (Multiclamp 700B) |
| Microscope | Olympus (China) Co., Ltd. (IX51/71/73) |
| Perfusion system | ALA Company, United States (VM8 gravity administration system) |

Cell line and cell culture:

HEK293 cell lines stably expressing hERG ion channels were purchased from Invitrogen. The cells were cultured in a culture medium containing 85% DMEM, 10% dialysed fetal bovine serum, a 0.1 mM MEM non-essential amino acid solution, a 100 U/mL penicillin-streptomycin solution, 25 mM HEPES, 5 μg/mL blasticidin and 400 μg/mL geneticin (G418). When the cell density was increased to 40%-80% of the bottom area of the culture dish, the cells were digested with trypsin and passaged three times a week. Before the test, the cells were cultured in a 6 cm culture dish at a density of $5 \times 10^5$, induced by adding 1 μg/mL doxycycline for 48 h, then digested and inoculated on slides for use in the subsequent manual patch-clamp testing.

Solution preparation:

Extracellular fluid (in mM): 132 mM sodium chloride, 4 mM potassium chloride, 3 mM calcium chloride, 0.5 mM magnesium chloride, 11.1 mM glucose, and 10 mM HEPES (the pH was adjusted to 7.35 by using sodium hydroxide).

Solution preparation for test compound:

A test compound was firstly dissolved in DMSO and prepared into a stock solution with a final concentration of 30 mM. The original stock solution was then diluted with DMSO in a certain ratio to form four gradient-dilution solutions with the concentrations of 10 mM, 3.33 mM, 1.11 mM and 0.37 mM, respectively. Before the beginning of the test, the gradient-dilution solutions of the test compound were diluted with the extracellular fluid to form working solutions with a series of gradient concentrations according to 1 : 1000, and the final concentrations of the working solutions were 30 μM, 10 μM, 3.33 μM, 1.11 μM and 0.37 μM, respectively. Five working solutions with different gradient concentrations were used to determine the potential inhibitory effect of the compound on the hERG potassium channel and used to fit a dose-response curve and calculate $IC_{50}$.

Test steps:

1. A small slide carrying HEK293 cells in a culture dish was placed in a perfusion tank on a micromanipulator.
2. An appropriate cell was adjusted to the centre of the visual field under an Olympus IX51, IX71 or IX73 inverted microscope. The tip of the glass electrode was found using a ×10 objective lens and adjusted to the centre of the visual field. Then the electrode was moved down using a micromanipulator, and meanwhile the coarse adjustment was performed so that the electrode slowly approached the cell.
3. When the electrode was close to the cell, a ×40 objective lens was used for observation, and fine-tuning was performed by means of the micromanipulator so that the electrode was gradually close to the surface of the cell.
4. Negative pressure was applied, allowing a seal with a resistance higher than 1GΩ to be formed between the electrode tip and cell membrane.
5. Fast transient capacitance current $C_{fast}$ was compensated in a voltage-clamp mode. Then, the membrane was broken by repeatedly applying short negative pressure, so that a whole-cell recording was finally formed.
6. When the membrane potential was clamped at -60 mV, the slow capacitance current $C_{slow}$, cell membrane capacitance (Cm) and membrane input resistance (Ra) were compensated, respectively.
7. After the cell was stabilized, the clamp voltage was changed to -90 mV, the sampling frequency was set to 20

kHz, and the filter frequency was set to 10 kHz. The detection condition for a leakage current was that the clamp voltage was changed to -80 mV, and the time course was 500 ms.

8. A method for testing hERG currents was as follows: a 4.8-second depolarization command voltage was applied to depolarize the membrane potential from -80 mV to +30 mV, and then a 5.2-second repolarization voltage was applied instantly to reduce the membrane potential to -50 mV and achieve channel deinactivation, so that the hERG tail current was observed. The peak value of the tail current was the magnitude of hERG currents.

9. The hERG currents used to detect the test compounds were continuously recorded for 120 seconds prior to administration, thereby evaluating the stability of test cells generating hERG currents. Only stable cells within a range accepted by the evaluation criteria can enter the subsequent compound detection.

10. The inhibitory effects of the test compounds on hERG currents were tested as follows: firstly, the hERG current measured in the extracellular fluid containing 0.1% DMSO was used as a test baseline. After the hERG current remained stable for at least 5 min, solutions containing the test compounds were perfused successively around the cells from a low concentration to a high concentration. Upon completion of each perfusion, waiting about 5 min allowed the compounds to be fully acted on the cells, and the hERG currents were recorded synchronously. After the recorded currents became stable, the last five hERG current values were recorded and averaged to obtain a final current value at a specific concentration. After a compound was tested, 150 nM Dofetilide was added to the same cell to completely inhibit its current, which served as a positive control for the cell. Meantime, the positive compound Dofetilide was subjected to a synchronous detection by using the same patch-clamp system before and after the end of the test on the test compound, so as to ensure the reliability and sensitivity of the whole detection system.

Data analysis: The percentage of current inhibition was calculated by the following formula. Data were output by PatchMaster or Clampex10.2 software. The dose-response curve was fitted by Graphpad Prism 8.0 software, and the $IC_{50}$ value was calculated. Test results were shown in Table 11.

$$\text{Inhibition ratio of tail current} = \left(1 - \frac{\text{Magnitude of tail current}_{\text{compound}} - \text{Magnitude of tail current}_{\text{positive drug}}}{\text{Magnitude of tail current}_{\text{blank}} - \text{Magnitude of tail current}_{\text{positive drug}}}\right) \times 100$$

Table 14 Test results

| No. | Test sample | hERG $IC_{50}$ [μM] |
|---|---|---|
| 1 | Compound I-1 | 9.368 |
| 2 | Dofetilide | 0.016 |
| 3 | Compound 3 | 23.113 |

The widely accepted and used criteria for evaluating whether a compound has an inhibitory effect on hERG potassium channels were as follows: non-significant inhibitory effect: $IC_{50}$ > 10 μM; medium-level inhibitory effect: 1 μM < $IC_{50}$ < 10 μM; and significant inhibitory effect: $IC_{50}$ < 1 μM. As can be seen from the test results, compound 3 exhibited a non-significant inhibitory effect on hERG potassium channels.

## Claims

1. A compound of formula (I) or a stereoisomer, solvate, prodrug, metabolite, pharmaceutically acceptable salt or eutectic thereof, wherein

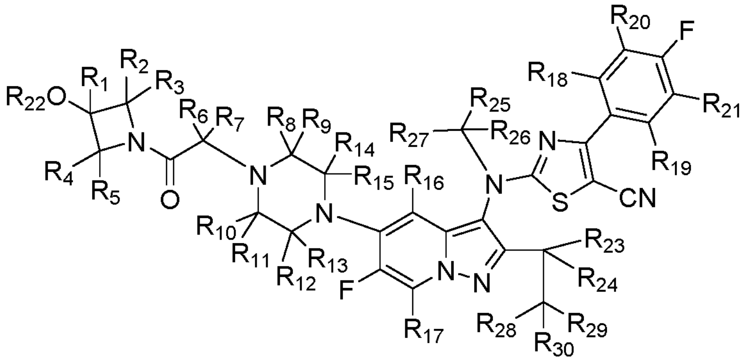

(I)

$R_1$, $R_2$, $R_3$, $R_4$, $R_5$, $R_6$, $R_7$, $R_8$, $R_9$, $R_{10}$, $R_{11}$, $R_{12}$, $R_{13}$, $R_{14}$, $R_{15}$, $R_{16}$, $R_{17}$, $R_{18}$, $R_{19}$, $R_{20}$, $R_{21}$, $R_{22}$, $R_{23}$, $R_{24}$, $R_{25}$, $R_{26}$, $R_{27}$, $R_{28}$, $R_{29}$ and $R_{30}$ are each independently selected from hydrogen or deuterium, provided that at least one of $R_1$, $R_2$, $R_3$, $R_4$, $R_5$, $R_6$, $R_7$, $R_8$, $R_9$, $R_{10}$, $R_{11}$, $R_{12}$, $R_{13}$, $R_{14}$, $R_{15}$, $R_{16}$, $R_{17}$, $R_{18}$, $R_{19}$, $R_{20}$, $R_{21}$, $R_{22}$, $R_{23}$, $R_{24}$, $R_{25}$, $R_{26}$, $R_{27}$, $R_{28}$, $R_{29}$ and $R_{30}$ is selected from a deuterium atom and the compound of formula (I) does not have the following structure:

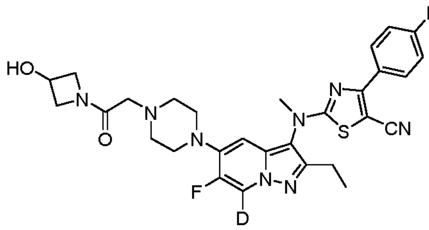

.

**2.** The compound of formula (I) or the stereoisomer, solvate, prodrug, metabolite, pharmaceutically acceptable salt or eutectic thereof according to claim 1, wherein
$R_{22}$ is selected from H.

**3.** The compound of formula (I) or the stereoisomer, solvate, prodrug, metabolite, pharmaceutically acceptable salt or eutectic thereof according to either claim 1 or claim 2, wherein

$R_1$ is selected from deuterium; or
$R_2$ and $R_3$ are selected from deuterium; or
$R_6$ and $R_7$ are selected from deuterium; or
$R_8$, $R_9$, $R_{10}$, $R_{11}$, $R_{12}$, $R_{13}$, $R_{14}$ and $R_{15}$ are selected from deuterium; or
$R_{16}$ is selected from deuterium; or
$R_{17}$ is selected from deuterium; or
$R_{18}$, $R_{19}$, $R_{20}$ and $R_{21}$ are selected from deuterium; or
$R_{23}$ and $R_{24}$ are selected from deuterium; or
$R_{25}$, $R_{26}$ and $R_{27}$ are selected from deuterium; or
$R_{28}$, $R_{29}$ and $R_{30}$ are selected from deuterium.

**4.** The compound of formula (I) or the stereoisomer, solvate, prodrug, metabolite, pharmaceutically acceptable salt or eutectic thereof according to either claim 1 or claim 2, wherein

$R_{23}$, $R_{24}$, $R_{25}$, $R_{26}$, $R_{27}$, $R_{28}$, $R_{29}$ and $R_{30}$ are selected from deuterium; or
$R_8$, $R_9$, $R_{10}$, $R_{11}$, $R_{12}$, $R_{13}$, $R_{14}$, $R_{15}$, $R_{25}$, $R_{26}$ and $R_{27}$ are selected from deuterium; or
$R_8$, $R_9$, $R_{10}$, $R_{11}$, $R_{12}$, $R_{13}$, $R_{14}$, $R_{15}$, $R_{23}$, $R_{24}$, $R_{25}$, $R_{26}$, $R_{27}$, $R_{28}$, $R_{29}$ and $R_{30}$ are selected from deuterium; or
$R_8$, $R_9$, $R_{10}$, $R_{11}$, $R_{12}$, $R_{13}$, $R_{14}$, $R_{15}$, $R_{16}$ and $R_{17}$ are selected from deuterium; or
$R_8$, $R_9$, $R_{10}$, $R_{11}$, $R_{12}$, $R_{13}$, $R_{14}$, $R_{15}$, $R_{18}$, $R_{19}$, $R_{20}$ and $R_{21}$ are selected from deuterium; or
$R_{23}$, $R_{24}$, $R_{28}$, $R_{29}$, $R_{30}$, $R_{18}$, $R_{19}$, $R_{20}$ and $R_{21}$ are selected from deuterium; or
$R_{25}$, $R_{26}$, $R_{27}$, $R_{18}$, $R_{19}$, $R_{20}$ and $R_{21}$ are selected from deuterium; or
$R_1$, $R_{25}$, $R_{26}$ and $R_{27}$ are selected from deuterium; or
$R_1$, $R_{23}$, $R_{24}$, $R_{28}$, $R_{29}$ and $R_{30}$ are selected from deuterium; or

$R_6$, $R_7$, $R_{25}$, $R_{26}$ and $R_{27}$ are selected from deuterium; or
$R_6$, $R_7$, $R_{23}$, $R_{24}$, $R_{28}$, $R_{29}$ and $R_{30}$ are selected from deuterium; or
$R_2$, $R_3$, $R_{25}$, $R_{26}$ and $R_{27}$ are selected from deuterium; or
$R_2$, $R_3$, $R_{23}$, $R_{24}$, $R_{28}$, $R_{29}$ and $R_{30}$ are selected from deuterium.

**5.** The compound of formula (I) or the stereoisomer, solvate, prodrug, metabolite, pharmaceutically acceptable salt or eutectic thereof according to claim 1, wherein the compound has a structure selected from one of:

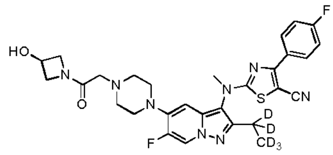
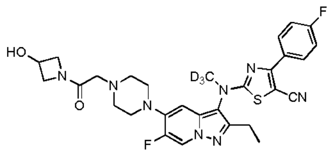
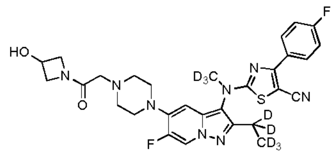
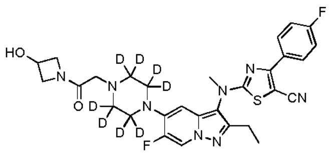
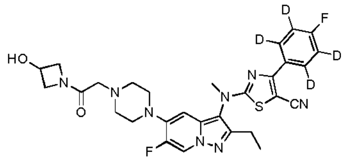
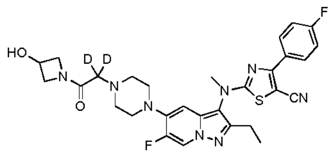
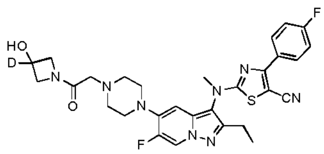
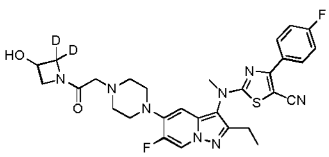
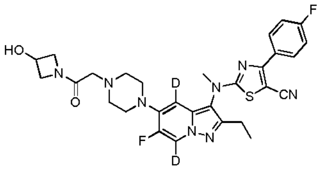
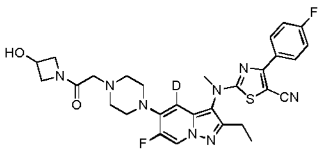
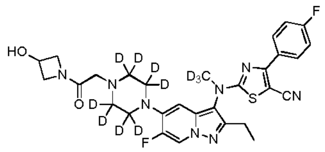
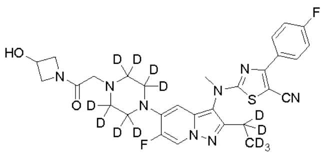
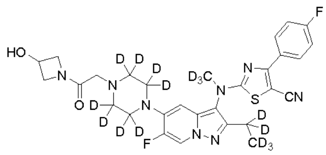
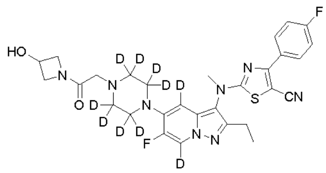
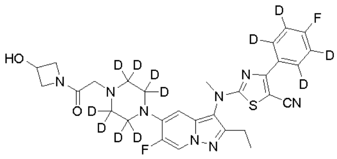
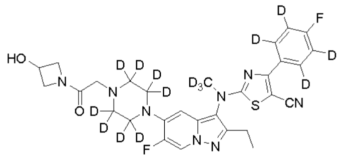
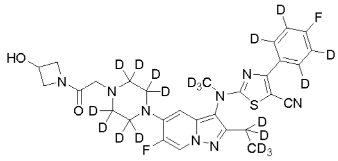
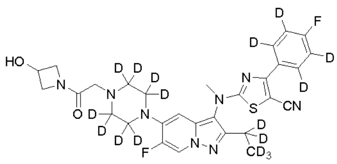
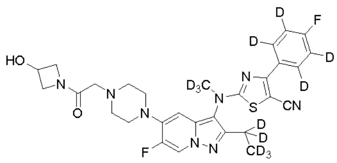
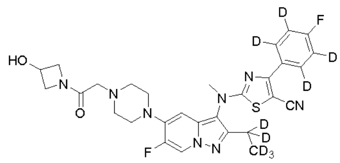

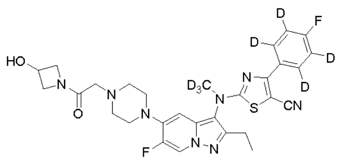

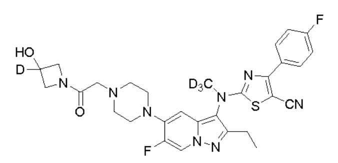

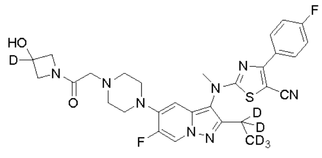

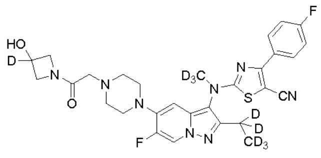

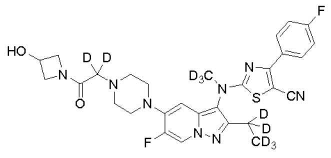

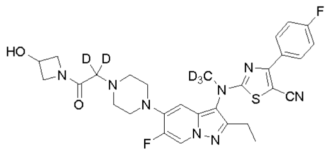

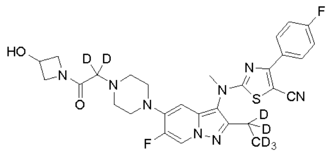

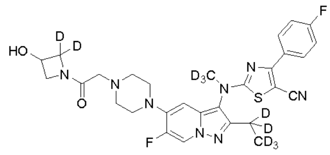

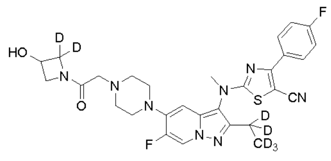

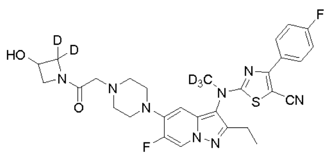

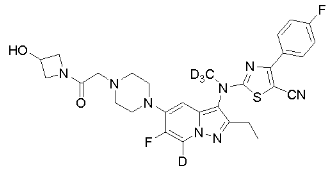

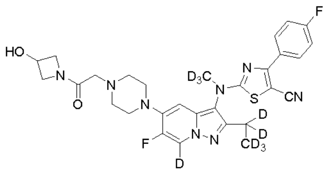

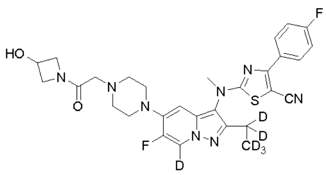

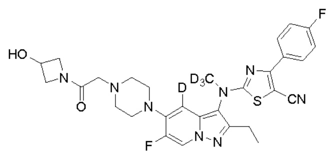

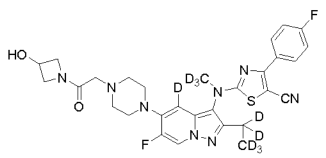

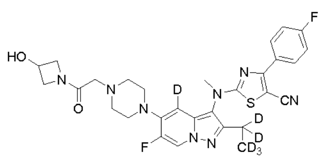

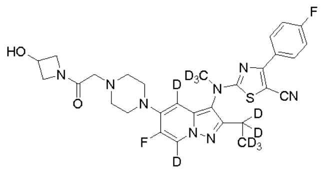

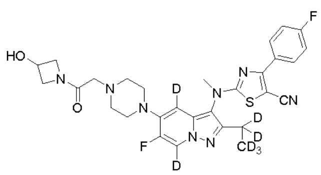

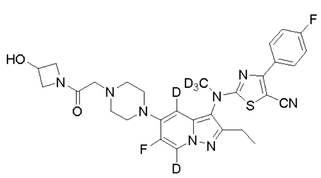

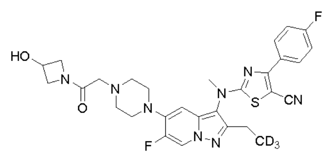

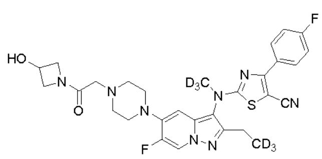

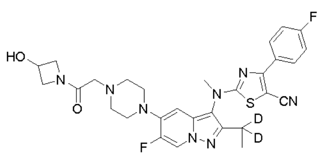 or 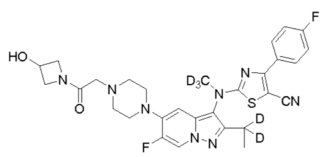.

**6.** A crystal form I of a compound of formula (A),

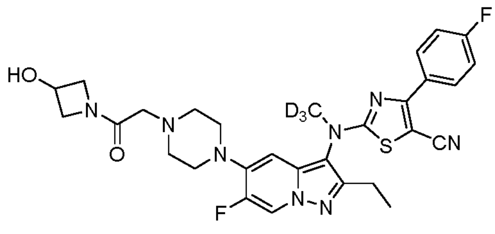

(A)

**characterized in that** the crystal form I has an X-ray powder diffraction pattern comprising characteristic diffraction peaks at 9.35 ± 0.2°, 10.32 ± 0.2°, 12.08 ± 0.2° and 15.10 ± 0.2° 2θ, as determined by using Cu-Kα radiation.

7. The crystal form I of the compound of formula (A) according to claim 6, wherein the crystal form I has an X-ray powder diffraction pattern further comprising characteristic diffraction peaks at 17.29 ± 0.2°, 18.26 ± 0.2° and 20.58 ± 0.2° 2θ.

8. The crystal form I of the compound of formula (A) according to claim 7, wherein the crystal form I has an X-ray powder diffraction pattern further comprising characteristic diffraction peaks at 6.92 ± 0.2°, 7.23 ± 0.2°, 13.46 ± 0.2°, 19.47 ± 0.2° and 24.85 ± 0.2° 2θ.

9. The crystal form I of the compound of formula (A) according to claim 8, wherein the crystal form I has an X-ray powder diffraction pattern substantially as shown in Figure 1.

10. The crystal form I of the compound of formula (A) according to claim 6, wherein the crystal form I has a TGA curve substantially as shown in Figure 2 and a DSC pattern substantially as shown in Figure 3.

11. A compound of formula (II-A), formula (II-B) or formula (II-C) as shown below:

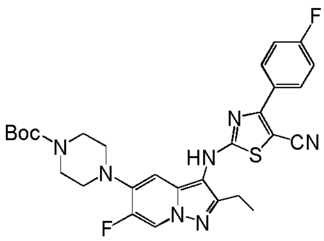

,

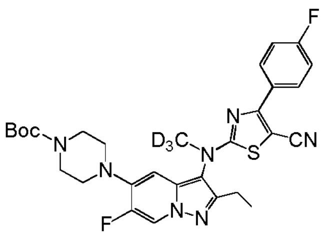

and

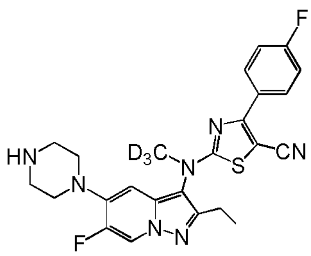

(II-A) (II-B) (II-C).

12. A method for preparing a compound of formula (A), **characterized in that** the method comprises the following steps:

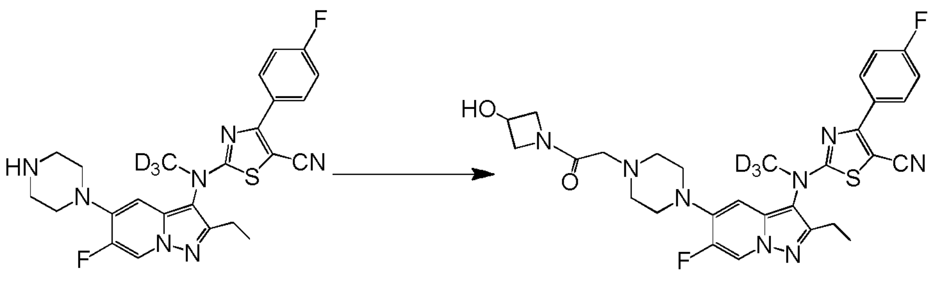

(II-C) (A)

dissolving a compound of formula (II-C) in an organic solvent A, and reacting the resulting solution with 2-chloro-1-(3-hydroxyazetidin-1-yl)ethanone and a base at 70°C-90°C to obtain the compound of formula (A).

13. The preparation method according to claim 12, **characterized in that** the organic solvent A is selected from at least one of acetonitrile, 2-methyltetrahydrofuran, ethylbenzene, methyl and ethyl glyoxylate and diethylene glycol tert-butyl ether; and the base is at least one of potassium carbonate and sodium carbonate.

14. The preparation method according to claim 12, **characterized in that** the method for preparing the compound of formula (A) further comprises the following steps:

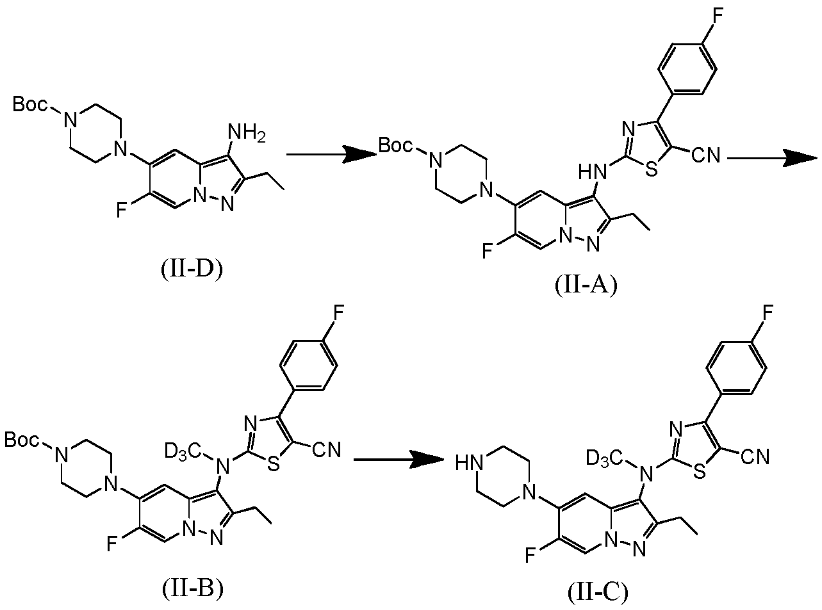

(II-D) (II-A)

(II-B) (II-C)

i. reacting a compound of formula (II-D) with 2-chloro-4-(4-fluorophenyl)thiazole-5-cyano, 2,6-dimethylpyridine and an aprotic polar solvent at 60°C-80°C to obtain a compound of formula (II-A);
ii. dissolving the compound of formula (II-A) obtained in step i in an organic solvent B, cooling the resulting solution to 0°C under nitrogen protection, adding a reducing agent, and after a reaction is completed, adding methyl-$d_3$ iodide to obtain a compound of formula (II-B); and
iii. dissolving the compound of formula (II-B) obtained in step ii in an organic solvent C, adding trifluoroacetic acid, and carrying out a reaction to obtain the compound of formula (II-C).

15. The preparation method according to claim 14, **characterized in that** the aprotic polar solvent is at least one of N,N-dimethylacetamide, dimethylformamide, hexamethylphosphoramide, acetonitrile, acetone and dimethyl sulfoxide; the organic solvent B is at least one of tetrahydrofuran, N,N-dimethylacetamide, dimethylformamide, acetonitrile and acetone; the reducing agent is sodium hydride; and the organic solvent C is at least one of dichloromethane, methyl acetate, dimethyl carbonate, propylene glycol methyl ether acetate and dimethyl nylon acid.

16. A pharmaceutical composition, **characterized in that** the pharmaceutical composition comprises the compound or the stereoisomer, solvate, prodrug, metabolite, pharmaceutically acceptable salt or eutectic thereof according to any one of claims 1-5, or the crystal form I according to any one of claims 6-10, and a pharmaceutically acceptable carrier and/or excipient.

17. The use of the compound or the stereoisomer, solvate, prodrug, metabolite, pharmaceutically acceptable salt or eutectic thereof according to any one of claims 1-5, or containing the crystal form I according to any one of claims 6-10 or the composition according to claim 16 in the preparation of a drug for treating an ATX-mediated disease.

18. The use according to claim 17, **characterized in that** the ATX-mediated disease is idiopathic pulmonary fibrosis.

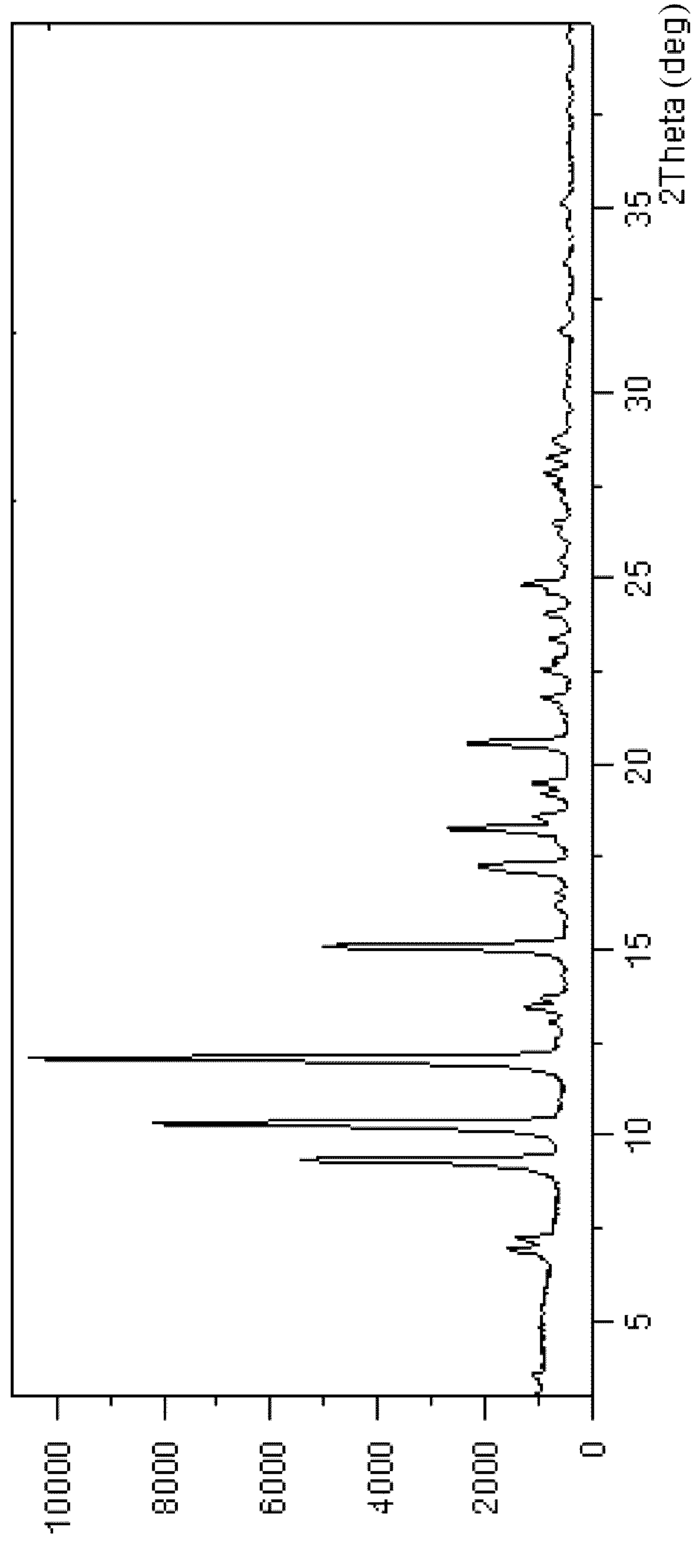
Intensity (counts)
10000
8000
6000
4000
2000
0
5
10
15
20
25
30
35
2Theta (deg)

*Fig. 1*

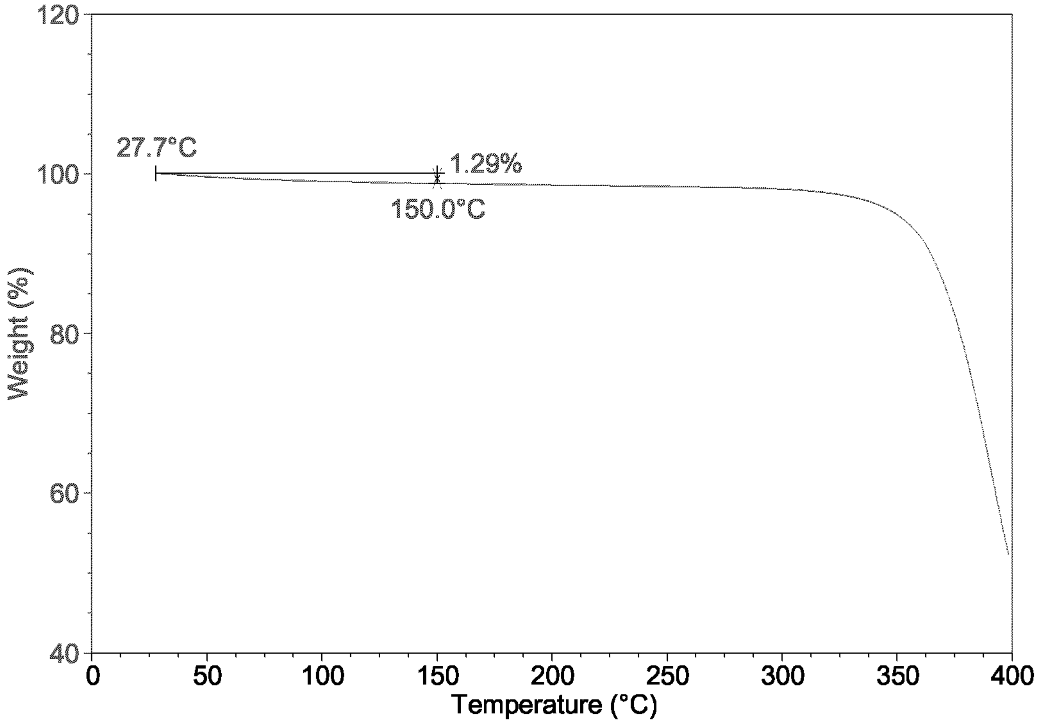
120
100
80
60
40
Weight (%)
27.7°C
1.29%
150.0°C
0
50
100
150
200
250
300
350
400
Temperature (°C)

*Fig. 2*

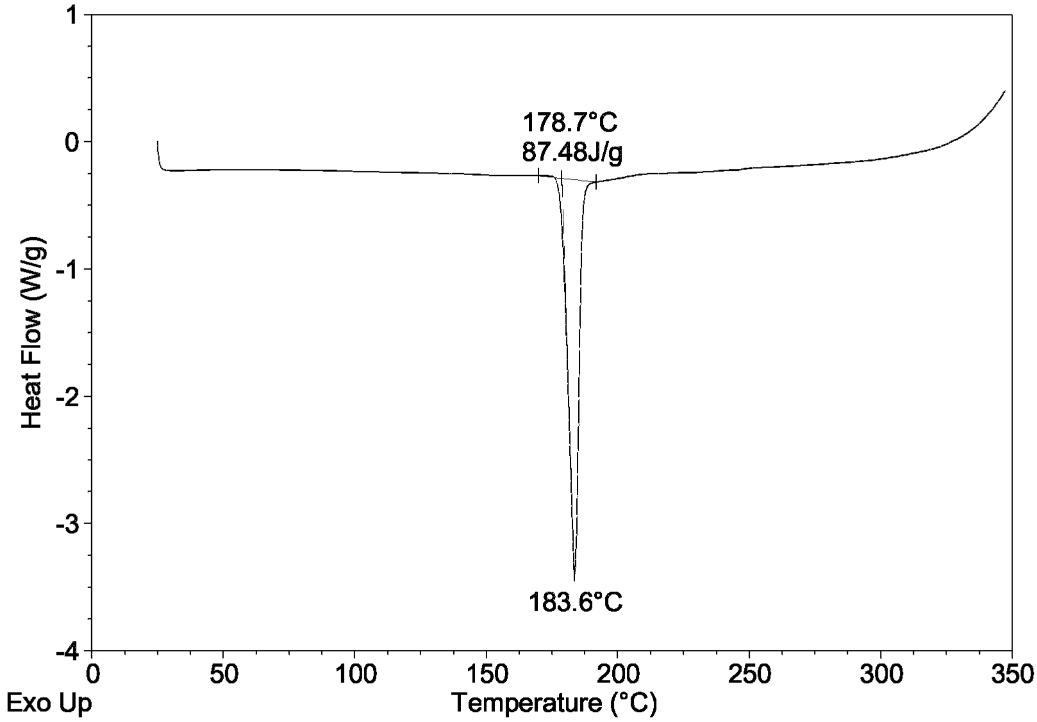
1
0
-1
-2
-3
-4
Heat Flow (W/g)
178.7°C
87.48J/g
183.6°C
0
50
100
150
200
250
300
350
Exo Up
Temperature (°C)

*Fig. 3*

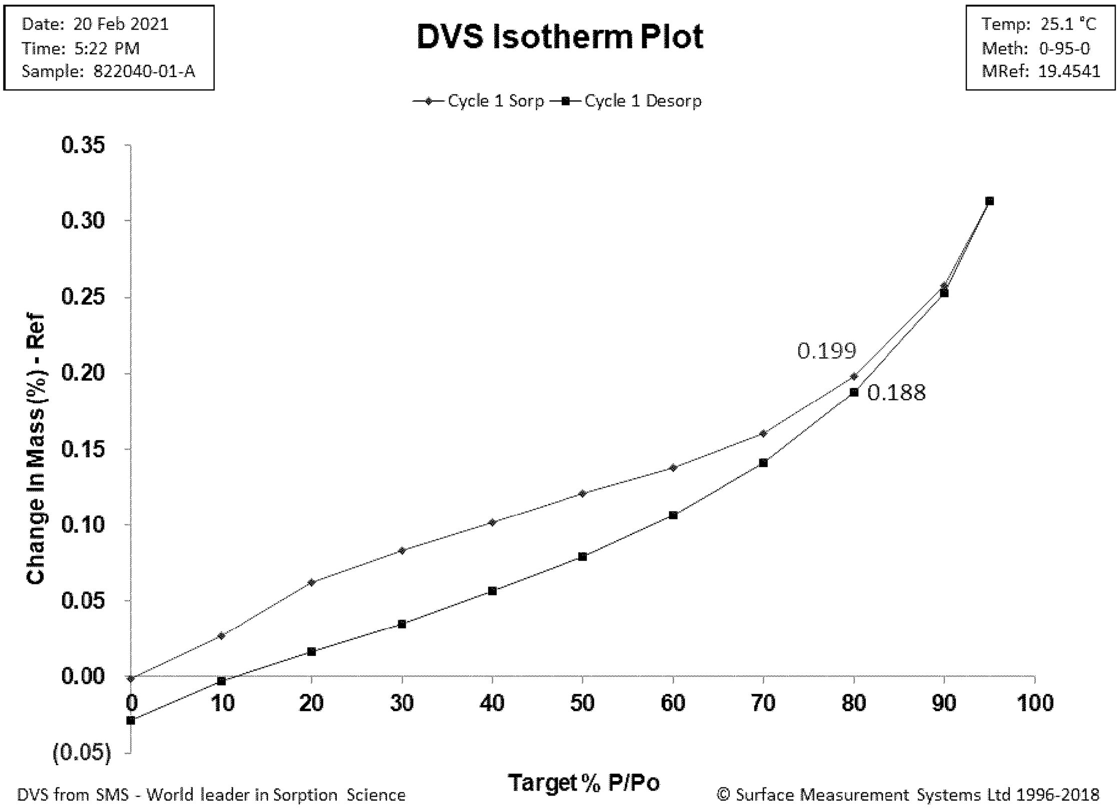
Date: 20 Feb 2021
Time: 5:22 PM
Sample: 822040-01-A
DVS Isotherm Plot
Temp: 25.1 °C
Meth: 0-95-0
MRef: 19.4541
Cycle 1 Sorp
Cycle 1 Desorp
Change In Mass (%) - Ref
0.35
0.30
0.25
0.20
0.15
0.10
0.05
0.00
(0.05)
0.199
0.188
0
10
20
30
40
50
60
70
80
90
100
Target % P/Po
DVS from SMS - World leader in Sorption Science
© Surface Measurement Systems Ltd 1996-2018

*Fig. 4*

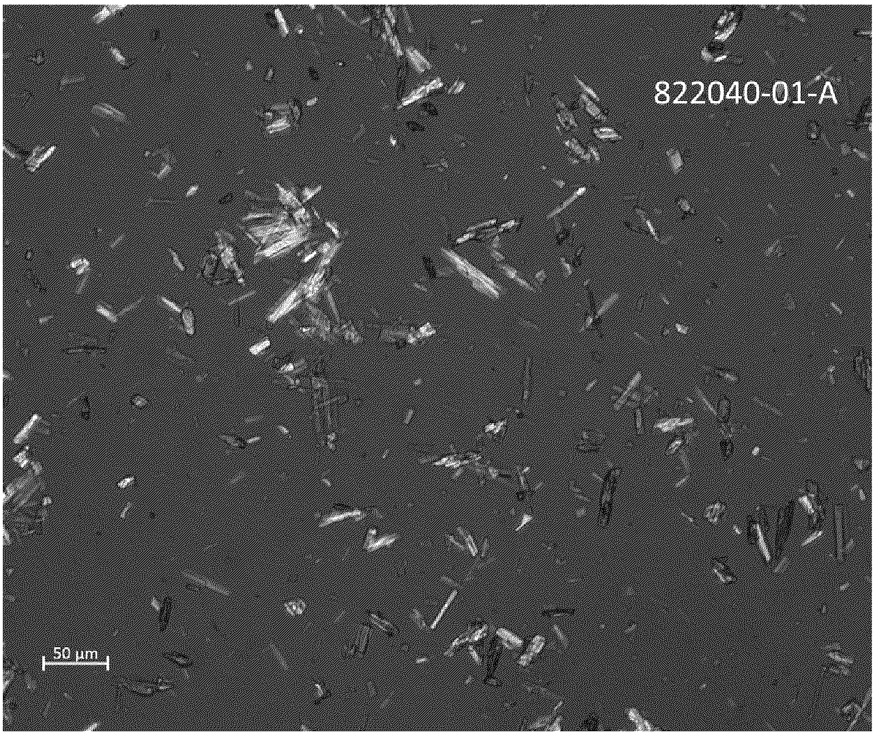
822040-01-A
50 µm

*Fig. 5*

**INTERNATIONAL SEARCH REPORT**

International application No.
**PCT/CN2021/128735**

**A. CLASSIFICATION OF SUBJECT MATTER**

C07D 471/04(2006.01)i； A61K 31/437(2006.01)i； A61P 35/00(2006.01)i； A61P 37/00(2006.01)i； A61P 29/00(2006.01)i； A61P 25/28(2006.01)i； A61P 11/00(2006.01)i； A61P 9/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNPAT; EPODOC; WPI; CNTXT; USTXT; REGISTRY(STN); CAPLUS(STN): 海思科, 李瑶, 张国彪, 张晓波, 唐平明, 陈娅姝, 余彦, 张晨, 严庞科, ATX, 抑制剂, 分泌酶, autotaxin, inhibitor

**C. DOCUMENTS CONSIDERED TO BE RELEVANT**

| Category* | Citation of document, with indication, where appropriate, of the relevant passages | Relevant to claim No. |
|---|---|---|
| X | CN 111801328 A (FRONTHERA U S PHARMACEUTICALS LLC) 20 October 2020 (2020-10-20) description pages 1-4, 49, 140, claims 1, 63, 70 | 1-18 |
| A | CN 105339370 A (GALAPAGOS N. V.) 17 February 2016 (2016-02-17) entire description | 1-18 |
| A | CN 105143221 A (GALAPAGOS N. V.) 09 December 2015 (2015-12-09) entire description | 1-18 |
| A | CN 110156772 A (SUZHOU XINNUOWEI PHARMACEUTICAL TECHNOLOGY CO., LTD.) 23 August 2019 (2019-08-23) entire description | 1-18 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **21 January 2022** | **29 January 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2021/128735**

| Patent document cited in search report | Publication date (day/month/year) | Patent family member(s) | Publication date (day/month/year) |
|---|---|---|---|
| CN 111801328 A | 20 October 2020 | WO 2019228403 A1 | 05 December 2019 |
| | | CA 3089527 A1 | 05 December 2019 |
| | | JP 2021526123 A | 30 September 2021 |
| | | EP 3728257 A1 | 28 October 2020 |
| | | EP 3728257 A4 | 23 June 2021 |
| | | US 20190367515 A1 | 05 December 2019 |
| CN 105339370 A | 17 February 2016 | US 2016214986 A1 | 28 July 2016 |
| | | US 9796719 B2 | 24 October 2017 |
| | | WO 2014202458 A1 | 24 December 2014 |
| | | US 2018127425 A1 | 10 May 2018 |
| | | EP 3010922 A1 | 27 April 2016 |
| | | EP 3010922 B1 | 15 March 2017 |
| | | JP 2016525072 A | 22 August 2016 |
| | | JP 6435323 B2 | 05 December 2018 |
| CN 105143221 A | 09 December 2015 | EP 3760630 A1 | 06 January 2021 |
| | | JP 2016512208 A | 25 April 2016 |
| | | JP 6339595 B2 | 06 June 2018 |
| | | TW 201936604 A | 16 September 2019 |
| | | US 2016244443 A1 | 25 August 2016 |
| | | US 9670204 B2 | 06 June 2017 |
| | | US 2014303140 A1 | 09 October 2014 |
| | | US 8993590 B2 | 31 March 2015 |
| | | WO 2014139882 A1 | 18 September 2014 |
| | | JP 2018135350 A | 30 August 2018 |
| | | JP 6517976 B2 | 22 May 2019 |
| | | US 11072611 B2 | 27 July 2021 |
| | | US 2019169184 A1 | 06 June 2019 |
| | | US 10526329 B2 | 07 January 2020 |
| | | TW 201444835 A | 01 December 2014 |
| | | TW I695002 B | 01 June 2020 |
| | | EP 2970255 A1 | 20 January 2016 |
| | | EP 2970255 B1 | 02 August 2017 |
| | | EP 2970255 B9 | 19 September 2018 |
| CN 110156772 A | 23 August 2019 | US 2020369676 A1 | 26 November 2020 |
| | | WO 2019158107 A1 | 22 August 2019 |
| | | JP 2021513551 A | 27 May 2021 |
| | | CN 111620865 A | 04 September 2020 |
| | | CN 111620864 A | 04 September 2020 |
| | | EP 3753943 A1 | 23 December 2020 |
| | | EP 3753943 A4 | 23 June 2021 |
| | | KR 20200120930 A | 22 October 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019228403 A1


### Non-patent literature cited in the description


- *J. Med. Chem.,* 2017, vol. 60, 3580-3590